# EUROPÄISCHE PATENTSCHRIFT

(11) **EP 1 778 685 B1**
(45) Veröffentlichungstag und Bekanntmachung des Hinweises auf die Patenterteilung: **26.03.2008**
(21) Anmeldenummer: 05769702.1
(22) Anmeldetag: 29.07.2005
(51) Int. Cl.: C07D 471/04, A61K 31/496

(54) **CARBOXAMIDE DES INDOLIZINS UND SEINER AZA- UND DIAZADERIVATE**
INDOLIZINE CARBOXAMIDES AND THE AZA AND DIAZA DERIVATIVES THEREOF
CARBOXAMIDES D'INDOLIZINE ET LEURS AZA- ET DIAZA-DERIVES

(30) Priorität: 02.08.2004 DE 102004037445; 02.08.2004 EP 04018251
(43) Veröffentlichungstag der Anmeldung: 02.05.2007
(73) Patentinhaber: SCHWARZ PHARMA AG, 40789 Monheim/Rhld. (DE)
(72) Erfinder: GMEINER, Peter, 91054 Buckenhof (DE); HÜBNER, Harald, 91336 Heroldsbach (DE); BETTINETTI, Laura, I-50053 Empoli (IT); SCHLOTTER, Karin, 86732 Oettingen (DE)
(74) Vertreter: Dressen, Frank
(86) Internationale Anmeldenummer: PCT/EP2005/008240
(87) Internationale Veröffentlichungsnummer: WO 2006/015737

(56) Entgegenhaltungen:
- EP-A- 0 496 692
- WO-A-03/028728
- WO-A-20/04024878

## Beschreibung

Dopamin gilt als wichtiger Neurotransmitter des zentralen Nervensystems. Seine Wirkung vermittelt Dopamin durch Bindung an fünf verschiedene Dopaminrezeptoren. Diese lassen sich aufgrund ihrer Morphologie und ihrer Art der Signalübertragung in die Klassen D1-like (D1 und D5) sowie D2-like (D2-, D3- und D4-Rezeptoren) einteilen (Neve, K.A. The Dopamine Receptors. Humana Press, 1997). Vor allem die Subtypen der D2-Familie spielen bei der Regulation zentralnervöser Vorgänge eine wichtige Rolle. Während die D2-Rezeptoren überwiegend in den Basalganglien exprimiert werden und dort an der Kontrolle und Modulation neuromotorischer Schaltkreise beteiligt sind, befinden sich D3-Rezeptoren vor allem im mesolimbischen System, in dem emotionale und kognitive Vorgänge gesteuert werden. Störungen in der Signaltransduktion dieser Rezeptoren führen zu zahlreichen neuropathologischen Veränderungen, die zum Teil schwerwiegende Erkrankungen hervorrufen. Somit stellt insbesondere der D3-Rezeptor ein vielversprechendes Target für die Entwicklung von Wirkstoffen zur Behandlung von psychiatrischen Erkrankungen wie der Schizophrenie oder der unipolaren Depressionen, von Bewusstseinsstörungen sowie zur Behandlung neurodegenerativer Krankheiten wie dem Parkinsonismus und den im Zuge einer Langzeittherapie auftretendenden Dyskinesien, aber auch zur Behandlung von Drogenabhängigkeit (Pulvirenti, L. et al. Trends Pharmacol. Sci. 2002, 23,151-153, Joyce, J.N. Pharmacol. Ther. 2001, 90, 231-259) dar. Anzustreben ist dabei ein möglichst D3-Rezeptor-selektives Bindungsprofil für solche Wirksubstanzen. Je nach intrinsischer Aktivität (voller Agonist, partieller Agonist, Antagonist oder inverser Agonist) können solche Liganden stimuliernd, modulierend oder auch hemmend auf das pathologisch veränderte Dopamin-Signaltransduktionssystem Einfluß nehmen und somit zur Therapie dieser Erkrankungen eingesetzt werden.

Verbindungen mit Arylpiperazin-Struktur sind bereits als dopaminrezeptoraktive Liganden beschrieben worden (Robarge, M.J. J. Med. Chem. 2001, 44, 3175-3186). Weiterhin sind Benzamide und Naphthamide mit Arylpiperazin-Partiaistruktur als Liganden von Dopaminrezeptoren bekannt (Perrone, R. J. Med. Chem.1998, 41, 4903-4909; EP 0 779 284 A1). Vor kurzer Zeit wurden auch Heteroarenamide als D3-Rezeptor-aktive Verbindungen beschrieben (Bettinetti, L. et al. J. Med. Chem. 2002, 45,4594-4597, Leopoldo, M. et al. J. Med. Chem. 2002, 45,5727-5735, WO 2004004729 A1). Kürzlich wurde außerdem von einem Phenylpiperazinylnaphthamid als selektiver D3-Partialagonist berichtet, der im Tiermodell hoffnungsvolle Aktivitäten zeigt, die für die Behandlung der Kokainsucht eingesetzt werden könnten (Pilla, M. et al. Nature 1999, 400, 371-375). Darüber hinaus konnte aufgrund der charakteristischen Eigenschaften dieser Verbindung eine Aufhebung der bei einer Langzeittherapie des Parkinsonismus mit dem Arzneistoff L-DOPA verursachten schweren Bewegungsanomalien (Dyskinesien) erzielt werden (Bezard, E. et al. Nature Med. 2003, 9, 762-767). Neueste Literatur beschreibt die neuroprotektive Wirkung D3-selektiver Partialagonisten gegen MPTP-induzierten Neuronenverlust bei Mäusen als murines Modell des Parkinsonismus (Boeckler, F. et al. Biochem. Pharmacol. 2003, 6,1025-1032).

Aus der Reihe der Arylpiperazinylheteroarencarboxamide sind vor allem Strukturbeispiele mit. Sauerstoff- schwefel- oder stickstoffhaltigen Heteroarencarbonsäurekomponenten beschrieben (ES 2027898; EP 343 961; US 3646047; US 3734915; WO 2004/024878; Leopoldo, M. et al. J. Med. Chem. 2002, 45, 5727-5735, WO 2004004729 A1). Indolizinsubstituierte Liganden werden in diesen Referenzen nicht offenbart.

Bettinetti, L. et al. J. Med. Chem. 2002, 45, 4594-4597 beschrieben erstmals einige wenige Pyrazolo[1,5-a]pyridine mit Affinität zum D3-Rezeptor. Andere Indolizin-substituierte Liganden sind hingegen bisher nicht bekannt.

Wir haben im Rahmen unserer Struktur-Wirkungsuntersuchungen von Dopaminrezeptorliganden neue Verbindungen der Formel (1) - (IX) entdeckt. Diese zeigten bei *in vitro* Untersuchungen besonders hohe Affinität und selektive Bindungseigenschaften am D3-Rezeptor. Einige Verbindungen weisen zudem auch bemerkenswerte Affinität zu serotonergen Rezeptoren, insbesondere zum 5-HT1a-Rezeptor auf.

Die erfindungsgemäßen Verbindungen könnten somit wertvolle Therapeutika zur Behandlung von ZNS-Erkrankungen, wie beispielsweise Schizophrenie oder verschiedenen Arten der Depression, zur Neuroprotektion bei neurodegenerativen Erkrankungen, bei Suchterkrankungen, Glaukoma, kognitiven Störungen, Restless Leg Syndrom, Hyperaktivitätssyndrom (ADHS), Hyperprolaktinämie, Hyperprolaktinom, Autismus, bei idiopathischen oder Medikamenten-induzierten extrapyramidalmotorischen Bewegungsstörungen, z.B. Akathisie, Rigor, Dystonie und Dyskinesien sowie verschiedenen Erkrankungen des Urinaltraktes darstellen.

Gegenstand dieser Erfindung sind Verbindungen der allgemeinen Formel I, 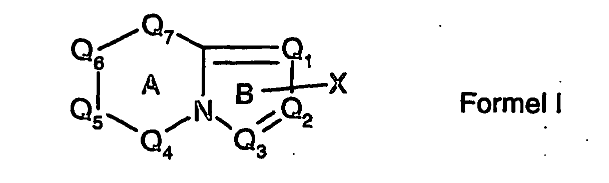 in der bedeuten:
A ist ein gesättigter oder aromatischer 6-gliedriger Ring;
B ist ein aromatischer 5-gliedriger Ring;
das aus A+B gebildete Heteroaren weist insgesamt höchstens drei N-Atome und genau eine Gruppe X auf;
Q1, Q2 und Q3 sind jeweils unabhängig voneinander N, CH oder C-R1;
Q4 ist N-R, CH-R1' oder C-R1 R1';
Q5, Q6 und Q7 sind unabhängig voneinander CH-R1' oder C-R1 R1';
R1 ist jeweils ausgewählt aus der Gruppe Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
R1' fehlt, wenn Ring A aromatisch ist oder ist Wasserstoff, wenn Ring A gesättigt ist;
R fehlt, wenn Ring A aromatisch ist oder ist ausgewählt aus Wasserstoff, Alkyl, Phenyl, Alkylcarbonyl, Phenylcarbonyl, Phenylalkyl und Phenylsulfonyl, wenn Ring A gesättigt ist;
X ist eine an ein C-Atom eines aromatischen Rings A oder B gebundene Gruppe der allgemeinen Formel X1 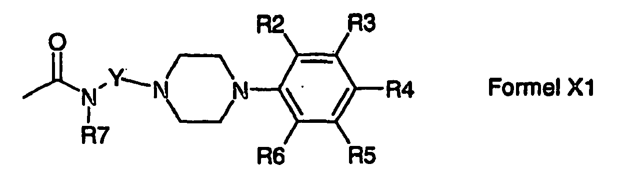 wobei gilt:
   Y ist eine unverzweigte, gesättigte oder ungesättigte Kohlenwasserstoffkette mit 2-5 Kohlenstoffatomen oder eine Kette -(CH2)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Phenylalkyloxycarbonyl, Alkyloxycarbonyl, Cyano, Nitro, Amino, Carboxy, Sulfo, Sulfamoyl, Sulfonylamino, Alkylaminosulfonyl und Alkylsulfonylamino, wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können;
   R7 ist Wasserstoff, Alkyl oder Phenylalkyl;

in Form der freien Base, deren physiologisch akzeptable Salze sowie möglicher Enantiomere und Diastereomere,
mit der Maßgabe, daß ausgenommen sind,
(a) Verbindungen, in denen der Heterozyklus ein Pyrazolo[1,5-a]pyridin ist, insbesondere wenn dieses als einzigen Substituenten die Gruppe X, aber keinen Substituenten R1 trägt, wobei für X gilt: R2 = Methoxy; R3, R4, R5, R6 und R7 sind jeweils Wasserstoff und
   (i) Y = Ethylen, n-Propylen oder n-Butylen oder
   (ii) Y = n-Pentylen und X ist in 2- oder 3-Position mit dem Pyrazolo[1,5-a] pyridin-Kem verknüpft
(b) Die Verbindung N-4-(4-(2-Chlorphenyl)piperazin-1-yl)butyl-7-methylpyrazolo[1,5-a]pyridin-3-ylcarbamid.

In den Verbindungen der allgemeinen Formel I, wie weiter oben definiert, kann die Gruppe X grundsätzlich mit jedem zur Bindung geeigneten, ringbildenden Kohlenstoff eines aromatischen Rings A oder B verknüpft sein. Ist A ein gesättigter Ring, ist X an ein Kohlenstoffatom des Rings B gebunden. Die Bedeutung der Gruppen Q1, Q2, Q3, Q4, Q5, Q6 und Q7 in Formel I, wie weiter oben beschrieben, ist dementsprechend erfindungsgemäß so zu verstehen, dass eines der von den Gruppen Q1, Q2, Q3, Q4, Q5, Q6 und Q7 umfassten ringbildenden Kohlenstoffe eines aromatischen Rings mit der Gruppe X substituiert ist und die Gruppe C-X bildet.

Der Begriff "gesättigter Ring A" und grammatikalische Äquivalente dieses Begriffs bedeuten in der vorliegenden Patentanmeldung, dass der Ring A maximal gesättigt ist, das heißt, alle ringbildenden Atome des Rings A, die nicht gleichzeitig Teil des aromatischen Rings B sind, sind vollständig gesättigt.

In einer Ausführungsform der Erfindung weisen die beiden Ringe A und B neben der Gruppe X höchstens 4, 3, 2 oder 1 Substituenten R1 auf oder sind abgesehen von der Gruppe X unsubstituiert.

In einer bevorzugten Ausführungsform der Erfindung sind die Substituenten R1 des Heteroarens in den erfindungsgemäßen Verbindungen der allgemeinen Formeln I, II, III, IV, V, VI, VII, VIII und IX ausgewählt aus der Gruppe Hydroxy, Fluor, Chlor, Brom, Trifluormethyl, Cyano, Amino, Carboxy, Sulfo, Sulfamoyl, unsubstituiertes oder mit Hydroxy substituiertes C1-C6 Alkyl, unsubstituiertes oder mit Hydroxy substituiertes C1-C6 Alkyloxy, unsubstituiertes oder mit Hydroxy substituiertes C1-C6 Alkylthio, unsubstituiertes C2-C6 Alkinyl, unsubstituiertes oder mit Fluor, Chlor oder Brom und/oder mit einer oder mehreren Methoxygruppen substituiertes Phenyl, unsubstituiertes oder mit Fluor, Chlor oder Brom und/oder mit einem oder mehreren Methoxygruppen substituiertes Phenoxy, -C(O)-C1-C6 Alkyl, wobei das Alkyl unsubstituiert oder mit Hydroxy substituiert ist, -C(O)-Phenyl, wobei das Phenyl jeweils unsubstituiert oder mit Fluor, Chlor oder Brom und/oder mit einer oder mehreren Methoxygruppen substituiert ist, C1-C6 Alkyloxycarbonyl, wobei das Alkyl unsubstituiert oder mit Hydroxy substituiert ist, C1-6 Alkylaminosulfonyl, insbesondere Methylaminosulfonyl und C1-6 Alkylsulfonylamino, insbesondere Methansulfonylamino.

Der Substituent Q4 in Ring A steht je nach Sättigungsgrad des Rings A für N-R, CH-R1' oder C-R1 R1'. In einem gesättigten Ring A steht R1' für Wasserstoff und Q4 ist ausgewählt aus NR, CH₂ und CH-R1, wobei R bevorzugt ausgewählt ist aus Wasserstoff, Phenylalkyl und Phenylsulfonyl und wobei R1 die Bedeutung hat wie weiter oben definiert. In einem aromatischen Ring A fehlen die Substituenten R und R1'; Q4 ist dann ausgewählt aus N, CH und C-R1. Umfasst Q4 ein Stickstoffatom, ist dieses bevorzugt ungeladen.

R2, R3, R5 und R6 sind in den erfindungsgemäßen Verbindungen der allgemeinen Formeln I, II, III, IV, V, VI, VII, VIII und IX bevorzugt und jeweils unabhängig voneinander ausgewählt aus der Gruppe Hydroxy, Fluor, Chlor, Brom, Trifluormethyl, Cyano, Amino, Carboxy, Sulfo, Sulfamoyl, unsubstituiertes oder mit Hydroxy substituiertes C1-C6 Alkyl, unsubstituiertes oder mit Hydroxy substituiertes C1-C6 Alkyloxy, unsubstituiertes oder mit Hydroxy substituiertes C1-C6 Alkylthio, unsubstituiertes C2-C6 Alkinyl, unsubstituiertes oder mit Fluor, Chlor oder Brom und/oder mit einer oder mehreren Methoxygruppen substituiertes Phenyl, unsubstituiertes oder mit Fluor, Chlor oder Brom und/oder mit einem oder mehreren Methoxygruppen substituiertes Phenoxy, -C(O)-C1-C6 Alkyl, wobei das Alkyl unsubstituiert oder mit Hydroxy substituiert ist, -C(O)-Phenyl, Phenylalkyloxy oder Phenylalkyloxycarbonyl, wobei das Phenyl jeweils unsubstituiert oder mit Fluor, Chlor oder Brom und/oder mit einer oder mehreren Methoxygruppen substituiert ist, C1-C6 Alkyloxycarbonyl, wobei das Alkyl unsubstituiert oder mit Hydroxy substituiert ist, C1-6 Alkylaminosulfonyl, insbesondere Methylaminosulfonyl und C1-6 Alkylsulfonylamino, insbesondere Methansulfonylamino, oder zwei benachbarte Reste R2, R3, R5 und R6 bilden gemeinsam mit den C-Atomen des Phenylrings an die sie gebunden sind, einen sauerstoffhaltigen 5-, 6- oder 7-gliedrigen Ring,
während R4 bevorzugt Wasserstoff repräsentiert.

In einer bevorzugten Ausführungsform der Erfindung ist Y in den erfindungsgemäßen Verbindungen eine Kette -(CH₂)ₚ-Z-(CH₂)ₒ-, wobei Z ausgewählt ist aus den Resten Cyclopentyl, Cyclohexyl und Cycloheptyl, und wobei p und o unabhängig voneinander ausgewählt sind aus 0,1 und 2 und zusammen einen Wert von höchstens 2 oder 1 ergeben oder beide 0 sind.

Y ist in in den Verbindungen der allgemeinen Formel I bevorzugt eine Kohlenwasserstoffkette der Formel -(CH2)_{q}- mit q=2, 3, 4 oder 5, ganz besonders bevorzugt mit n=4 oder 5. X stellt somit besonders bevorzugt eine Gruppe der allgemeinen Formel X2 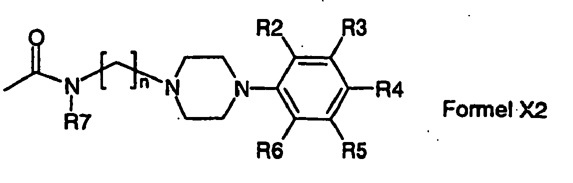 dar, in der n den Wert 4 oder 5 hat und die Substituenten R2, R3, R4, R5, R6 und R7 die Bedeutung haben, wie weiter oben beschrieben.

In einer Ausführungsform der Erfindung ist wenigstens einer der Substituenten R2, R3, R5 und R6 ein Halogenatom, insbesondere Fluor oder Chlor, während R4 bevorzugt Wasserstoff repräsentiert.

In einer anderen bevorzugten Ausführungsform steht wenigstens einer der beiden Reste R2 und R3 für einen von Wasserstoff abweichenden Substituenten, insbesondere für Alkyl, Phenyl, Alkyloxy, Phenylalkyloxy, Alkylthio, Trifluoromethyl, Cyano, eine Nitrogruppe oder ein Halogen, insbesondere Methyl, Methoxy, Ethoxy, Benzyloxy, Methylmercapto, Trifluormethyl, Cyano, Nitro, Fluor oder Chlor, besonders bevorzugt sind R2 und R3 beide Halogen, ganz besonders bevorzugt Chlor, während die Reste R4, R5 und R6 in den erfindungsgemäßen Verbindungen bzw. in Formel X1 und Formel X2 jeweils für Wasserstoff stehen.

In einer bevorzugten Ausführungsform der Erfindung ist, insbesondere wenn der Heterozyklus ein Pyrazolo[1,5-a]pyridin ist, einer der beiden Substituenten R2 oder R3 ausgewählt aus Alkyl, Phenyl, Alkyloxy, Phenylalkyloxy, Alkylthio, Trifluoromethyl, Cyano, eine Nitrogruppe oder ein Halogen, insbesondere Methyl, Methoxy, Ethoxy, Benzyloxy, Methylmercapto, Trifluormethyl, Cyano, Nitro, Fluor oder Chlor, besonders bevorzugt sind R2 und R3 beide Halogen, ganz besonders bevorzugt Chlor.

In einer weiteren bevorzugten Ausführungsform der Erfindung bilden in den Verbindungen der allgemeinen Formel I zwei benachbarte Substituenten ausgewählt aus R2, R3, R5 und R6, und insbesondere die Substituenten R2 und R3 gemeinsam mit dem Phenylrest, an den sie gebunden sind, ein Chroman oder Dihydrobenzofuran, während R4 bevorzugt Wasserstoff repräsentiert.

Eine bevorzugte Ausführungsform der Erfindung betrifft Verbindungen der allgemeinen Formel I, wobei gilt:
(a) die beiden Ringe A und B des Heteroarens weisen neben der Gruppe X höchstens 2 Substituenten R1 auf
(b) R7 ist Wasserstoff
(c) X stellt eine Gruppe der allgemeinen Formel X2 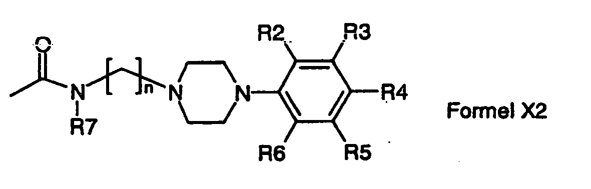 dar, in der n den Wert 4 oder 5 hat und
(d) R2, R3, R5 und R6 sind bevorzugt und jeweils unabhängig voneinander ausgewählt aus der Gruppe Hydroxy, Fluor, Chlor, Brom, Trifluormethyl, Cyano, Amino, Carboxy, Sulfo, Sulfamoyl, unsubstituiertes oder mit Hydroxy substituiertes C1-C6 Alkyl, unsubstituiertes oder mit Hydroxy substituiertes C1-C6 Alkyloxy, unsubstituiertes oder mit Hydroxy substituiertes C1-C6 Alkylthio, unsubstituiertes C2-C6 Alkinyl, unsubstituiertes oder mit Fluor, Chlor oder Brom und/oder mit einem oder mehreren Methoxygruppen substituiertes Phenoxy,-C(O)-C1-C6 Alkyl, wobei das Alkyl unsubstituiert oder mit Hydroxy substituiert ist, -C(O)-Phenyl, Phenylalkyl, Phenylalkyloxy oder Phenylalkyloxycarbonyl, wobei das Phenyl jeweils unsubstituiert oder mit Fluor, Chlor-oder Brom und/oder mit einer oder mehreren Methoxygruppen substituiert ist, C1-C6 Alkyloxycarbonyl, wobei das Alkyl unsubstituiert oder mit Hydroxy substituiert ist, C1-6 Alkylaminosulfonyl, insbesondere Methylaminosulfonyl und C1-6 Alkylsulfonylamino, insbesondere Methansulfonylamino, oder zwei benachbarte Reste R2, R3, R5 und R6 bilden gemeinsam mit den C-Atomen des Phenylrings an die sie gebunden sind, einen sauerstoffhaltigen 5-, 6- oder 7-gliedrigen Ring;
(e) R4 repräsentiert Wasserstoff; unter der Maßgabe daß, wie weiter oben beschrieben, einige Verbindungen per Proviso ausgenommen sind.

Beispiele für erfindungsgemäße Indolizin-Derivate der allgemeinen Formel I sind: 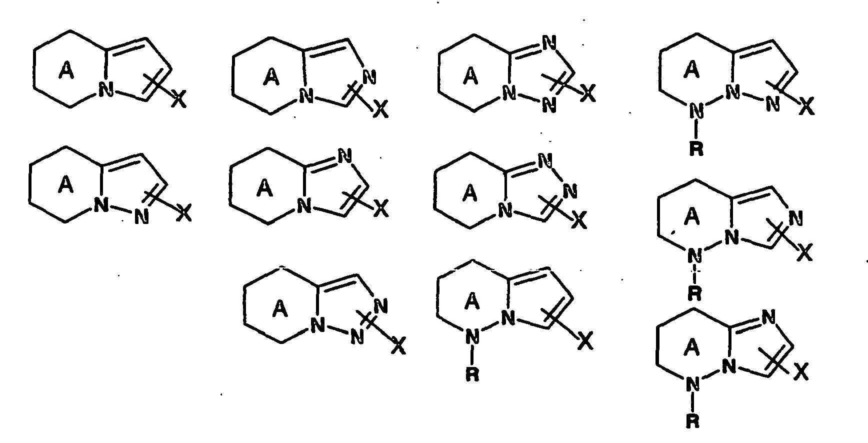 wobei gilt:
der Ring A ist jeweils gesättigt oder aromatisch;
die ringbildenden C-Atome der Ringe A und B können jeweils unabhängig voneinander mit R1 substituiert sein;
R, R1 und X haben die Bedeutung wie weiter vorstehend beschrieben.

Eine bevorzugte Ausführungsform der Erfindung betrifft Verbindungen mit der Formel II 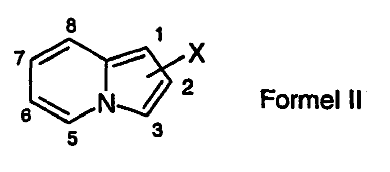 in der bedeuten:
der Indolizin-Kem Kann in den Positionen 1-3 und 5-8, wie in Formel II dargestellt, außer der Gruppe X noch einen oder mehrere, z.B.1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 1-3 oder 5-8 des Indolizins verknüpft und stellt eine Gruppe der allgemeinen Formel X1 dar 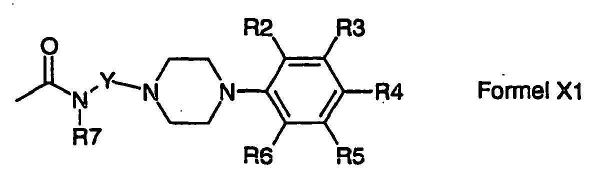 worin gilt:
   Y ist eine unverzweigte, gesättigte oder ungesättigte Kohlenwasserstoffkette mit 2-5 Kohlenstoffatomen oder eine Kette -(CH₂)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino,wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können; wobei R4 bevorzugt Wasserstoff repräsentiert.
   R7 ist Wasserstoff, Alkyl oder Phenylalkyl..

in einer Ausführungsform der Erfindung ist das Heteroaren in Formel II bis auf die Gruppe X unsubstituiert oder trägt in den Positionen 1 und/oder 2 einen oder mehrere Reste R1, wie weiter oben definiert, insbesondere Cyano oder Alkyl, z.B. Methyl.

Bevorzugt ist der Substituent X mit der 1,2 und 3-Position des Indolizins (Formel II) verknüpft.

In einer Ausführungsform der Erfindung ist Y in den Verbindungen der allgemeinen Formel II eine Kette -(CH2)ₒ-Z-(CH2)ₚ, wobei Z ausgewählt ist aus den Resten Cyclopentyl, Cyclohexyl und Cycloheptyl und wobei o und p jeweils unabhängig voneinander den Wert 0,1 oder 2 haben und bevorzugt beide zusammen einen Wert von höchstens 2 oder 1 aufweisen oder beide 0 sind.

Y ist in in den Verbindungen der allgemeinen Formel II bevorzugt eine Kohlenwasserstoffkette der Formel -(CH2)_{q}- mit q=2, 3, 4 oder 5, ganz besonders bevorzugt mit n=4 oder 5. X stellt somit in Formel II besonders bevorzugt eine Gruppe der allgemeinen Formel X2 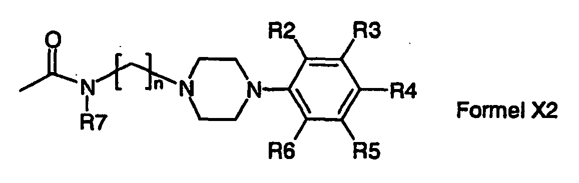 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

in einer Ausführungsform der Erfindung ist wenigstens einer der Substituenten R2, R3, R5 und R6 in den Verbindungen der allgemeinen Formel II eine C1-6 Alkyloxygruppe, z.B. ein Methoxy oder ein Halogenatom, insbesondere Fluor oder Chlor, während R4 bevorzugt Wasserstoff repräsentiert.

In einer anderen bevorzugten Ausführungsform steht wenigstens einer der beiden Reste R2 und R3 in den Verbindungen der allgemeinen Formel II für einen von Wasserstoff abweichenden Substituenten, insbesondere für Halogen oder C1-6 Alkyloxy, während die Reste R4, R5 und R6 in Formel II jeweils für Wasserstoff stehen.

In einer bevorzugten Ausführungsform der Erfindung ist einer der beiden Substituenten R2 oder R3 in den Verbindungen der allgemeinen Formel II eine C1-6 Alkyloxygruppe, insbesondere Methoxy, oder ein Halogen, insbesondere Fluor oder Chlor, besonders . bevorzugt sind R2 und R3 beide Halogen, ganz besonders bevorzugt Chlor.

In einer weiteren bevorzugten Ausführungsform der Erfindung bilden in den Verbindungen der allgemeinen Formel II zwei benachbarte Substituenten ausgewählt aus R2, R3, R5 und R6, und insbesondere die Substituenten R2 und R3 gemeinsam mit dem Phenylrest, an den sie gebunden sind, ein Chroman oder Dihydrobenzofuran, während R4 bevorzugt Wasserstoff repräsentiert.

Eine andere bevorzugte Ausführungsform der Erfindung betrifft Verbindung mit der Formel III 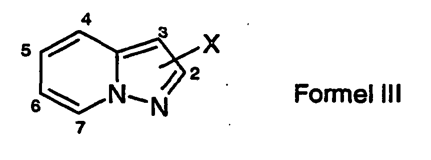 in der bedeuten:
der Pyrazolo[1,5-a]pyridin-Kern kann in den Positionen 2-7, wie in Formel III dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 2-7 des Pyrazolo[1,5-a]pyridins verknüpft und stellt eine Gruppe der allgemeinen Formel X1 dar 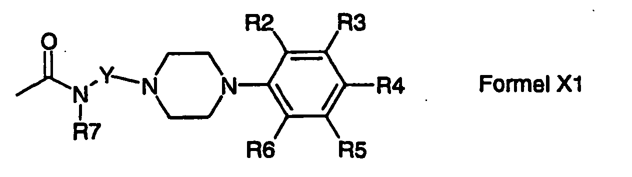 worin gilt:
   Y ist eine unverzweigte, gesättigte oder ungesättigte Kohlenwasserstoffkette mit 2-5 Kohlenstoffatomen oder eine Kette -(CH2)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino, wobei zwei benachbarte Reste R2, R3, R4, R5 und R6 gemeinsam mit den C-Atomen des Phenylrings, an die sie gebunden sind, einen sauerstoffhaltigen 5-, 6- oder 7-gliedrigen Ring bilden können, wobei R4 bevorzugt Wasserstoff repräsentiert;
   R7 ist Wasserstoff, Alkyl oder Phenylalkyl.

Bevorzugt ist die Gruppe X mit den Positionen 2, 5 oder 6 des Pyrazolo[1,5-a]pyridins der Formel III verbunden.

In einer Ausführungsform ist der Pyrazolo[1,5-a]-pyridin-Kern in mindestens einer der Positionen 5 oder 6 substituiert. In einer bevorzugten Ausführungsform der Erfindung trägt das Pyrazolo[1,5-a]pyridin in Position 5 einen Methoxy- oder CF₃-Rest und/oder in Position 6 ein Halogenatom, insbesondere wenn X an Position 2 des Heteroarens gebunden ist

In einer anderen bevorzugten Ausführungsform ist der Pyrazolo[1,5-a]-pyridin-Kern in den Verbindungen der allgemeinen Formel 111 abgesehen vom obligatorischen Substituenten X unsubstituiert, insbesondere wenn X an den Positionen 5 oder 6 des Heteroarens gebunden ist.

Y ist in den Verbindungen der allgemeinen Formel III bevorzugt eine Kohlenwasserstoffkette der Formel -(CH2)_{q}- mit q=2, 3, 4 oder 5, ganz besonders bevorzugt mit n=4 oder 5. Die Gruppe X stellt daher in Formel III besonders bevorzugt eine Gruppe der allgemeinen Formel X2 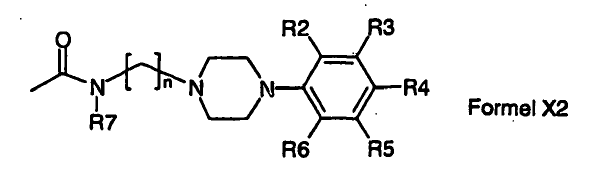 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

In einer Ausführungsform der Erfindung ist wenigstens einer der Substituenten R2, R3, R5 und R6 in den Verbindungen der allgemeinen Forme III ein Alkyl (insbesondere Methyl), Phenyl, Alkyloxy (insbesondere Methyloxy und Ethyloxy), Phenylalkyloxy (insbesondere Phenyloxy), Alkylthio (insbesondere Methylthio), Trifluoromethyl, Cyano oder eine Nitrogruppe oder ein Halogenatom, insbesondere Fluor oder Chlor, während R4 bevorzugt Wasserstoff repräsentiert.

In einer anderen bevorzugten Ausführungsform steht wenigstens einer der beiden Reste R2 und R3 in den Verbindungen der allgemeinen Formel III für einen von Wasserstoff abweichenden Substituenten, insbesondere für Halogen, Alkyl (insbesondere Methyl), Phenyl, Alkyloxy (insbesondere Methyloxy und Ethyloxy), Phenylalkyloxy (insbesondere Benzyloxy), Alkylthio (insbesondere Methylthio), Trifluoromethyl, Cyano oder Nitro, während die Reste R4, R5 und R6 jeweils für Wasserstoff stehen.

In einer bevorzugten Ausführungsform der Erfindung ist R4 Wasserstoff und einer der beiden Substituenten R2 oder R3 in den Verbindungen der allgemeinen Formel III ein Halogen, Alkyl (insbesondere Methyl), Phenyl, Alkyloxy (insbesondere Methyloxy und Ethyloxy), Phenylalkyloxy (insbesondere Benzyloxy), Alkylthio (insbesondere Methylthio), Trifluoromethyl, Cyano oder Nitro, insbesondere Fluor oder Chlor, besonders bevorzugt sind R2 und R3 beide Halogen oder Alkyl, ganz besonders bevorzugt Chlor oder Methyl.

In einer Ausführungsform der Erfindung steht R2 in den Verbindungen der allgemeinen Formel III für eine C1-6 Alkyloxygruppe, insbesondere für Methoxy, vorausgesetzt
(a) mindestens einer der Substituenten R3, R5, R6 und R7 repräsentiert einen von Wasserstoff abweichenden Rest und/oder
(b) der Pyrazolo[1,5-a]pyridin-Kern ist mit mindestens einem Substituenten R1 substituiert.

In einer anderen Ausführungsform der Erfindung ist R2 kein Methoxy. In einer anderen Ausführungsform der Erfindung ist R2 in den Verbindungen der allgemeinen Formel III kein Alkyloxy.

In einer weiteren Ausführungsform der Erfindung bilden in den Verbindungen der allgemeinen Formel III zwei benachbarte Substituenten ausgewählt aus R2, R3, R5 und R6, und insbesondere die Substituenten R2 und R3 gemeinsam mit dem Phenylrest, an den sie gebunden sind, ein Chroman oder Dihydrobenzofuran.

Eine weitere bevorzugte Ausführungsform der Erfindung sind Verbindungen mit der allgemeinen Formel IV, 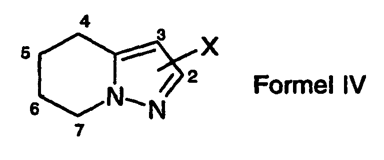 in der bedeuten:
der Tetrahydropyrazolo[1,5-a]pyridin-Kem kann In den Positionen 2-7 wie in Formel IV dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist bevorzugt mit Position 2 oder 3 des Tetrahydropyrazolo[1,5-a]pyridins verknüpft und stellt eine Gruppe der allgemeinen Formel X1 dar 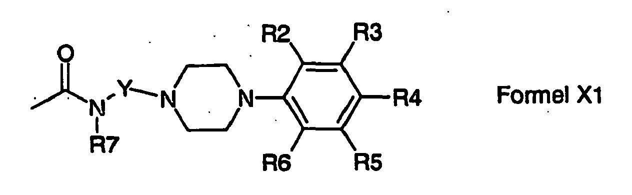 worin gilt:
   Y ist eine unverzweigte, gesättigte oder ungesättigte Kohlenwasserstoffkette mit 2-5 Kohlenstoffatomen oder eine Kette -(CH₂)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino, wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können, wobei R4 bevorzugt Wasserstoff repräsentiert;
   R7 ist Wasserstoff, Alkyl oder Phenylalkyl.

In einer Ausführungsform der Erfindung ist das Heteroaren in Formel IV bis auf die Gruppe X unsubstituiert oder trägt in den Positionen 5 und/oder 6 einen oder mehrere Reste R1, wie weiter oben definiert, insbesondere Alkyl, z.B. Methyl.

Y ist in den Verbindungen der allgemeinen Formel IV bevorzugt eine Kohlenwasserstoffkette der Formel -(CH₂)_{q}- mit q=2, 3, 4 oder 5, ganz besonders bevorzugt mit n=4 oder 5. Besonders bevorzugt stellt X somit in Formel IV eine Gruppe der allgemeinen Formel X2 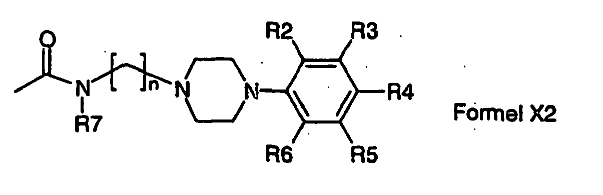 dar, in der n den Wert 4 oder 5 hat und die Substituenten R2, R3, R4, R5, R6 und R7 die Bedeutung haben, wie weiter oben beschrieben.

Bevorzugt ist R7 Wasserstoff.

In einer Ausführungsform der Erfindung ist wenigstens einer der Substituenten R2, R3, R5 und R6 in den Verbindungen der allgemeinen Formel IV eine C1-6 Alkyloxygruppe, insbesondere Methoxy, oder ein Halogenatom, insbesondere Fluor oder Chlor.

In einer anderen bevorzugten Ausführungsform steht wenigstens einer der beiden Reste R2 und R3 in den Verbindungen der allgemeinen Formel IV für einen von Wasserstoff abweichenden Substituenten, insbesondere für Halogen oder C1-C6 Alkyloxy, während die Reste R4, R5 und R6 jeweils für Wasserstoff stehen.

In einer bevorzugten Ausführungsform der Erfindung ist einer der beiden Substituenten R2 oder R3 in den Verbindungen der allgemeinen Formel IV eine C1-6 Alkyloxygruppe, insbesondere Methoxy oder Halogen, insbesondere Fluor oder Chlor, besonders bevorzugt sind R2 und R3 beide Halogen, ganz besonders bevorzugt Chlor, während R4 bevorzugt Wasserstoff repräsentiert.

In einer weiteren Ausführungsform der Erfindung bilden in den Verbindungen der allgemeinen Formel IV zwei benachbarte Substituenten ausgewählt aus R2, R3, R5 und R6, und insbesondere die Substituenten R2 und R3 gemeinsam mit dem Phenylrest, an den sie gebunden sind, ein Chroman oder Dihydrobenzofuran, während R4 bevorzugt Wasserstoff repräsentiert.

Eine andere bevorzugte Ausführungsform der Erfindung betrifft Verbindungen mit der Formel V 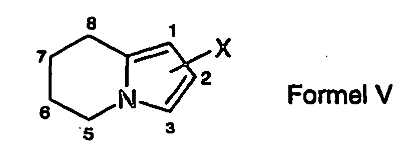 in der bedeuten:
der Tetrahydroindolizin-Kern kann in den Positionen 1-3 und 5-8, wie in Formel V dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 1-3 des Tetrahydroindolizins verknüpft und stellt eine Gruppe der allgemeinen Formel X1 dar 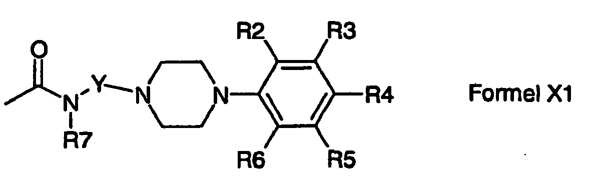 worin gilt:
   Y ist eine unverzweigte, gesättigte oder ungesättigte Kohlenwasserstoffkette mit 2-5 Kohlenstoffatomen oder eine Kette -(CH2)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino,wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können, wobei R4 bevorzugt Wasserstoff repräsentiert;
   R7 ist Wasserstoff, Alkyl oder Phenylalkyl.

In einer Ausführungsform der Erfindung ist das Heteroaren in Formel V bis auf die Gruppe X unsubstituiert.

Bevorzugt ist der Substituent X mit der 1,2 und 3-Position des Tetrahydroindolizins (Formel V) und ganz besonders bevorzugt mit Position 2 verknüpft.

Y ist in in den Verbindungen der allgemeinen Formel V bevorzugt eine Kohlenwasserstoffkette der Formel -(CH2)_{q}- mit q=2, 3, 4 oder 5, ganz besonders bevorzugt mit n=4 oder 5. X stellt somit in Formel V besonders bevorzugt eine Gruppe der allgemeinen Formel X2 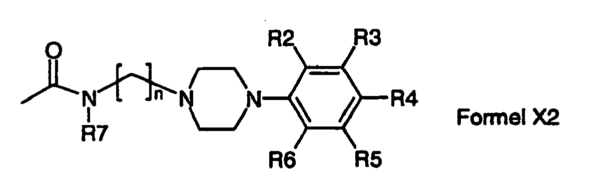 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

In einer Ausführungsform der Erfindung ist wenigstens einer der Substituenten R2, R3, R5 und R6 in den Verbindungen der allgemeinen Formel V eine C1-6 Alkyloxygruppe, z.B. ein Methoxy oder ein Halogenatom, insbesondere Fluor oder Chlor, während R4 bevorzugt Wasserstoff repräsentiert.

Eine andere bevorzugte Ausführungsform der Erfindung betrifft Verbindungen mit der Formel VI 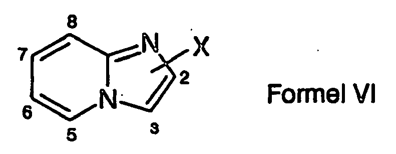 in der bedeuten:
der Heteroarenkern kann in den Positionen 2-3 und 5-8, wie in Formel VI dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 2-3 oder 5-8 des Heteroarens verknüpft und stellt eine Gruppe der allgemeinen Formel X1 dar 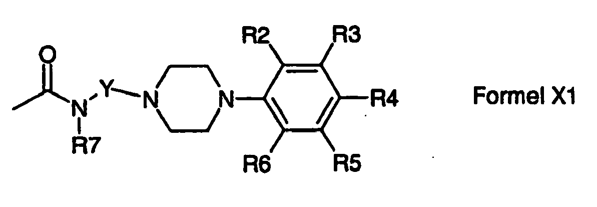 worin gilt:
   Y ist eine unverzweigte, gesättigte oder ungesättigte Kohlenwasserstoffkette mit 2-5 Kohlenstoffatomen oder eine Kette -(CH2)ₒ-Z-(CH₂)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino, wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können,
   wobei R4 bevorzugt Wasserstoff repräsentiert;
   R7 ist Wasserstoff, Alkyl oder Phenylalkyl.

In einer Ausführungsform der Erfindung ist das Heteroaren in Formel VI bis auf die Gruppe X unsubstituiert oder trägt in 2- oder 6-Position einen Rest R1 wie weiter oben definiert, insbesondere Alkyl, z.B. Methyl, oder Halogen.

Bevorzugt ist der Substituent X mit der 2,3 oder 6-Position des Heteroarens (Formel VI) verknüpft

Y ist in in den Verbindungen der allgemeinen Formel VI bevorzugt eine Kohlenwasserstoffkette der Formel -(CH2)_{q}- mit q=2, 3, 4 oder 5, ganz besonders bevorzugt mit n=4 oder 5. X stellt somit in Formel VI besonders bevorzugt eine Gruppe der allgemeinen Formel X2 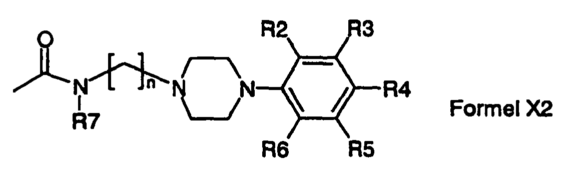 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

In einer Ausführungsform der Erfindung ist wenigstens einer der Substituenten R2, R3, R5 und R6 in den Verbindungen der allgemeinen Formel VI eine Methoxygruppe oder ein Halogenatom, insbesondere Fluor oder Chlor, während R4 bevorzugt Wasserstoff repräsentiert.

Eine andere bevorzugte Ausführungsform der Erfindung betrifft Verbindungen mit der Formel VII 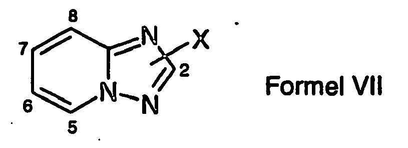 in der bedeuten:
der Heteroarenkern kann in den Positionen 2 und 5-8, wie in Formel VII dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 2 oder 5-8 des Heteroarens verknüpft und stellt eine Gruppe der allgemeinen Formel X1 dar 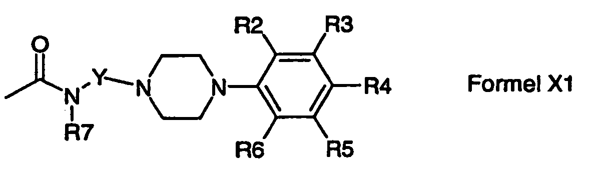 worin gilt:
   Y ist eine unverzweigte, gesättigte oder ungesättigte Kohlenwasserstoffkette mit 2-5 Kohlenstoffatomen oder eine Kette -(CH2)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl; Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Allcylsulfonylamino,wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können, wobei R4 bevorzugt Wasserstoff repräsentiert;
   R7 ist Wasserstoff, Alkyl oder Phenylalkyl.

In einer Ausführungsform der Erfindung ist das Heteroaren in Formel VII bis auf die Gruppe X unsubstituiert.

Bevorzugt ist der Substituent X mit der 2-Position des Heteroarens (Formel VII) verknüpft.

Y ist in in den Verbindungen der allgemeinen Formel VII bevorzugt eine Kohlenwasserstoffkette der Formel -(CH2)_{q}- mit q=2, 3, 4 oder 5, ganz besonders bevorzugt mit n=4 oder 5. X stellt somit in Formel VII besonders bevorzugt eine Gruppe der allgemeinen Formel X2 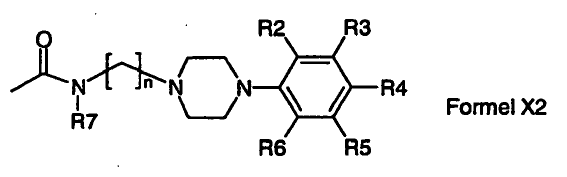 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

In einer Ausführungsform der Erfindung ist wenigstens einer der Substituenten R2, R3, R5 und R6 in den Verbindungen der allgemeinen Formel VII ein Halogenatom, insbesondere Fluor oder Chlor, während R4 bevorzugt Wasserstoff repräsentiert.

Eine bevorzugte Ausführungsform der Erfindung betrifft Verbindungen mit der Formel VIII 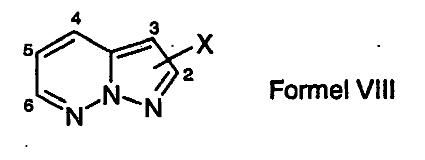 in der bedeuten:

Der Heteroarenkern kann in den Positionen 2-6, wie in Formel VIII dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 2-6 des Heteroarens verknüpft und stellt eine Gruppe der allgemeinen Formel X1 dar 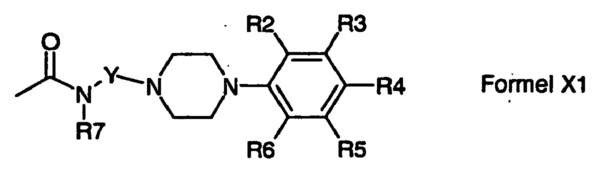 worin gilt:
Y ist eine unverzweigte, gesättigte oder ungesättigte Kohlenwasserstoffkette mit 2-5 Kohlenstoffatomen oder eine Kette -(CH₂)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino, wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können, wobei R4 bevorzugt Wasserstoff repräsentiert,
R7 ist Wasserstoff, Alkyl oder Phenylalkyl.

In einer Ausführungsform der Erfindung ist das Heteroaren in Formel VIII bis auf die Gruppe X unsubstituiert.

Bevorzugt ist der Substituent X mit der 2-Position des Heteroarens (Formel VIII) verknüpft.

Y ist in in den Verbindungen der allgemeinen Formel VIII bevorzugt eine Kohlenwasserstoffkette der Formel -(CH2)_{q}- mit q=2, 3, 4 oder 5, ganz besonders bevorzugt mit n=4 oder 5. X stellt somit in Formel VIII besonders bevorzugt eine Gruppe der allgemeinen Formel X2 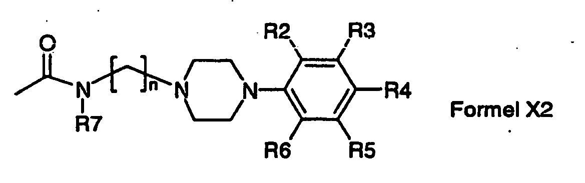 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

In einer Ausführungsform der Erfindung ist wenigstens einer der Substituenten R2, R3, R5 und R6 in den Verbindungen der allgemeinen Formel VIII ein Halogenatom, insbesondere Fluor oder Chlor, während R4 bevorzugt Wasserstoff repräsentiert.

Eine bevorzugte Ausführungsform der Erfindung betrifft Verbindungen mit der Formel IX 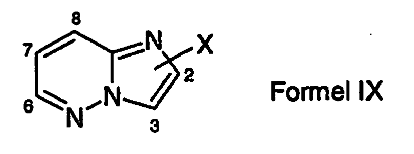 in der bedeuten:
der Heteroarenkern kann in den Positionen 2-3 und 6-8, wie in Formel IX dargestellt, außer der Gruppe X noch einen oder mehrere, z.B. 1, 2, 3 oder 4 weitere Substituenten R1 tragen, die jeweils unabhängig voneinander ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
X ist mit einer beliebigen Position 2-3 oder 6-8 des Heteroarens verknüpft und stellt eine Gruppe der allgemeinen Formel X1 dar 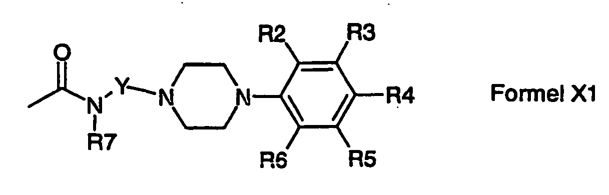 worin gilt:
   Y ist eine unverzweigte, gesättigte oder ungesättigte Kohlenwasserstoffkette mit 2-5 Kohlenstoffatomen oder eine Kette -(CH₂)ₒ-Z-(CH₂)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
   R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino,wobei zwei benachbarte Reste R2, R3, R4, R5 und R6 gemeinsam mit den C-Atomen des Phenylrings an die sie gebunden sind, einen sauerstoffhaltigen 5-, 6- oder 7-gliedrigen Ring bilden können, wobei R4 bevorzugt Wasserstoff repräsentiert,
   R7 ist Wasserstoff, Alkyl oder Phenylalkyl.

In einer Ausführungsform der Erfindung ist das Heteroaren in Formel IX bis auf die Gruppe X unsubstituiert oder trägt in Position 2 und/oder Position 6 einen Rest R1 wie weiter oben definiert, insbesondere Phenyl oder Halogen.

Bevorzugt ist der Substituent X mit der 2- oder der 3-Position des Heteroarens (Formel IX) verknüpft.

Y ist in in den Verbindungen der allgemeinen Formel IX bevorzugt eine Kohlenwasserstoffkette der Formel -(CH2)_{q}- mit q=2, 3, 4 oder 5, ganz besonders bevorzugt mit n=4 oder 5. X stellt somit in Formel IX besonders bevorzugt eine Gruppe der allgemeinen Formel X2 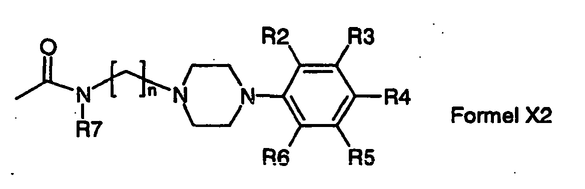 dar, in der n den Wert 4 oder 5 hat und die Substituenten R2, R3, R4, R5, R6 und R7 die Bedeutung haben, wie weiter oben beschrieben.

R7 ist bevorzugt Wasserstoff.

In einer Ausführungsform der Erfindung ist wenigstens einer der Substituenten R2, R3, R5 und R6 in den Verbindungen der allgemeinen Formel IX einen Methoxyrest oder ein Halogenatom, insbesondere Fluor oder Chlor, während R4 bevorzugt Wasserstoff repräsentiert.

Die Erfindung betrifft auch physiologisch akzeptable Salze der erfindungsgemäßen Verbindungen. Beispiele für solche Salze sind in den nachstehenden Definitionen beschrieben.

Dem Fachmann ist ferner klar, dass je nach Wahl der Substituenten geometrische Isomere und/oder optisch aktive Verbindungen entstehen können. In diesem Fall sind sowohl die Isomere, Racemate als auch die jeweiligen reinen enantiomeren bzw. gegebenenfalls diastereomeren Formen Gegenstand der vorliegenden Erfindung.

Die in der Beschreibung und in den anliegenden Patentansprüchen genannten Substituenten umfassen insbesondere die nachfolgend erläuterten Gruppen.

"Alkyl" kann eine verzweigte oder unverzweigte Alkylgruppe sein, die vorzugsweise 1 bis 10 C-Atome, besonders bevorzugt 1 bis 6 C-Atome ("C1-C6 Alkyl") und ganz besonders bevorzugt 1, 2 oder 3 C-Atome aufweist. "C1-C6 Alkyl" umfasst z.B. Methyl, Ethyl, n-Propyl, iso-Propyl, n-Butyl, iso-Butyl, s-Butyl, t-Butyl, n-Pentyl, iso-Pentyl, Neopentyl, t-Pentyl, 1-Methylbutyl, 2-Methylbutyl, 1-Ethylpropyl, 1,2-Dimethytpropyl und n-Hexyl. "Alkyl" kann auch zyklisch sein oder einen zyklischen Teil enthalten, wobei Zyklen mit 3-7 C-Atomen bevorzugt werden, z.B. Cyclopropyl, Cyclobutyl, Cyclopentyl, Cyclohexyl oder Cycloheptyl. Bevorzugt ist "Alkyl" nicht zyklisch und enthält keinen zyklischen Teil. Alkylgruppen können zusätzlich mit einem oder mehreren Substituenten substituiert sein, insbesondere mit Hydroxy oder Amin. Bevorzugt ist "Alkyl" unsubstituiert oder mit Hydroxy substituiert.

"Alkenyl" und "Alkinyl" weisen mindestens eine Doppel- bzw. Dreifachbindung auf. Sie können verzweigt oder unverzweigt sein und weisen vorzugsweise 2 bis 6 C-Atome auf. Alkenyle oder Alkinyle sind vorzugsweise so an den Heteroaren- oder Phenylring des Grundgerüsts der Verbindung gebunden, dass die Doppel- bzw. Dreifachbindung mit dem aromatischen Ring konjugiert ist. Alkenyl und Alkinyl können zusätzlich mit einem oder mehreren Substituenten substituiert sein, vorzugsweise mit Phenyl, wobei sich die Phenylgruppe dann besonders bevorzugt am C-Atom 2 befindet (wenn Alkenyl oder Alkinyl über das C-Atom 1 an den Heteroaren- oder Phenylring des Grundgerüsts gebunden ist). Bevorzugt sind die Alkenyle oder Alkinyle unsubstituiert.

"Alkyloxy" ist die Gruppe -O-Alkyl, worin Alkyl vorzugsweise aus den oben für "Alkyl" angegebenen Gruppen ausgewählt ist. Bevorzugt ist "Alkyloxy" eine C1-C6-Alkyloxygruppe, besonders bevorzugt Methoxy.

"Alkylthio" kann auch als "Alkylmercapto" bezeichnet werden und ist die Gruppe -S-Alkyl, worin Alkyl vorzugsweise aus den oben für "Alkyl" angegebenen Gruppen ausgewählt ist. Bevorzugt ist "Alkylthio" eine C1-C6-Alkyl-S-Gruppe.
"Alkylaminosulfonyl" umfasst die Gruppen -SO₂-NH-Alkyl und -SO₂-N-Dialkyl, worin Alkyl vorzugsweise aus den oben für "Alkyl" angegebenen Gruppen ausgewählt ist. Bevorzugt ist "Alkyl" in "Alkylaminosulfonyl" eine C1-C6-Alkylgruppe. Beispiele für "Alkylaminosulfonyl" sind z.B. Methylaminosulfonyl, N,N-Dimethylaminosulfonyl oder Butylaminosulfonyl.

"Alkylsulfonylamino" ist die Gruppe -NH-SO₂-Alkyl, worin Alkyl vorzugsweise aus den oben für "Alkyl" angegebenen Gruppen ausgewählt ist. Bevorzugt ist "Alkylsulfonylamino" eine C1-C6-Alkylsulfonylaminogruppe, z.B. Methansulfonylamino.

"Phenyl" ist bevorzugt unsubstituiert, kann aber gegebenenfalls ein oder mehrfach unabhängig substituiert sein, z.B. mit Alkoxy, Alkyl, Trifluormethyl oder Halogen.

"Phenylalkyl" ist die Gruppe -Alkyl-Phenyl, wobei Phenyl und Alkyl die Bedeutung haben, wie vorstehend definiert. Phenylalkyl umfasst beispielsweise Phenylethyl und Benzyl und ist bevorzugt Benzyl.

"Phenoxy" ist die Gruppe -O-Phenyl, worin Phenyl die Bedeutung hat, wie weiter vorstehend definiert.

"Phenylalkyloxy" ist die Gruppe Phenylalkyl-O-, worin Phenylalkyl die Bedeutung hat, wie weiter vorstehend definiert.

"Alkylcarbonyl" umfasst die Gruppe -C(O)-Alkyl, worin Alkyl vorzugsweise aus den oben für "Alkyl" angegebenen Gruppen ausgewählt ist, und besonders bevorzugt -C(O)-C1-C6-Alkyl ist. "Alkylcarbonyl" ist vorzugsweise Acetyl, Propionyl oder Butyryl.

"Phenylcarbonyl" ist -C(O)-Phenyl, worin Phenyl die Bedeutung hat, wie weiter oben definiert

"Alkyloxycarbonyl" ist die Gruppe -C(O)-O-Alkyl, worin Alkyl vorzugsweise aus den oben für "Alkyl" angegebenen Gruppen ausgewählt ist. Bevorzugt ist "Alkoxycarbonyl" eine (C1-C6-Alkyl)oxycarbonylgruppe.

"Phenylalkyloxycarbonyl" ist die Gruppe Phenylalkyl-O-C(O)-, worin Phenylalkyl die Bedeutung hat, wie weiter vorstehend definiert.

"Halogen" umfasst Fluor, Chlor, Brom und Iod, und ist bevorzugt Fluor, Chlor oder Brom.

"Sulfamoyl" umfasst die Gruppe -SO₂-NH₂.

"Sulfonylamino" umfasst die Gruppe -NH-SO₂H.

"Physiologisch akzeptable Salze" schließen nicht-toxische Additionssalze einer Base, insbesondere einer Verbindung der Formeln (I) bis (IV) in Form der freien Base, mit organischen oder anorganischen Säuren ein. Beispiele für anorganische Säuren schließen HCl, HBr, Schwefelsäure und Phosphorsäure ein. Organische Säuren schließen Essigsäure, Propionsäure, Brenztraubensäure, Buttersäure, α-, β- oder γ-Hydroxybuttersäure, Valeriansäure, Hydroxyvaleriansäure, Capronsäure, Hydroxycapronsäure, Caprylsäure, Caprinsäure, Laurinsäure, Myristinsäure, Palmitinsäure, Stearinsäure, Glykolsäure, Milchsäure, D-Glucuronsäure, L-Glucuronsäure, D-Galacturonsäure, Glycin, Benzoesäure, Hydroxybenzoesäure, Gallussäure, Salicylsäure, Vanillinsäure, Cumarsäure, Kaffeesäure, Hippursäure, Orotsäure, L-Weinsäure, D-Weinsäure, D,L-Weinsäure, meso-Weinsäure, Fumarsäure, L-Äpfelsäure, D-Äpfelsäure, D,L-Äpfelsäure, Oxalsäure, Malonsäure, Bernsteinsäure, Maleinsäure, Oxalessigsäure, Glutarsäure, Hydroxyglutarsäure, Ketoglutarsäure, Adipinsäure, Ketoadipinsäure, Pimelinsäure, Glutaminsäure, Asparaginsäure, Phthalsäure, Propantricarbonsäure, Zitronensäure, Isozitronensäure, Methansulfonsäure, Toluolsulfonsäure, Benzolsulfonsäure, Camphersulfonsäure, Embonsäure und Trifluormethansulfonsäure ein.

Die folgenden Verbindungen stellen konkrete Ausführungsformen der erfindungsgemäßen Verbindungen dar:
(B69): N-4-(4-(2-Methoxyphenyl)piperazin-1-yl)butylindolizin-1-ylcarbamid
(B1): N-4-(4-(2-Methoxyphenyl)piperazin-1-yl)butylindolizin-2-ylcarbamid
(B2): N-4-(4-(3-Chlor-2-methoxyphenyl)piperazin-1-yl)butylindolizin-2-ylcarbamid
(B3): N-4-(4-(2,3-Dichlorphenyl)piperazin-1-yl)butylindolizin-2-ylcarbamid
(B4): N-4-(4-(2,3-Difluorphenyl)piperazin-1-yl)butylindolizin-2-ylcarbamid
(B5): N-4-(4-(2,3-Dihydrobenzofuran-7-yl)piperazin-1-yl)butylindolizin-2-ylcarbamid
(B49): N-4-(4-(Chroman-8-yl)piperazin-1-yl)butylindolizin-2-ylcarbamid
(B70): N-4-(4-(2-Methoxyphenyl)piperazin-1-yl)butyl-5,6,7,8-tetrahydroindolizin-2-ylcarbamid
(B71): N-4-(4-(2,3-Dichlorphenyl)piperazin-1-yl)butyl-5,6,7,8-tetrahydroindolizin-2-ylcarbamid
(B72): N-4-(4-(2-Methoxyphenyl)piperazin-1-yl)butyl-1-cyano-2-methylindolizin-3-ylcarbamid
(B6): N-2-(4-(2,3-Dichlorphenyl)piperazin-1-yl)ethylpyrazolo[1,5-*a*]pyridin-2-ylcarbamid
(B7): N-3-(4-(2,3-Dichlorphenyl)piperazin-1-yl)propylpyrazolo[1,5-*a*]pyridin-2-ylcarbamid
(B73): N-4-(4-Phenylpiperazin-1-yl)butylpyrazolo[1,5-*a*]pyridin-2-ylcarbamid
(B74): N-4-(4-(2-Methylphenyl)piperazin-1-yl)butylpyrazolo[1,5-*a*]pyridin-2-ylcarbamid
(B75): N-4-(4-(2-Biphenyl)piperazin-1-yl)butylpyrazolo[1,5-*a*]pyridin-2-ylcarbamid
(B76): N-4-(4-(2-Ethoxyphenyl)piperazin-1-yl)butylpyrazolo[1,5-*a*]pyridin-2-ylcarbamid
(B77): N-4-(4-(2-Benzyloxyphenyl)piperazin-1-yl)butylpyrazolo[1,5-*a*]pyridin-2-ylcarbamid
(B78): N-4-(4-(2-Methylmercaptophenyl)piperazin-1-yl)butylpyrazolo[1,5-*a*]pyridin-2-ylcarbamid
(B79): N-4-(4-(2-Fluorphenyl)piperazin-1-yl)butylpyrazolo[1,5-*a*]pyridin-2-ylcarbamid
(B80): N-4-(4-(2-Trifluormethylphenyl)piperazin-1-yl)butylpyrazolo[1,5-*a*]pyridin-2-ylcarbamid
(B81): N-4-(4-(2-Cyanophenyl)piperazin-1-yl)butylpyrazolo[1,5-a]pyridin-2-ylcarbamid
(B82): N-4-(4-(2-Nitrophenyl)piperazin-1-yl)butylpyrazolo[1,5-*a*]pyridin-2-ylcarbamid
(B83): N-4-(4-(4-Methoxyphenyl)piperazin-1-yl)butylpyrazolo[1,5-*a*]pyridin-2-ylcarbamid
(B84): N-4-(4-(3-Chlor-2-methoxyphenyl)piperazin-1-yl)butylpyrazolo[1,5-*a*]pyridin-2-ylcarbamid
(B8): N-4-(4-(2,3-Dichlorphenyl)piperazin-1-yl)butylpyrazolo[1,5-*a*]pyridin-2-ylcarbamid
(B85): N-4-(4-(2,3-Dimethylphenyl)piperazin-1-yl)butylpyrazolo[1,5-*a*]pyridin-2-ylcarbamid
(B86): N-4-(4-(2,3-Dihydrobenzofuran-7-yl)piperazin-1-yl)butylpyrazolo[1,5-*a*]pyridin-2-ylcarbamid
(B87): N-4-(4-(Chroman-8-yl)piperazin-1-yl)butylpyrazolo[1,5-*a*]pyridin-2-ylcarbamid
(B88): N-4-(4-(2,4-Dimethoxyphenyl)piperazin-1-yl)butylpyrazolo[1,5-*a*]pyridin-2-ylcarbamid
(B10): N-4-(4-(2-Methoxyphenyl)piperazin-1-yl)butyl-3-brompyrazolo[1,5-*a*]pyridin-2-ylcarbamid
(B11): N-4-(4-(2-Methoxyphenyl)piperazin-1-yl)butyl-3-chlorpyrazolo[1,5-*a*]pyridin-2-ylcarbamid
(B50): N-4-(4-(2,3-Dichlorphenyl)piperazin-1-yl)butyl-5-methoxypyrazolo[1,5-*a*]pyridin-2-ylcarbamid
(B51): N-4-(4-(2-Methoxyphenyl)piperazin-1-yl)butyl-5-methoxypyrazolo[1,5-*a*]pyridin-2-ylcarbamid
(B12): N-4-(4-(2-Methoxyphenyl)piperazin-1-yl)butyl-5-methylpyrazolo[1,5-*a*]pyridin-2-ylcarbamid
(B52): N-4-(4-(2,3-Dichlorphenyl)piperazin-1-yl)butyl-5-trifluormethylpyrazolo[1,5-*a*]pyridin-2-ylcarbamid
(B53): N-4-(4-(2-Methoxyphenyl)piperazin-1-yl)butyl-5-trifluormethylpyrazolo[1,5-*a*]pyridin-2-ylcarbamid
(B54): N-4-(4-(2,3-Dichlorphenyl)piperazin-1-yl)butyl-6-brompyrazolo[1,5-*a*]pyridin-2-ylcarbamid
(B13): N-4-(4-(2-Methoxyphenyl)piperazin-1-yl)butyl-6-brompyrazolo[1,5-*a*]pyridin-2-ylcarbamid
(B55): N-4-(4-(2,3-Dichlorphenyl)piperazin-1-yl)butyl-6-chlorpyrazolo[1,5-*a*]pyridin-2-ylcarbamid
(B56): N-4-(4-(2-Methoxyphenyl)piperazin-1-yl)butyl-6-chlorpyrazolo[1,5-a]pyridin-2-ylcarbamid
(B57): N-4-(4-(2,3-Dichlorphenyl)piperazin-1-yl)butyl-6-fluorpyrazolo[1,5-*a*]pyridin-2-ylcarbamid
(B58): N-4-(4-(2-Methoxyphenyl)piperazin-1-yl)butyl-6-fluorpyrazolo[1,5-*a*]pyridin-2-ylcarbamid
(B14): N-4-(4-(2-Methoxyphenyl)piperazin-1-yl)butyl-3-methoxycarbonylpyrazolo[1,5-*a*] pyridin-2-ylcarbamid
(B15): N-4-(4-(2,3-Dichlorphenyl)piperazin-1-yl)butyl-3-methoxycarbonylpyrazolo[1,5-*a*] pyridin-2-ylcarbamid
(B9): N-5-(4-(2,3-Dichlorphenyl)piperazin-1-yl)pentylpyrazolo[1,5-*a*]pyridin-2-ylcarbamid
(B59): N-5-(4-(2,3-Dichlorphenyl)piperazin-1-yl)pentyl-5-methoxypyrazolo[1,5-*a*]pyridin-2-ylcarbamid
(B60): N-5-(4-(2-Methoxyphenyl)piperazin-1-yl)pentyl-5-methoxypyrazolo[1,5-*a*]pyridin-2-ylcarbamid
(B61): N-5-(4-(2,3-Dichlorphenyl)piperazin-1-yl)pentyl-5-trifluormethylpyrazofo[1,5-*a*]pyridin-2-ylcarbamid
(B62): N-5-(4-(2-Methoxyphenyl)piperazin-1-yl)pentyl-5-trifluormethylpyrazolo[1,5-*a*]pyridin-2-ylcarbamid
(B63): N-5-(4-(2,3-Dichlorphenyl)piperazin-1-yl)pentyl-6-brompyrazolo[1,5-*a*]pyridin-2-ylcarbamid
(B64): N-5-(4-(2-Methoxyphenyl)piperazin-1-yl)pentyl-6-brompyrazolo[1,5-*a*]pyridin-2-ylcarbamid
(B65): N-5-(4-(2,3-Dichlorphenyl)piperazin-1-yl)pentyl-6-chlorpyrazolo[1,5-*a*]pyridin-2-ylcarbamid
(B66): N-5-(4-(2-Methoxyphenyl)piperazin-1-yl)pentyl-6-chlorpyrazolo[1,5-*a*]pyridin-2-ylcarbamid
(B67): N-5-(4-(2,3-Dichlorphenyl)piperazin-1-yl)pentyl-6-fluorpyrazolo[1,5-*a*]pyridin-2-ylcarbamid
(B68): N-5-(4-(2-Methoxyphenyl)piperazin-1-yl)pentyl-6-fluorpyrazolo[1,5-*a*]pyndin-2-ylcarbamid
(B16): *trans*-N-(4-((4-(2-Methoxyphenyl)piperazin-1-yl)methyl)cyclohex-1-yl)methyl-pyrazolo[1,5-*a*]pyridin-2-ylcarbamid
(B17): *trans*-N-(4-((4-(2,3-Dichlorphenyl)piperazin-1-yl)methyl)cyclohex-1-yl)methyl-pyrazolo[1,5-*a*]pyridin-2-ylcarbamid
(B18): N-2-(4-(2,3-Dichlorphenyl)piperazin-1-yl)ethylpyrazolo[1,5-*a*]pyridin-3-ylcarbamid
(B19): N-3-(4-(2,3-Dichlorphenyl)piperazin-1-yl)propylpyrazolo[1,5-*a*]pyridin-3-ylcarbamid
(B20): N-4-(4-(2,3-Dichlorphenyl)piperazin-1-yl)butylpyrazoto[1,5-*a*]pyridin-3-ylcarbamid
(B21): N-5-(4-(2,3-Dichlorphenyl)piperazin-1-yl)pentylpyrazolo[1,5-*a*]pyridin-3-ylcarbamid
(B22): *trans*-N-(4-((4-(2-Methoxyphenyl)piperazin-1-yl)methyl)cyclohex-1-yl)methyl-pyrazolo[1,5-*a*]pyridin-3-ylcarbamid
(B23): *trans*-N-(4-((4-(2,3-Dichlorphenyl)piperazin-1-yl)methyl)cyclohex-1-yl)methyl-pyrazolo[1,5-*a*]pyridin-3-ylcarbamid
(B24): N-2-(4-(2,3-Dichlorphenyl)piperazin-1-yl)ethylpyrazolo[1,5-*a*]pyridin-5-ylcarbamid
(B25): N-3-(4-(2,3-Dichlorphenyl)piperazin-1-yl)propylpyrazolo[1,5-*a*]pyridin-5-ylcarbamid
(B26): N-4-(4-(2,3-Dichlorphenyl)piperazin-1-yl)butylpyrazolo[1,5-*a*]pyridin-5-ylcarbamid
(B27): N-5-(4-(2,3-Dichlorphenyl)piperazin-1-yl)pentylpyrazolo[1,5-*a*]pyddin-5-ylcarbamid
(B28): N-4-(4-(2-Methoxyphenyl)piperazin-1-yl)butyl-3-brompyrazolo[1,5-*a*]pyridin-5-ylcarbamid
(B29): N-4-(4-(2-Methoxyphenyl)piperazin-1-yl)butyl-3-chlorpyrazolo[1,5-*a*]pyridin-5-ylcarbamid
(B30): N-5-(4-(2-Methoxyphenyl)piperazin-1-yl)pentylpyrazolo[1,5-*a*]pyridin-5-ylcarbamid
(B31): N-2-(4-(2,3-Dichlorphenyl)piperazin-1-yl)ethylpyrazolo[1,5-*a*]pyridin-6-ylcarbamid
(B32): N-3-(4-(2,3-Dichlorphenyl)piperazin-1-yl)propylpyrazolo[1,5-*a*]pyridin-6-ylcarbamid
(B33): N-4-(4-(2,3-Dichlorphenyl)piperazin-1-yl)butylpyrazolo[1,5-*a*]pyridin-6-ylcarbamid
(B34): N-5-(4-(2,3-Dichlorphenyl)piperazin-1-yl)pentylpyrazolo[1,5-*a*]pyridin-6-ylcarbamid
(B35): N-2-(4-(2,3-Dichlorphenyl)piperazin-1-yl)ethyl-4,5,6,7-tetrahydropyrazolo[1,5-*a*] pyridin-2-ylcarbamid
(B36): N-3-(4-(2,3-Dichlorphenyl)piperazin-1-yl)propyl-4,5,6,7-tetrahydropyrazolo[1,5-*a*] pyridin-2-ylcarbamid
(B37): N-4-(4-(2,3-Dichlorphenyl)piperazin-1-yl)butyl-4,5,6,7-tetrahydropyrazolo[1,5-*a*] pyridin-2-ylcarbamid
(B39): N-4-(4-(2-Methoxyphenyl)piperazin-1-yl)butyl-4,5,6,7-tetrahydropyrazolo[1,5-*a*]pyridin-2-ylcarbamid
(B40): N-4-(4-(2-Methoxyphenyl)piperazin-1-yl)butyl-5-methyl-4,5,6,7-tetrahydropyrazolo[1,5-*a*]pyridin-2-ylcarbamid
(B89): N-5-(4-(2-Methoxyphenyl)piperazin-1-yl)pentyl-4,5,6,7-tetrahydropyrazolo[1,5-*a*] pyridin-2-ylcarbamid
(B38): N-5-(4-(2,3-Dichlorphenyl)piperazin-1-yl)pentyl-4,5,6,7-tetrahydropyrazolo[1,5-*a*] pyridin-2-ylcarbamid
(B41): N-2-(4-(2,3-Dichlorphenyl)piperazin-1-yl)ethyl-4,5,6,7-tetrahydropyrazolo[1,5-*a*] pyridin-3-ylcarbamid
(B42): N-3-(4-(2,3-Dichlorphenyl)piperazin-1-yl)propyl-4,5,6,7-tetrahydropyrazolo[1,5-a] pyridin-3-ylcarbamid
(B43): N-4-(4-(2,3-Dichlorphenyl)piperazin-1-yl)butyl-4,5,6,7-tetrahydropyrazolo[1,5-*a*]pyridin-3-ylcarbamid
(B44): N-5-(4-(2,3-Dichlorphenyl)piperazin-1-yl)pentyl-4,5,6,7-tetrahydropyrazolo[1,5-*a*] pyridin-3-ylcarbamid
(B90): N-4-(4-(2-Methoxyphenyl)piperazin-1-yl)butyl-6-chlorimidazo[1,2-*a*]pyridin-2-ylcarbamid
(B91): N-4-(4-(2,3-Dichlorphenyl)piperazin-1-yl)butyl-6-chlorimidazo[1,2-*a*]pyridin-2-ylcarbamid
(B92): N-4-(4-(2-Methoxyphenyl)piperazin-1-yl)butyl-6-chlor-2-methylimidazo[1,2-*a*]pyridin-3-ylcarbamid
(B93): N-4-(4-(2-Methoxyphenyl)piperazin-1-yl)butylimidazo[1,2-*a*]pyridin-6-ylcarbamid
(B94): N-4-(4-(2,3-Dichlorphenyl)piperazin-1-yl)butyl-1,2,4-triazolo[1,5-*a*]pyridin-2-ylcarbamid
(B95): N-4-(4-(2,3-Dichlorphenyl)piperazin-1-yl)butylpyrazolo[1,5-*b*]pyridazin-2-ylcarbamid
(B96): N-4-(4-(2-Methoxyphenyl)piperazin-1-yl)butyl-6-chlorimidazo[1,2-*b*]pyridazin-2-ylcarbamid
(B97): N-4-(4-(2-Methoxyphenyl)piperazin-1-yl)butyl-6-chlor-2-phenylimidazo[1,2-*b*]pyridazin-3-ylcarbamid
sowie pharmazeutisch akzeptable Salze dieser Verbindungen.

Verbindungen der Formeln (I), (II), (III), (IV), (V), (VI), (VII), (VIII) und (IX) wie definiert, sind als Arzneimittel geeignet. Die erfindungsgemäßen Verbindungen umfassen affine oder sogar hochaffine Liganden für D3-Rezeptoren.

Der Begriff "affiner D3-Ligand" umfasst Verbindungen, die in einem Radioligandexperiment Bindung (vgl. Hübner, H. et al. J. Med. Chem. 2000, 43, 756-762 sowie der Abschnitt "Biologische Aktivität") an humane Dopamin D3-Rezeptoren mit einem Ki-Wert von nicht mehr als 500 nM zeigen Für "affine" Liganden anderer Rezeptoren gilt die Definition entsprechend.

Der Begriff "hochaffine D3-Liganden" umfasst Verbindungen, die in einem Radioligandexperiment (vgl. Hübner, H. et al. J. Med. Chem. 2000, 43, 756-762 sowie der Abschnitt "Biologische Aktivität") Bindung an humane Dopamin D3-Rezeptoren mit einem Ki-Wert von vorzugsweise nicht mehr als etwa 30 nM, besonders bevorzugt nicht mehr als 3 nM zeigen. Für "hochaffine" Liganden anderer Razeptoren gilt die Definition entsprechend.

Ein Aspekt der vorliegenden Erfindung betrifft selektive D3-Liganden. Der Begriff "selektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 10 niedriger als für mindestens fünf der sieben folgenden Rezeptoren ist: Dopamin-Rezeptoren D1, D2long, D2short und D4.4, Serotonin-Rezeptoren 5-HT1A und 5-HT2 und Alpha 1 Adrenozeptor.

Ein anderer Aspekt der Erfindung betrifft hochselektive Dopamin D3-Liganden. Der Begriff "hochselektive D3-Liganden° umfasst Verbindungen, die im Radioligandexperiment für den D3-Rezeptor, wie im nachfolgenden Abschnitt "Biologische Aktivität° beschrieben, einen Ki-Wert aufweisen, der um einen Faktor von zumindest 100 niedriger als für mindestens drei, bevorzugt für alle der Dopamin-Rezeptoren D1, D2long, D2short und D4.4 ist

D3-Liganden können am D3-Rezeptor agonistische, antagonistische oder partialagonistische Wirkung haben. Die entsprechenden intrinsischen Aktivitäten der erfindungsgemäßen Verbindungen lassen sich in Mitogeneseassays messen, wie in der Literatur beschrieben (Hübner, H. et al. J. Med. Chem. 2000, 43, 4563-4569 und Löber, S. Bioorg. Med. Chem. Lett. 2002, 12.17, 2377-2380). In Abhängigkeit von der Pathophysiologie der zugrunde liegenden Erkrankung kann therapeutisch eine stärker agonistische, stärker antagonistische oder eine partiaiagonistische Aktivität gewünscht, sein.

Schließlich weisen einige der erfindungsgemäßen Substanzen auch signifikante Affinität für weitere pharmakologisch interessante Rezeptoren, wie z.B. den Serotonin-Rezeptor, insbesondere den 5-HT1 a-Rezeptor, oder den Dopamin D2-Rezeptor auf.

Anstelle einer hochselektiven Dopamin D3-Rezeptorbindung kann je nach Art der zu behandelnen Erkrankung auch eine Bindung an einen weiteren Rezeptor gewünscht sein.

Beispielsweise kann zur Behandlung der Schizophrenie eine Verbindung attraktiv sein, die ein hochaffiner D3-Ligand und gleichzeitig ein affiner oder sogar hochaffiner 5-HT1a-Rezeptorligand ist. In einer anderen Ausführungsform der Erfindung kann zur Behandlung von Dyskinesien eine Verbindung gewünscht sein, die neben D3-modulatorischen Eigenschaften auch D2-agonistische und 5-HT1 a-modulatorische Eigenschaften aufweist. In anderen Fällen, z.B. bei der Behandlung der urinalen Inkontinenz kann sogar eine stärkere Selektivität für den Serotonin-Rezeptor wünschenswert sein.

Die vorliegende Erfindung erlaubt daher in exzellenter Weise eine Feineinstellung der gewünschten Affinität, Aktivität und Selektivität bezüglich verschiedener pharmakologisch bedeutsamer Rezeptoren insbesondere der Dopamin D3- Rezeptoren, aber auch beispielsweise bezüglich des 5-HT1 a-Rezeptors oder des D2-Rezeptors.

Ein weiterer Gegenstand der Erfindung ist daher ein Arzneimittel, das eine oder mehrere der Verbindungen der allgemeinen Formeln (I), (II), (III), (IV), (V), (VI), (VII), (VIII) und (IX) oder eine der konkret aufgeführten Verbindungen wie oben definiert, gegebenenfalls in Form eines pharmazeutisch akzeptablen Salzes sowie eines pharmazeutisch akzeptablen Hilfsmittels enthält.

Die Erfindung betrifft auch die Verwendung einer oder mehrerer der Verbindungen der allgemeinen Formeln (I), (II), (III), (IV), (V), (VI), (VII), (VIII) und (IX) oder einer der konkret aufgeführten Verbindungen, gegebenenfalls in Form eines pharmazeutisch akzeptablen Salzes, zur Behandlung, der hier genannten Indikationen sowie zur Herstellung eines Arzneimittels für die hier genannten Indikationen.

Der Begriff "Behandlung" einer Erkrankung umfasst in dieser Patentanmeldung (a) die Therapie einer bereits bestehenden Erkrankung sowie (b) die Prophylaxe einer noch nicht oder noch nicht vollständig ausgeprägten Erkrankung, wenn für das Auftreten einer solchen Erkrankung ein Risiko besteht.

Bevorzugt werden zur Herstellung von Arzneimittel solche erfindungsgemäßen Verbindungen ausgewählt, die hochaffine D3-Liganden sind. Besonders bevorzugt werden selektive oder sogar hochselektive D3-Liganden verwendet.

In einer anderen Ausführungsform der Erfindung werden Verbindungen ausgewählt, die affin oder sogar hochaffin auch oder insbesondere für den 5-HT1 a-Rezeptor sind.

Die erfindungsgemäßen Verbindungen haben Potential in der Therapie oder Prophylaxe einer Reihe von Erkrankungen, die insbesondere mit einer Störung des Dopaminstoffwechsels oder der dopaminergen Signalkaskade, bzw. gegebenenfalls der serotonergen Signalübertragung einhergehen.

Ein Gegenstand der Erfindung ist daher die Verwendung einer erfindungsgemäßen Verbindung, wie in dieser Patentanmeldung inklusive der Ansprüche und den Beispielen beschrieben, zur Herstellung eines Arzneimittels zur Behandlung von Erkrankungen, die mit einer Störung des Dopaminstoffwechsels und/oder der dopaminergen Signalkaskade einhergehen.

Ein anderer Gegenstand der Erfindung ist die Verwendung einer erfindungsgemäßen Verbindung, wie in dieser Patentanmeldung inklusive der Ansprüche und den Beispielen beschrieben, zur Herstellung eines Arzneimittels zur Behandlung von Erkrankungen, die mit einer Störung des Serotoninstoffwechsels und/oder der serotonergen Signalübertragung einhergehen.

Erkrankungen, in deren Pathogenese dopaminerge und/oder serotonerge Prozesse involviert sind, sind insbesondere Erkrankungen des zentralen Nervensystems (ZNS). Ein Gegenstand der Erfindung ist daher die Verwendung einer erfindungsgemäßen Verbindung, wie in dieser Patentanmeldung inklusive der Ansprüche und den Beispielen beschrieben, zur Herstellung eines Arzneimittels zur Behandlung von ZNS-Erkrankungen.

Der Begriff "ZNS-Erkrankungen" umfasst in dieser Patentanmeldung sowohl Störungen, die ihren Ursprung im ZNS haben und deren Symptome sich überwiegend oder ausschließlich im ZNS bemerkbar machen, wie z.B. Psychosen, Depressionen oder kognitive Störungen, als auch Erkrankungen, die ihren Ursprung im ZNS haben, deren Symptome sich aber zumindestens zum Teil in anderen Zielorganen bemerkbar machen, wie z.B. extrapyramidal-motorische Bewegungsstörungen oder Hyperprolaktinämie.

Beispiele für ZNS-Erkrankungen, die mit den erfindungsgemäßen Verbindungen behandelt werden können, sind
(1) Psychosen und Angststörungen, inklusive Manien, idiopathischen Psychosen, Schizophrenie, Zwangsstörungen, Panikattacken, Phobien, Essstörungen, aggressive und autoagressive Störungen, Stereotypien und andere Persönlichkeitsstörungen
(2) Drogenabhängigkeit, z.B. Kokain-, Alkohol-, Oplat- und Nikotinsucht;
(3) Stimmungsstörungen, z.B. depressive Störungen insbesondere "major depression", manisch-depressive Störungen, organisch-bedingte Depressionen, z.B. im Zusammenhang mit neurodegenerativen Erkrankungen wie Morbus Parkinson oder Alzheimer
(4) Bewegungsstörungen, inklusive Tremor, Rigor, Dyskinesien, Dystonien, wie bei Morbus Parkinson, Parkinsonismus (idiopathisch, z.B. bei Parkinson-Plus-Syndrom oder Medikamenten-induziert, z.B. nach L-Dopa oder Neuroleptika-Behandlung), Segawa-Syndrom, Tourette-Syndrom, Restless-Leg Syndrom
(5) Schlafstörungen, inklusive durch Dopaminagonisten ausgelöste Narkolepsie oder Morbus Parkinson-assoziierte Schlafstörungen
(6) Übelkeit: hier können Dopaminantagonisten entweder alleine oder in Kombination mit 5-HT3 Antagonisten eingesetzt werden
(7) Kognitive Störungen und Demenzerkrankungen
(8) Hyperprolaktinämie; Hyperprolaktinom sowie bei Medikamenten-unterstütztem Abstillen nach Schwangerschaften
(9) Glaukoma
(10) Hyperaktivitätssyndrom (ADHS);
(11) Autismus, bzw. mit Autismus verbundene Störungen, insbesondere bei Verbindungen mit ausgeprägter serotonerger Wirkkomponente
(12) Schlaganfall, insbesondere bei Verbindungen, mit ausgeprägter serotonerger Wirkkomponente

Als weitere therapeutische Anwendung kann die Behandlung und Vorbeugung von neurodegenerativen Erkrankungen genannt werden, da die Substanzen auf Grund ihrer neuroprotektiven Wirkung die Zerstörung oder den Verlust von Neuronen als Ursache oder Folge eines pathophysiologischen Ereignisses verzögern oder zum Stillstand bringen können. Solche Erkrankungen sind beispielsweise die amyotrope Lateralsklerose, die Alzheimersche Erkrankung, Chorea Huntington, Epilepsie, Morbus Parkinson oder Synucleopathien, z.B. vom Typ des Parkinson-Plus-Syndroms.

Neben der Behandlung von Erkrankung, die eindeutig unter Beteiligung des ZNS entstehen und/oder verlaufen, können die erfindungsgemäßen Substanzen auch zur Behandlung weiterer Erkrankungen verwendet werden, die nicht, nicht eindeutig oder nicht ausschließlich ZNS-assoziiert sind. Solche Erkrankungen sind insbesondere Störungen des Urinaltrakts, wie z.B. sexuelle Dysfunktion, insbesondere männliche erektile Dysfunktion und urinale Inkontinenz. Zur Behandlung urinaler inkontinenz sind insbesondere Verbindungen mit ausgeprägter serotonerger Wirkkomponente geeignet.

Ein Gegenstand der Erfindung ist daher die Verwendung einer erfindungsgemäßen Verbindung zur Herstellung eines Arzneimittels zur Behandlung von Erkrankungen des Urinaltrakts, insbesondere von männlicher erektiler Dysfunktion und urinaler Inkontinenz.

Erkrankungen, für die die erfindungsgemäßen Verbindungen besonders geeignet sind, sind Schizophrenie, depressive Störungen, L-Dopa- oder Neuroleptika-induzierte Bewegungsstörungen, Morbus Parkinson, Segawa-Syndrom, Restless-Leg-Syndrom, Hyperprolaktinämie, Hyperprolaktinom, Hyperaktivitätssyndrom (ADHS) und urinale Inkontinenz.

Bewegungsstörungen, die der Therapie mit den erfindungsgemäßen Substanzen besonders zugänglich sind, sind insbesondere
- Morbus Parkinson-assoziierte Bewegungsstörungen, z.B. Rigor, Tremor, Dystonie und Dyskinesie,
- Segawa-Syndrom
- Neuroleptika-induzierte (tardive) extrapyramidalmotorische Bewegungsstörungen, insbesondere Dyskinesie, Dystonie und Akathisie,
- L-Dopa-induzierte extrapyramidalmotorische Bewegungsstörungen, insbesondere Dyskinesien und Dystonien
- Restless Leg Syndrom

Schließlich können die erfindungsgemäßen Arzneimittel in Abhängigkeit von der zu behandelnden Erkrankung auch als Kombinationspräparat zur gleichzeitigen oder sequentiellen Gabe ausgebildet sein.

Beispielsweise kann eine Verkaufseinheit, die eine zur Behandlung der Parkinson-Erkrankung enthaltende L-Dopa Medikation enthält, auch eine pharmazeutische Zusammensetzung umfassen, die eine oder mehrere der erfindungsgemäßen Verbindungen mit z.B. hochselektivern, partialagonistischem dopaminergem und/oder serotonergem Wirkprofil enthält. Dabei können L-Dopa und die erfindungsgemäße Verbindung in der gleichen pharmazeutischen Formulierung, z.B. einer Kombinationstablette, oder auch in unterschiedlichen Applikationseinheiten vorliegen, z.B. in Form zweier separater Tabletten. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Eine Ausführungsform der Erfindung betrifft daher ein Arzneimittel, das L-Dopa oder ein Neuroleptikum sowie eine erfindungsgemäße Verbindung zur gleichzeitigen oder zeitlich aufeinanderfolgenden Verabreichung an den Patienten enthält.

In einer anderen Ausführungsform der Erfindung kann die Verkaufseinheit ein Kombinationspräparat sein oder zwei Applikationseinheiten enthalten, die zwei der erfindungsgemäßen Verbindungen mit unterschiedlichem Rezeptorprofil, z.B. einen hochaffinen, hochselektiven D3-Modulator und einen hochaffinen 5-HT1a-Modulator enthalten.

Üblicherweise bestehen die erfindungsgemäßen Arzneimittel aus einer pharmazeutischen Zusammensetzung, die neben den erfindungsgemäßen Verbindungen, wie oben beschrieben, mindestens einen pharmazeutisch annehmbaren Träger oder Hilfsstoff enthält.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur intravenösen, intramuskulären, intrakutanen, subkutanen, oralen, bukkalen, sublingualen, nasalen, transdermalen, inhalativen, rektalen oder intraperitonealen Verabreichung angepasst sein.

Entsprechende Formulierungen und hierfür geeignete pharmazeutische Träger bzw. Hilfsstoffe, wie Füllstoffe, Sprengmittel, Bindemittel, Gleitmittel, Stabilisatoren, Aromastoffe, Antioxidantien, Konservierungsmittel, Dispersions- oder Lösungsmittel, Puffer oder Elektrolyte, sind dem Fachmann auf dem Gebiet der Pharmazeutik bekannt und sind beispielsweise in Standardwerken wie Sucker, Fuchs und Speiser ("Pharmazeutische Technologie", Deutscher Apotheker Verlag, 1991) und Remington ("The Science and Practice of Pharmacy", Lippincott, Williams & Wilkins, 2000) beschrieben.

In einer bevorzugten Ausführungsform der Erfindung werden die pharmazeutischen Zusammensetzungen, die die erfindungsgemäßen Verbindungen enthalten, oral verabreicht und können beispielsweise als Kapsel, Tablette, Pulver, Granulat, Dragee oder in flüssiger Form vorliegen.

Dabei kann die Formulierung als schnell freisetzende Darreichungsform ausgestaltet sein, wenn ein rascher Wirkeintritt gewünscht ist. Entsprechende orale Formulierungen sind beispielsweise beschrieben in EP 0 548 356 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt

Alternative pharmazeutische Zubereitungen können beispielsweise Infusions- oder Injektionslösungen, Öle, Suppositorien, Aerosole, Sprays, Pflaster, Mikrokapseln oder Mikropartikel sein.

Die Verbindungen der Formeln (I) bis (IX) wurden nach Methoden hergestellt, die teilweise bereits in der Literatur beschrieben sind (Bettinetti, L. et al. J. Med. Chem. 2002, 45, 4594-4597). Dazu wurden die Säurederivate vom Typ (A) entweder käuflich erworben, nach Literaturvorschrift synthetisiert oder deren Herstellungsmethoden in unseren Labors ausgearbeitet und dann in Form ihrer Carbonsäurechloride oder alternativ durch Verwendung spezieller Aktivierungsreagenzien wie zum Beispiel Hydroxybenzotriazol, Hydroxyazabenzotriazol, HATU (Kienhöfer, A. Synlett 2001, 1811-1812) oder TBTU (Knorr, R. Tetrahedron Lett. 1989, 30, 1927-1930) aktiviert und mit der freien Base vom Typ (C) zu den Derivaten der Formel (I) bis (IX) umgesetzt:

Eine erfindungsgemäße Verbindung nach den Formeln (I) bis (IX) kann hergestellt werden durch Umsetzung eines Säurederivats A 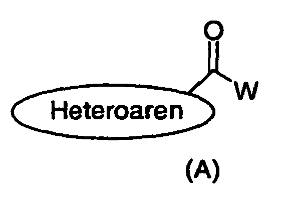 mit eines freien Base der allgemeinen Formel C 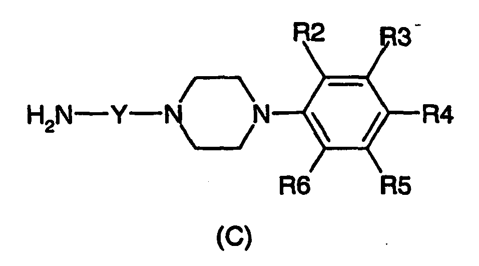 wobei gilt:
W ist ausgewählt aus OH, Cl, Br oder einer Gruppe 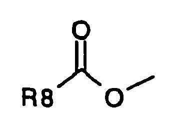 in der R8 für Alkyl steht;
Heteroaren steht jeweils für eine Gruppe, die ausgewählt ist aus 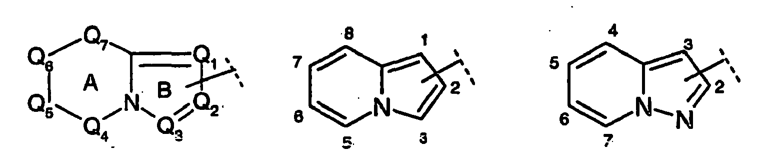 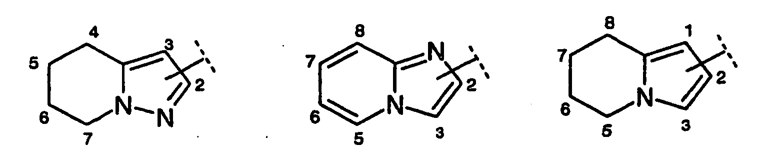 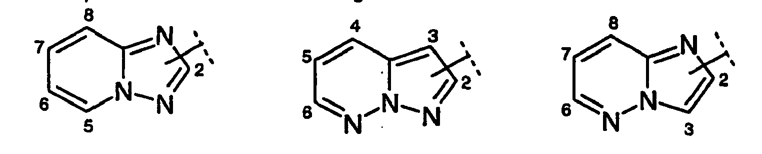 wobei
   A, B, Q1,Q2, Q3, Q4, Q5, Q6 und Q7 jeweils die Bedeutung haben, wie weiter vorstehend definiert und wobei die durchkreuzte Bindung bei den Heteroarenen für eine Bindung der Gruppe -C(O)-W an ein C-Atom eines aromatischen Rings des Heteroarens steht;
   die Heteroarene ein oder mehrfach substituiert sein können, wie vorstehend und in den Ansprüchen definiert;
   Y, R2, R3, R4, R5 und R6 jeweils die Bedeutung haben, wie vorstehend und in den Ansprüchen definiert,
   und wobei für den Fall, dass der Substituent W eine Hydroxygruppe ist, die entsprechende Säuregruppe vor der.Umsetzung mit der freien Base der allgemeinen Formel C durch Zugabe von Aktivierungsreagenzien, wie z.B. Hydroxybenzotriazol, Hydroxyazabenzotriazol, HATU oder TBTU aktiviert wird.

W ist bevorzugt Chlor, Brom oder OH und besonders bevorzugt Chlor oder OH.

Ein wichtiger Aspekt bei der Synthese dieser Zielverbindungen ist die effiziente und kostengünstige Gewinnung von Synthesevorstufen. Bei der Herstellung der Pyrazolo[1,5-*a*]pyridine, einer wichtigen Strukturklasse dieser Erfindung, erfolgt der Aufbau des heterozyklischen Grundkörpers über eine Cycloadditionsreaktion von N-Aminopyridin mit substituierten Propiolsäureestem (Bettinetti, L. et al. J. Med. Chem. 2002, 45, 4594-4597). Bislang gelingt die Herstellung der Pyridinvorstufen durch N-Aminierung mit Hydroxylamin-Derivaten wie nachfolgend formuliert:

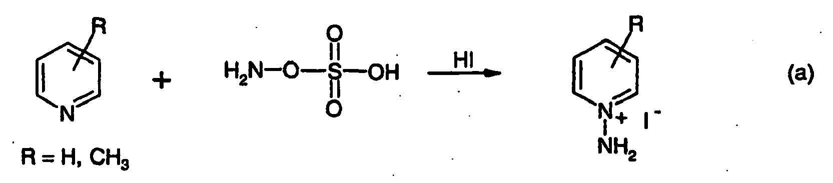

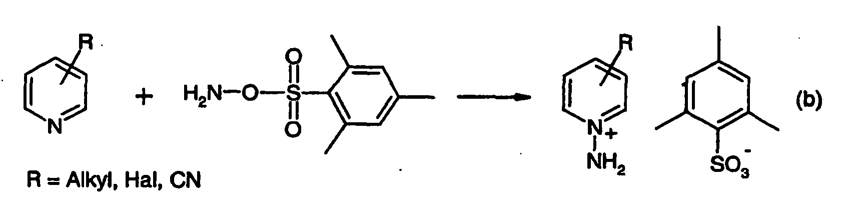

Dabei ist die Anwendung von Reaktion (a) stark eingeschränkt auf die Umsetzung von Pyridin oder Picolin, wogegen die Aminierung von substituierten Pyridinen nach Reaktion (b) aufgrund der hohen Kosten für den Einsatz des Aminierungsreagenzes Hydroxylamin-O-mesitylsulfonsäureester limitiert ist.

Wir beschreiben in dieser Erfindung einen effizienten und kostengünstigen Syntheseweg zur Herstellung unterschiedlich substituierter Pyrazolo[1,5-*a*]pyridine ausgehend von der Synthese der notwendigen N-Aminopyridine durch Umsetzung mit O-(2,4-Dinitrophenyl)hydroxylamin nach (c) (Legault, C. et al. J. Org. Chem. 2003, 68, 7119-7122) und anschließender Cycloadditionsreaktion mit Propiolsäureestern, wie es im nachfolgenden Formelschema exemplarisch formuliert ist (d): 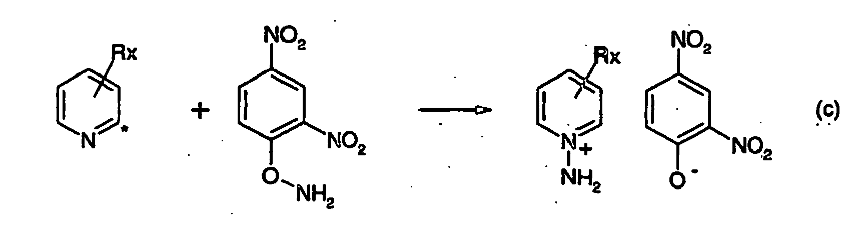 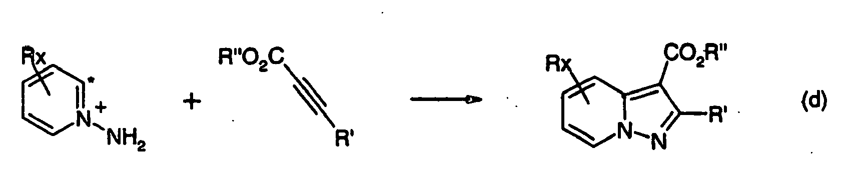 worin Rx für 0, 1, 2, 3 oder 4 gleiche öder verschiedene Substituenten ausgewählt aus Halogen, Alkyl, Alkylcarbonyl, Phenylcarbonyl, Hydroxyalkyl, Cyano, Trifluormethyl, und Alkyloxycarbonyl steht, * eine unsubstituierte CH-Gruppe kennzeichnet und worin R' ausgewählt ist aus Wasserstoff, Alkyl, Phenyl und Alkyloxycarbonyl und worin R" für Alkyl steht

Ein Gegenstand der Erfindung ist daher die Herstellung eines Carbonsäurederivats eines Pyrazolo[1,5-a]pyridins mit der allgemeinen Formel 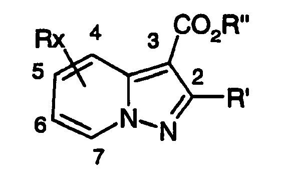 durch Umsetzung eines Pyridins der Formel 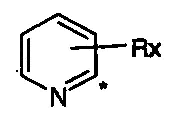 mit O-(2,4-Dinitrophenyl)hydroxylamin zu einem N-Aminopyridin der Formel 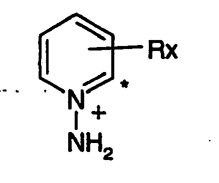 und anschließender Cycloadditionsreaktion mit einem Propiolsäureester der Formel 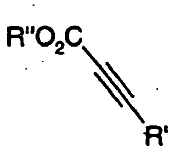 worin Rx für 0, 1, 2, 3 oder 4 gleiche oder verschiedene Substituenten ausgewählt aus Halogen, Alkyl, Alkylcarbonyl, Phenylcarbonyl, Hydroxyalkyl, Cyano, Trifluormethyl, und Alkyloxycarbonyl steht, *eine unsubstituierte CH-Gruppe kennzeichnet und worin R' und R" ausgewählt sind aus Wasserstoff, Alkyl, Phenyl und Alkyloxycarbonyl.

### SYNTHESE DER HETEROARENCARBONSÄURE-DERIVATE:

### Herstellung von Heteroarencarbonsäuren des Typs A1:

### Indolizin-2-carbonsäure

Die Herstellung der Indolizin-2-carbonsäure erfolgt durch Synthese des lndolifin-2-carboxymethylesters nach Literatur (Bode, M.L. Chem. Soc. Perkin. Trans. 1993, 1809-1813) und nachfolgender Hydrolyse.
Dazu werden 0,05 g (2,86 mmol) des 2-Indolizincarbonsäuremethylesters in 5 ml Methanol und 5 ml THF gelöst. Anschließend mit 2,5 ml 2n NaOH versetzt und 10 Stunden bei Raumtemperatur gerührt. Die Reaktionslösung wird am Rotationsverdampfer eingeengt und mit Wasser verdünnt, anschließend mit Hexan gewaschen, mit HCI auf pH 3-4 eingestellt und in Diethylether aufgenommen. Nach Trocknung mit MgSO₄ wird das Lösungsmittel abgedampft.
Ausbeute: 0,04 g (85%).
Smp.: 222°C. MS: m/z 161 (M⁺). IR (NaCl): 3429; 2924; 2852; 1741; 1664; 723. ¹H NMR (CDCl₃, 360 MHz) δ (ppm): 6.53-6.57 (m, 1 H, H-6); 7.67-6.72 (m, 1 H, H-7); 6.88 (s, 1H, H-1); 7.34 (d, J=9.0 Hz, 1 H, H-8); 7.87-7.88 (m, 2H,H-3, H-5).

### Herstellung von Heteroarencarbonsäuren des Typs A2:

### Pyrazolo[1,5-a]pyridin-2-carbonsäure, Pyrazolo[1,5-a]pyridin-3-carbonsäure, Pyrazolo[1,5-a]pyridin-5-cärbonsäüre, Pyrazolo[1,5-a]pyridin-6-carbonsäure, 5-Methoxypyrazolo[1,5-alpyridin-2-carbonsäure,5-Methylpyrazolo[1,5-a]pyridin-2-carbonsäure, 5-Trifluomethyl-pyrazolo[1,5-a]pyridin-2-carbonsäure,6-Brompyrazolo[1,5-a]pyridin-2-carbonsäure, 6-Chlorpyrazolo[1,5-a]pyridin-2-carbonsäure, 6-Fluorpyrazolo[1,5-a]pyridin-2-carbonsäure,

Die Synthese dieser Säurekomponenten erfolgt wie in der Literatur beschrieben (Bettinetti, L. et al. J. Med. Chem. 2002, 45, 4594-4597).

Die Synthese von 5-Methoxypyrazolo[1,5-*a*]pyridin-2-carbonsäure erfolgt analog der allgemeinen Bedingungen zur Synthese von Heteroarencarbonsäuren des Typs (A2). Ausbeute: 0,28 g (72%).
Smp.: 220°C. MS: m/z 192 (M⁺). IR (NaCl): 3050, 2939, 1704, 1652, 1411, 1230. ¹H NMR (DMSO, 360 MHz) δ (ppm): 3.84 (s, 3H, CH₃O); 6.72 (dd, J=7.5 Hz, 2.5 Hz, 1H, H-6); 6.82 (s, 1H, H-3); 7.09 (d, J=2.5 Hz, 1H, H-4), 8.58 (d, J=7.5 Hz, 1H, H-7), 12.96 (s, 1H, COOH).

Die Synthese von 5-Methylpyrazolo[1,5-*a*]pyridin-2-carbonsäure erfolgt analog der allgemeinen Bedingungen zur Synthese von Heteroarencarbonsäuren des Typs (A2). Ausbeute: 0,43 g (93%).
Smp.: 203°C. MS: m/z 176 (M⁺). IR (NaCl): 3133, 3050, 1697, 1405, 1270, 937. ¹H NMR (DMSO, 360 MHz) δ (ppm): 6.87-6.90 (m, 1 H, H-6); 6.91 (s, 1H, H-3); 7.52 (s, 1H, H-4); 8.62 (d, J=7.4 Hz, 1 H, H-7); 13.00 (br s, 1 H, COOH). ¹³C NMR (DMSO, 90 MHz) δ (ppm): 163.6, 145.3, 140.5, 134.6, 128.2, 117.1, 116.9, 98.5, 20.6.

Die Synthese von 5-Trifluormethylpyrazolo[1,5-*a*]pyridin-2-carbonsäure erfolgt analog der allgemeinen Bedingungen zur Synthese von Heteroarencarbonsäuren des Typs (A2). Ausbeute: 0,54 g (84%).
Smp.: 230°C. MS: m/z 230 (M⁺). IR (NaCl): 3445, 1698, 1495, 1460, 1331, 1241. ¹H NMR (DMSO, 360 MHz) δ (ppm): 7.30 (dd, J=7.4, 2.1 Hz, 1 H , H-6); 7.32 (s, 1 H, H-3); 8.36 (s, 1H, H-4); 8.97 (dd, J=7.4 Hz, 0.7 Hz, 1 H, H-7); 13.34 (br s, 1 H, COOH). ¹³C NMR (DMSO, 90 MHz) δ (ppm): 163.0 (CO₂H), 146.6 (C-7), 138.8 (C-2), 130.6 (C-3a), 124.4 (q, J= 34 Hz, C-5), 123.3 (q, J= 273 Hz, CF₃), 118.0 (q, J= 5 Hz, C-6), 109.6 (q, J= 3 Hz, C-4), 103.0 (C-3).

Die Synthese von 6-Brompyrazolo[1,5-*a*]pyridin-2-carbonsäure erfolgt analog der allgemeinen Bedingungen zur Synthese von Heteroarencarbonsäuren des Typs (A2).
Ausbeute: 0,27 g (70%).
Smp.: 226°C. MS: m/z 240 (M⁺), 242 ((M+2)⁺). IR (NaCl): 3135, 3070, 1701,1402, 1265, 920. ¹H NMR (DMSO, 360 MHz) δ (ppm): 7.12 (d, J=0.72 Hz, 1H, H-3); 7.44 (dd, J=9.6 Hz, J=1.8 Hz, 1H, H-5); 7.78 (dd, J=9.6 Hz, J=0.72 Hz, 1H, H-4); 9.16 (s, 1 H, H-7); 13.19 (br s, 1 H, COOH). ¹³C NMR (DMSO, 90 MHz) δ (ppm): 163.2, 145.5, 139.1, 129.2, 127.5,120.2, 108.4, 100.8.

Die Synthese von 6-Chlorpyrazolo[1,5-*a*]pyridin-2-carbonsäure erfolgt analog der allgemeinen Bedingungen zur Synthese von Heteroarencarbonsäuren des Typs (A2). Ausbeute: 0,35 g (80%).
Smp.: 233°C. MS: m/z 196 (M⁺), 198 ((M+2)⁺). IR (NaCl): 3444, 3080,1699, 1506, 1495, 1269, 1063. ¹H NMR (DMSO, 360 MHz) δ (ppm): 7.13 (d, J=0.9 Hz, 1H, H-3); 7.36 (dd, J=9.5 Hz, 1.8 Hz, 1 H, H-5); 7.83 (dd, J=9.5 Hz, 0.9 Hz,1 H, H-4); 8.97 (br s, 1H, H-7). ¹³C NMR (DMSO, 90 MHz) δ (ppm): 163.1, 145.7, 139.0, 127.1, 125.7, 121.3, 120.0, 100.7.

Die Synthese von 6-Fluorpyrazolo[1,5-*a*]pyridin-2-carbonsäura erfolgt analog der allgemeinen Bedingungen zur Synthese von Heteroarencarbonsäuren des Typs (A2).
Ausbeute: 0,17 g (71%).
Smp.: 245°C. MS: m/z 180 (M⁺). IR (NaCl): 3135, 3080,1698,1510, 1494.1269, 1064. ¹H NMR (DMSO, 360 MHz) δ (ppm): 7.14 (d, J=0.9 Hz, 1H, H-3); 7.43 (ddd, J=9.8 Hz, 8.4 Hz, 2.3 Hz, 1 H, H-6); 7.88 (ddd, J=9.8 Hz, 5.9 Hz, 0.7 Hz, 1 H, H-4); 9.05 (br d, J=4.8 Hz, 1H, H-7). ¹³C NMR (DMSO, 90 MHz) δ (ppm): 163.1 (CO₂H), 153.9 (d, J=236 Hz, C-6), 145.6 (d, J=3 Hz, C-3a), 138.3 (C-2), 120.0 (d, J=9 Hz, C-4), 116.8 (d, J=26 Hz, C-7), 116.3 (d, J=41 Hz, C-5), 100.7 (C-3).

### Herstellung von Heteroarencarbonsäuren des Typs A3:

### 3-Brompyrazolo[1,5-a]pyridin 2-carbonsäure, 3-Chlorpyrazolo[1,5-a]pyridin-2-carbonsäure, 3-Brompyrazolo[1,5-a]pyridin-5-carbonsäure, 3-Chlorpyrazolo[1,5-a]pyridin-5-carbonsäure

0,10 g (0,6 mmol) Pyrazolo[1,5-*a*]pyridin-2-carbonsäure (A2) und 0,13 g (0,75 mmol) N-Bromsuccinimid werden mit 7 ml Chloroform unter Schutzgasatmosphäre gemischt und 55 Stunden bei Raumtemperatur gerührt. Anschließend wird das Lösungsmittel im Vakuum abgedampft; Reinigung durch Flashchromatographie (CH₂Cl₂-MeOH: 90-10) ergibt 3-Brompyrazolo[1,5-*a*]pyridin-2-carbonsäure.
Ausbeute 0,11 g (73%).
Smp.: > 300°C dec. MS: m/z 240 (M⁺), 242 ((M+2)⁺). IR (NaCl): 3382, 1643, 1577, 1523, 1467, 1396. ¹H NMR (DMSO, 360 MHz) δ (ppm): 7.06-7.10 (m, 1H, H-6); 7.34-7.41 (m, 1H, H-5); 7.59 (d, J=8,9 Hz, 1H, H-4); 9.24 (d, J=6.7 Hz, 1 H, H-7).

Die Synthese von 3-Chlorpyrazolo[1,5-*a*]pyridin-2-carbonsäure erfolgt analog der allgemeinen Bedingungen zur Synthese von Heteroarencarbohsäuren des Typs (A3). Ausbeute: 60 mg (49%).
Smp.: > 300°C dec. MS: m/z 196 (M⁺), 198 ((M+2)⁺). IR (NaCl): 3396, 3099,1633,1604, 1504, 1403, 1348. ¹H NMR (DMSO, 360 MHz) δ (ppm): 7.04-7.08 (m, 1H, H-5); 7.34-7.38 (m, 1 H, H-6); 7.61 (d, J=9.2 Hz, 1 H, H-4); 9.08 (br d, J=5.7 Hz,1H, H-7).

Die Synthese von 3-Brompyrazolo[1,5-*a*]pyridin-5-carbonsäure erfolgt analog der allgemeinen Bedingungen zur Synthese von Heteroarencarbonsäuren des Typs (A3). Ausbeute: 0,13 g (87%).
Smp.: > 300°C dec. MS: m/z 240 (M⁺), 242 ((M+2)⁺). IR (NaCl): 3382, 1643, 1577, 1523, 1467, 1396. ¹H NMR (DMSO, 360 MHz) δ (ppm): 7.38 (d, J=6.7 Hz, 1 H, H-6); 8.07 (s, 1H, H-4); 8.25 (s, 1 H, H-2); 8.75 (d, J=7.1 Hz, 1H, H-7).

Die Synthese von 3-Chlorpyrazolo[1,5-*a*]pyridin-5-carbonsäure erfolgt analog der allgemeinen Bedingungen zur Synthese von Heteroarencarbonsäuren des Typs (A3). Ausbeute: 75 mg (99%).
Smp.:180°C. MS: m/z 196 (M⁺), 198 ((M+2)⁺). IR (NaCl): 3406, 3100, 1710,1576,1529, 1509, 1396. ¹H NMR (DMSO, 360 MHz) δ (ppm): 7.42-7.45 (m, 1 H, H-6); 8.10 (s, 1 H, H-4); 8.16 (s, 1 H Hz, H-2); 8.63 (br d, J=7.1 Hz, 1H, H-7).

### Herstellung von Heteroarencarbonsäuren des Typs A4:

### 4,5,6,7-Tetrahydropyrazolo[1,5-a]pyridin-2-carbonsäure, 4,5,6,7-Tetrahydropyrazolo[1,5-a]pyridin-3-carbonsäure, 4,5,6,7-Tetrahydro-5-methylpyrazolo[1,5-a]pyridin-2-carbonsäure

0,20 g (1,2 mmol) Pyrazolo[1,5-*a*]pyridin-2-carbonsäure (A2) werden in 10 ml Ethanol gelöst und mit 40 mg Pd/C 10% bei 16 bar H₂-Druck und 80°C in einem 100 ml Druckrohr für 4 Stunden hydriert. Abfiltrieren von Pd-Kohle und Einrotierten ergibt 4,5,6,7-Tetrahydropyrazolo[1,5-*a*]pyridin-2-carbonsäure.
Ausbeute: 0.20 g (98%).
Smp.: 118°C. MS: m/z 166 (M⁺), IR (NaCl): 3135, 2951, 2867, 1717, 1215, 771. ¹H NMR (DMSO, 360 MHz) ö (ppm): 1.74-1.81 (m, 2H, H-5); 1.94-2.00 (m, 2H, H-6); 2.75 (t, J=6.4 Hz, 2H, H-4); 4.08-4.11 (m, 2H, H-7), 6.41 (s, 1H, H-3). ¹³C NMR (DMSO, 90 MHz) ö (ppm): 163.4, 142.2, 140.2, 105.4, 48.0, 22.7, 22.0, 19.6.

Die Synthese von 4,5,6,7-Tetrahydropyrazolo[1,5-*a*]pyridin-3-carbonsäure erfolgt analog der allgemeinen Bedingungen zur Synthese von Heteroarencarbonsäuren des Typs (A4). Ausbeute: 0,30 g (37%).
Smp.: 210°C. MS: m/z 166 (M⁺), IR (NaCl): 3399, 2957, 2921, 1705, 1551, 1230. ¹H NMR (DMSO, 360 MHz) δ (ppm): 9.75-1.82 (m, 2H, H-5); 1.91 -1.98 (m, 2H, H-6); 2.93 (t, J=6.4 Hz, 2H, H-4); 4.17-4.20 (t, J=6.0 Hz, 2H, H-7), 7.72 (s, 1 H, H-2).

Die Synthese von racemischem 4,5,6,7-Tetrahydro-5-methylpyrazolo[1,5-*a*]pyridin-3-carbonsäure erfolgt analog der allgemeinen Bedingungen zur Synthese von Heteroarencarbonsäuren des Typs (A4).
Ausbeute: 0.197 g (96%).
Smp.: 163°C. MS: m/z 180 (M⁺), IR (NaCl): 3343, 2360, 2927, 2871, 1691, 1396, 1240, 780. ¹H NMR (DMSO, 360 MHz) δ (ppm): 1.14 (d, J=6.7 Hz, 3H, CH₃); 1.70-1.82 (m, 1H, H-5); 1.97-2.11 (m, 2H, H-6); 2.39 (dd, J=16.3 Hz, J=10.3 Hz, 1 H, H-4); 2.96 (dd, J=16.3 Hz, J=4.6 Hz, 1 H, H-4); 4.09-4.17 (m, 1 H, H-7); 4.37-4.43 (m, 1 H, H-7); 6.56 (d, 1 H, 0.72 Hz, H-3).

### Herstellung von Heteroarencarbonsäuren des Typs A5:

### Indolizin-1-carbonsäure

Die Herstellung der Indolizin-1-carbonsäure erfolgt durch Synthese des Indolizin-1-carbonsäuremethylesters nach Literatur (Zhang, L. Feng, L., Sun, L. Hu, Y., Hu, H., *Synthesis* **2000,**1733-1737) und nachfolgender Hydrolyse.
Dazu werden 0,2 g (1,14 mmol) des Indolizincarbonsäuremethylesters in 5 ml Methanol und 5 ml THF gelöst. Anschließend mit 2,5 ml 2n NaOH versetzt und 10 Stunden bei Raumtemperatur gerührt. Die Reaktionslösung wird am Rotationsverdampfer eingeengt und mit Wasser verdünnt, anschließend mit Hexan gewaschen, mit HCl auf pH 3-4 eingestellt und in Diethylether aufgenommen. Nach Trocknung mit MgSO₄ wird das Lösungsmittel abgedampft .
Ausbeute: 0,072 g (39%).
Smp.: 196-198 °C. MS: m/z 161 (M⁺). IR (NaCl): 3362; 2925; 2853; 1633; 720.

### Herstellung von Heteroarencarbonsäuren des Typs A6:

### Tetrahydroindolizin-2-carbonsäure

0,06 g (0,375 mmol) Indolizin-2-carbonsäure (A1) werden in 10 ml Ethanol gelöst und mit 13 mg Pd/C 10% bei 16 bar H₂-Druck und 80°C in einem 100 ml Druckrohr für 6 Stunden hydriert. Abfiltrieren von Pd-Kohle und Verdampfen des Lösungsmittels ergibt 5,6,7,8-Tetrahydroindolizin-2-carbonsäure.
Ausbeute: 0.20 g (98%).
Smp.: 131-134 °C. MS: m/z 166 ((M+H)⁺), IR (NaCl): 3135, 2951, 2867, 1717, 1215, 771. ¹H NMR (DMSO, 360 MHz) δ (ppm): 1.69-1.76 (m, 2H, H-6); 1.82-1.88 (m, 2H, H-7); 2.65-2.69 (m, 2H, H-5); 3.90-3.95 (m, 2H, H-8), 6.05 (s, 1 H, H-3); 7.17 (s, 1 H, H-1).

### Herstellung von Heteroarencarbonsäuren des Typs A7:

### 1-Cyano-2-methylindolizin-3-carbonsäure

Der bei *Ambinter, Paris (F)* gekaufte 1-Cyano-2-methylindolizin-3-carbonsäureethylester(0,05 g (0,21 mmol)) wird in 5 ml Methanol und 5 ml THF gelöst. Anschließend wird mit 2,5 ml 2n NaOH versetzt und 4 Stunden bei Raumtemperatur gerührt. Die Reaktionslösung wird am Rotationsverdampfer eingeengt und mit Wasser verdünnt, anschließend mit Hexan gewaschen, mit HCl auf pH 3-4 eingestellt und in Diethylether aufgenommen. Nach Trocknung mit MgSO₄ wird das Lösungsmittel abgedampft .
Ausbeute: 0,04 g (90%).
MS: m/z 201 ((M+H)⁺).

### Herstellung von Heteroarencarbonsäuren des Typs A8:

### Imidazo[1,2-a]pyridin-6-carbonsäure

Zur Synthese werden 0,1 g (0,57 mmol) Imidazo[1,2-*a*]pyridin-6-carbonsäuremethylester *(Bionet Research Ltd., Camelford (UK))* in 5 ml Methanol und 5 ml THF gelöst. Anschließend wird mit 5 ml 2n NaOH versetzt und 4 Stunden bei Raumtemperatur gerührt. Die Reaktionslösung wird am Rotationsverdampfer eingeengt und mit Wasser verdünnt, anschließend mit Hexan gewaschen, mit HCl auf pH 3-4 eingestellt und in Diethylether -aufgenommen.-Die wässrige Phase wird durch Gefriertrocknung lyophilisiert, dann der Rückstand mit Ethanol ausgewaschen und filtriert. Nach Trocknung mit MgSO₄ wird das Lösungsmittel abgedampft.
Ausbeute: 0,02 g (22%) weiße harzige Substanz.
MS: m/z 163 ((M+H)⁺). IR (NaCl): 3378; 1643.

### Herstellung von Heteroarencarbonsäuren des Typs A9:

### 1,2,4-Triazolo[1,5-a]pyridin-2-carbonsäure

Die Herstellung der 1,2,4-Triazolo[1,5-*a*]pyridin-2-carbonsäure erfolgt durch Synthese des 1,2,4-Triazolo[1,5-*a*]pyridin-2-carbonsäureethylesters nach Literatur (Gomez, E., Avedano, C., McKillop, A., Tetrahedron 1986, 2625-2634) und nachfolgender Hydrolyse.
Dazu werden 0,05 g (0,26 mmol) des 1,2,4-Triazolo[1,5-*a*]pyridin-2-carbonsäureethylesters in 5 ml Methanol gelöst. Anschließend wird mit 2,5 ml 5n NaOH versetzt und 4 Stunden bei Raumtemperatur gerührt. Die Reaktionslösung wird am Rotationsverdampfer eingeengt und mit Wasser verdünnt, anschließend mit Hexan gewaschen, mit HCl auf pH 3-4 eingestellt und in Diethylether und Ethylacetat aufgenommen. Nach Trocknung mit MgSO₄ wird das Lösungsmittel abgedampft.
Ausbeute: 0,010 g (23%) weißes Harz.
MS: m/z 164 ((M+H)⁺).

### Herstellung von Heteroarencarbonsäuren des Typs A10:

### Pyrazolo[1,5-b]pyridazin-2-carbonsäure

Die Herstellung der Pyrazolo[1,5-*b*]pyridazin-2-carbonsäure erfolgt durch Synthese des Dimethylpyrazolo[1,5-b]pyridazin-2,3-dicarboxylats nach Literatur (Kobayashi, Y. Kutsuma, T., Morinaga, K., Chem. Pharm. Bull. 1971, 2106-2115) und nachfolgender saurer Hydrolyse und Decarboxylierung.
Dazu werden 0,20 g (1,0 mmol) des Dimethylpyrazolo[1,5-*b*]pyridazin-2,3-dicarboxylats in 10 ml H₂SO₄ (40%) suspendiert und dann für 2,5 Stunden auf 110 °C erhitzt. Die Reaktionslösung wird auf RT und dann auf 0 °C abgekühlt. Die gekühlte Lösung wird mit NaOH (5N) neutralisiert und mit HCI auf pH 3 eingestellt. Dann wird in Diethylether aufgenommen. Nach Trocknung mit MgSO₄ wird das Lösungsmittel abgedampft. Ausbeute: 0,045 g (28%) weißer Feststoff.
Smp.: 263-265°C). MS: m/z 164 ((M+H)⁺).

### EFFIZIENTE SYNTHESE VON SUBSTITUIERTEN PYRAZOLO[1,5-a]PYRIDINEN:

Die von uns entwickelte, effiziente Synthese von substituierten Pyrazolo[1,5-*a*]pyridinen wird am Beispiel der Herstellung von Pyrazolo[1,5-*a*]pyridin-5-ylcarbonsäure beschrieben.

### Herstellung der N-Aminopyridine:

### N-Amino-4-hxdroxymethylpyridinium 2,4-dinitrophenolat

Zu einer Lösung von 1,50 g (7,54 mmol) *O*-(2,4-Dinitrophenyl)hydroxylamin in 10 ml Methylenchlorid werden unter Schutzgasatmosphäre 0,75 g (6,89 mmol) 4-Hydroxymethylpyridin zugetropft und 21 Stunden bei Raumtemperatur gerührt Nach Zugabe von Diethylether wird der ausgefallene Feststoff abfiltriert und mit Ether gewaschen. Das Produkt wird ohne Reinigung für die nächste Reaktion eingesetzt.
Ausbeute: 1,78 g (84%).
Smp.: 108°C.

### Herstellung der Pyrazolo[1,5-a]pyridincarbonsäuren:

### 5-Hydroxymethylpyrazolo[1,5-a]pyridin-3-ylcarbonsäuremethylester

Zu einer Lösung von 1,5 g (4,9 mmol) N-Amino-4-hxdroxymethylpyridinium 2,4-dinitrophenolat in 8 ml trockenem DMF wird 0,97 g (7 mmol) Kaliumcarbonat gegeben und 0,45 g (5,3 mmol) Propiolsäuremethyylester zugetropft. Nach 20 Stunden Rühren bei Raumtemperatur wird abfiltriert, das Lösungsmittel evapuriert, der Rückstand mit Wasser aufgenommen, mit Diethylether extrahiert und die organische Phase mit MgSO₄ getrocknet. Evapurieren des Lösungsmittels und Reinigung mit Flashchromatographie an Kieselgel (EtOAc-Benzin:3-7) ergibt 5-Hydroxymethylpyrazolo[1,5-a]pyridin-3-ylcarbonsäuremethylester.
Ausbeute: 0,46 g (46%).

Die analytischen Daten sowie die weiteren Syntheseschritte zur Gewinnung von Pyrazolo[1,5-a]pyridin-5-ylcarbonsäure sind in der Literatur beschrieben (Bettinetti, L. et al. J. Med. Chem. 2002, 45, 4594-4597).

### SYNTHESE DER AMINKOMPONENTEN:

### Herstellung der Amine vom Typ C1:

### 4-(4-(2,3-Dichlorphenyl)piperazin-1-yl)alkylamin, 4-(4-(2-Methoxyphenyl)piperazin-1-yl)alkylamin

Für die Herstellung der Arylpiperazinylamine vom Typ (C1) können z.B. käuflich zugängliche 2-Methoxy- bzw. 2,3-Dichlorphenylpiperazine mit Brombutylphthalimid in Xylol alkyliert werden. Anschließende Hydrazinolyse der phthalimidsubstituierten Strukturen liefert die primären Amine vom Typ (A1). Dies wird anhand des folgenden Reaktionsschemas exemplarisch verdeutlicht:

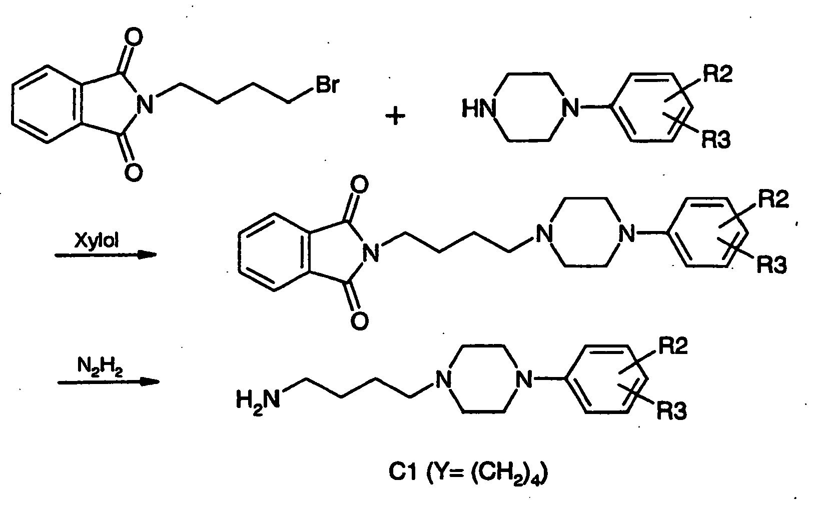

2,3 g (10 mmol) 2,3-Dichlorphenylpiperazin (Base) werden in 10 ml Xylol gelöst und auf 70°C erhitzt. Dann werden 1,4 g (5 mmol) 4-Brombutylphthalimid (gelöst in 20 ml Xylol) zugetropft und das Reaktionsgemisch für 24 Stunden bei 125°C erhitzt. Nach Abkühlen der Mischung auf 0°C wird abfiltriert und das Filtrat evapuriert. Das entstandene N-4-(4-(2,3-Dichiorphenyl)piperazin-1-yl)butylphthalimid wird durch Flashchromatographie an SiO₂ mit Ethylacetat gereinigt.
Ausbeute: 4,0 g (92%).
Zu einer Suspension von N-4-(4-(2,3-Dichlorphenyl)piperazin-1-yl)butylphthalimid in 40 ml Ethanol wird eine Lösung von 0,45 ml 80%igem Hydrazinhydrat (2,5 eq) in 5 ml Ethanol zugetropft. Die Mischung wird für 3 Stunden unter Rückfluss erhitzt, anschließend auf Raumtemperatur abgekühlt, der dabei ausfallende Feststoff abfiltriert, und die ethanolische Lösung im Vakuum abgedampft. Reinigung mit Flashchromatographie (CH₂Cl₂-MeOH-Me₂EtN:90-8-2) liefert die freie Base 4-(4-(2,3-Dichlorphenyl)piperazin-1-yl)butylamin.
Ausbeute: 0,900 g (60%).
MS: m/z 301 (M⁺), 303 ((M+4)⁺), 305 (M+4)⁺); IR: (NaCl): 3397, 2939, 2817, 1641, 1572, 1500, 1482, 1376, 1240, 1152, 1118, 1023, 917, 791, 749, 698, 661. ¹H NMR (CDCl₃, 360 MHz) δ (ppm): 1.48-1.64 (m, 4H,CH₂-CH₂); 2.44 (t, J=7.6 Hz, 2H, CH₂N); 2.64 (m, 4H, pip); 2.72-2.76 (m, 2H, H₂N-CH₂); 3.07 (m, 4H, pip); 6.93-6.99 (m, 1 H, Phenyl H-5); 7.11-7.17 (m, 2H, Phenyl H-4, Phenyl H-6).

### Herstellung der Amine vom Typ C2:

### 4-(4-(3-Chlor-2-methoxyphenyl)piperazin-1-yl)butylamin, 4-(4-(2,3-Difluorphenyl)piperazin-1-yl)butylamin

Ein alternativer Syntheseweg zur Gewinnung verschieden substituierter Phenylpiperazinylalirylamine vom Typ (C2) stellt die Reaktion des Piperazins mit einem Cyanoallrylhalogenid entsprechender Kettenlänge dar; wie es exemplarisch im folgenden Reaktionsschemas verdeutlicht wird:

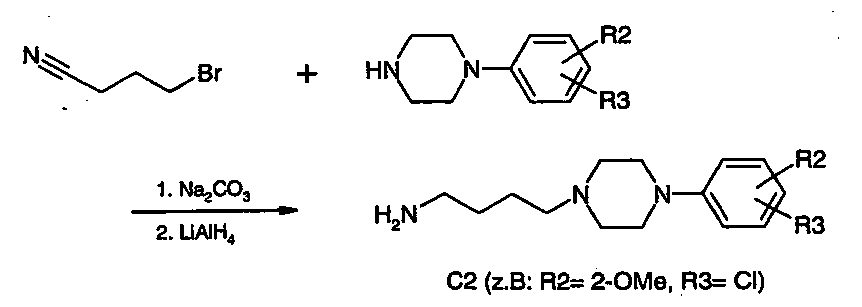

Die entsprechenden 2,3-disubstituierten Phenylpiperazine sind durch Paladium-katalysierte Aminierung von 2,3-substituierten Halogenaromaten mit Piperazin zugänglich:

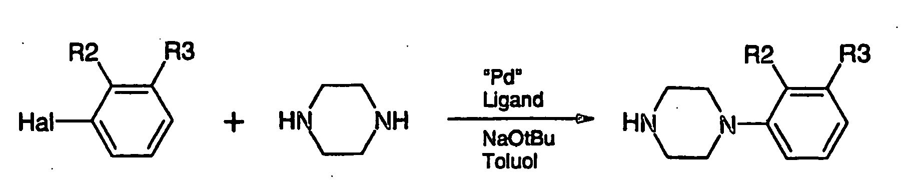

So werden für die Synthese von 4-(4-(3-Chlor-2-methoxyphenyl)piperazin-1-yl)butylamin 1,7 g (10 mmol) Piperazin (Base) mit 1,35 g NaOtBu (14 mmol), 0,024 g Pd(II)Acetat (0,5 mol%), 0,12 g P(OtBu)₃ (2 mol%l versetzt und mit 1,3 ml Dichloranisol (10 mmol) in 20 ml Toluol gelöst. Nach 21 Stunden Erhitzen auf 70°C wird die Mischung auf Raumtemperatur abgekühlt, filtriert und das Filtrat anschließend evapuriert um 4-(3-Chlor-2-methoxyphenyl)piperazin zu erhalten.
Ausbeute: 0,8 g (37%).
0,8 g (3,7 mmol) 4-(3-Chlor-2-methoxyphenyl)piperazin und 0.8 g (7,5 mmol) Na₂CO₃ werden in 20 ml Acetonitril gelöst, für 15 Stunden unter Rückfluss erhitzt, anschließend auf Raumtemperatur abgekühlt und die Lösung im Vakuum abgedampft. Der Rückstand wird mit Wasser aufgenommen und die wässrige Phase mit Methylenchlorid extrahiert, dieses getrocknet (mit MgSO₄) und das Lösungsmittel abgedampft. Reinigung durch Flashchromatographie (CHCl₃-EtOAc:1-1) liefert 4-(4-(3-Chlor-2-methoxyphenyl)piperazin-1yl)butyronitril.
Ausbeute: 0,4 g (35%).
Anschließend werden 0,15 g 4-(4-(3-Chlor-2-methoxyphenyl)piperazin-1yl)butyronitril (0,5 mmol) in 5 ml trockenem Diethylether gelöst und auf 0°C gekühlt. Dann werden langsam 1,0 ml LiAlH₄-Lösung (1 M In Diethylether) zugetropft und 1 Stunde bei Raumtemperatur gerührt. Nach erneutem Abkühlen auf 0°C wird mit gesättigter NaHCO₃-Lösung versetzt, durch eine Glasfritte mit Celite/MgSO₄/Celite filtriert und mit Methylenchlorid gewaschen. Evapurieren des Filtrats ergibt 4-(4-(3-Chlor-2-methoxyphenyl)piperazin-1-yl)butylamin. Ausbeute: 0,143g (96).
MS: m/z 297 (M⁺), 299 ((M+2)⁺), 301 ((M+4)⁺). IR: (NaCl): 3386, 2937, 2821,1635, 1584, 1540, 1474, 1450, 1251, 1132, 1001, 964, 782, 744, 680, 668. ¹H NMR (CDCl₃, 360 MHz) δ (ppm): 1.60-1.67 (m, 4H, CH₂-CH₂); 2.41-2.45 (m, 2H, H₂N-CH₂); 2.61 (m, 4H, pip); 3.14 (m, 4H, pip); 3.22-3.26 (m, 2H, CH₂N); 3.86 (s, 1H, OCH₃); 6.79-6.82 (m, 1H, Phenyl); 6.95 (dd, J=8.0 Hz, J=8.0 Hz, 1 H, Phenyl H-5); 7.00 (dd, J=1.8 Hz, J=8.0 Hz, 1H, Phenyl).

Für die Herstellung von 4-(4-(2,3-Difluorphenyl)piperazin-1-yl)butylamin werden 0,56 g (5 mmol) Piperazin (Base) mit 0,675 g NaOtBu (7 mmol), 0,046 g Pd₂(dba)₃ (0,5 mol%), 0,093 g BINAP (2 mol%), 0,56 ml (5 mmol) 1-Brom-2,3-difluorbenzol in 20 ml Toluol gelöst und für 18 Stunden auf 115°C erhitzt. Nach Abkühlen der Reaktionslösung auf Raumtemperatur wird abfiltriert und das Filtrat evapuriert um 2,3-Difluorphenylpiperazin zu erhalten.
Ausbeute: 0,55 g (55%).
Die nachfolgende Umsetzung zum 4-(4-(2,3-Difluorphenyl)piperazin-1-yl)butylamin erfolgt analog der oben beschriebenen Synthese von Aminen des Typs (B2).
Ausbeute: 0,173 g (78% über 2 Reaktionsschritte).
MS: m/z 269 (M⁺). IR: (NaCl): 3355, 2939, 2823, 1621, 1595, 1504, 1478, 1259, 1247, 1143, 1007, 774, 714. ¹H NMR (CDCl₃, 360 MHz) δ (ppm): 1.47-1.60 (m, 4H,CH₂-CH₂); 2.39-2.44 (m, 2H, H₂N-CH₂); 2.61-2.65 (m, 4H, pip); 2.71-2.75 (m, 2H, CH₂N); 3.12-3.15 (m, 4H, pip); 6.67-6.71 (m, 1 H, Phenyl); 6.73-6.80 (m, 1 H, Phenyl); 6.92-6.99 (m, 1 H, Phenyl).

### Herstellung der Amine vom Typ C3:

### 4-(4-(2,3-Dihydrobenzofuran-7 yl)piperazin-1 yl)butylamin, 4-(4-(Chroman-8-yl)piperazin-1-yl)butylamin

Die Synthese erfolgt zunächst analog der Literatur (Kerrigan, F. Tetrahedron Lett. 1998, 2219-2222) bis zur Gewinnung von 2,3-Dihydrobenzofuran-7-ylpiperazin mit einer Ausbeute von 54% über 4 Reaktionsschritte. Anschließend wird die freie Base analog der allgemeinen Bedingungen zur Synthese von Aminen des Typs (C2) alkyliert und das entstandene Nitril zu 4-(4-(2,3-Dihydrobenzofuran-7-yl)piperazin-1yl)butylamin reduziert. Ausbeute: 0,27 g (86% über 2 Reaktionsschritte).
MS: m/z 275 (M⁺). IR: (NaCl): 3359, 2939, 2820, 1609, 1487, 1456, 1254, 1190, 1132, 1012, 942, 870, 755. 661. ¹H NMR (CDCl₃, 360 MHz) δ (ppm): 1.43-1.63 (m, 4H,CH₂-CH₂); 2.34-2.40 (m, 2H, H₂N-CH₂); 2.62 (m, 4H, pip); 2.72-2.74 (m, 2H, O-CH₂-CH₂); 3.15-3.21 (m, 6H, pip, CH₂N); 4.56-4.61 (m, 2H, O-CH₂-CH₂); 6.69-6.71 (m, 1 H, Phenyl); 6.77-6.86 (m, 2H, Phenyl).

Die Herstellung von 4-(4-(Chroman-8-yl)piperazin-1-yl)butylamin erfolgt analog der allgemeinen Bedingungen zur Synthese von Aminen des Typs (C3).
Ausbeute: 0,058 g (57% über 2 Reaktionsschritte).

MS: m/z 289 (M⁺). IR: (NaCl): 3354, 2933, 2870, 2814, 1664, 1479, 1461, 1247, 1196, 1024, 870, 737, ¹H NMR (CDCl₃, 360 MHz) ö (ppm): 1.46-1.59 (m,4H,CH₂-CH₂); 1.96-2.03 (m, 2H, O-CH₂-CH₂-CH₂); 2.39-2.44 (m, 2H, CH₂-N); 2.65 (m, 4H, pip); 2.70-2.74 (m, 2H, O-CH₂-CH₂-CH₂; 2.77-2.80 (m, 2H, CH₂-NH₂); 3.08 (m, 4H, pip); 4.24-4.27 (m, 2H, O-CH₂-CH₂-CH₂); 6.71-6.79 (m, 3H, Phenyl).

### Herstellung der Amine vom Typ C4:

### trans-4-(4-Aminomethylcyclohex-1-ylmethyl)-1-(2-methoxyphenyl)piperazin, trans-4-(4-Aminomethylcyclohex-1-ylmethyl)-1-(2,3-dichlorphenyl)piperazin

Die Synthese der Aminkomponenten mit Methylcyclohexylmethyl-Spacer zwischen Aminstickstoff und Piperazin wird wie folgt durchgeführt:

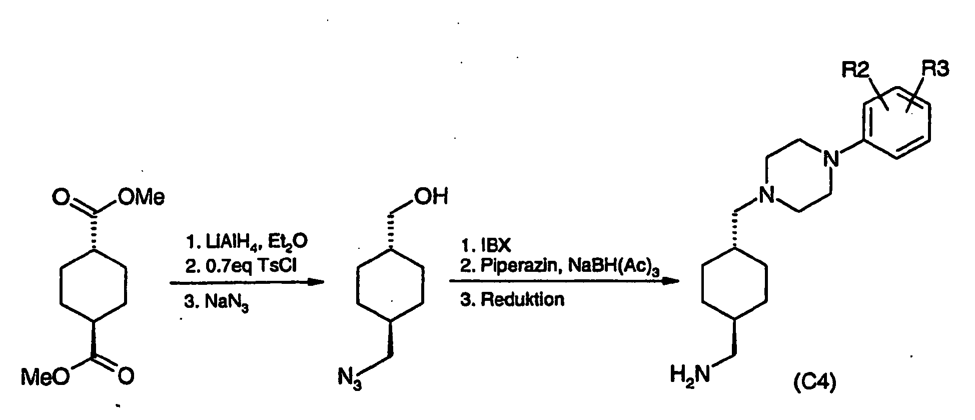

Ausgehend von 1,4-Cyclohexylidendicarbonsäuredimethylester erfolgt die Umsetzung zu 4-Azidomethylcyclohex-1-ylmethanol nach Literatur (Watanabe, T. Chem. Pharm. Bull.. 1995, 43, 529-531). Anschließende Oxidation zum Aldehyd, reduktive Aminierung mit den entsprechenden Phenylpiperazinen und Reduktion der Azidogruppe zum primären Amin liefert die Amine des Typs (C4).

Zur Synthese von trans-4-Azidomethylcyclohex-1-ylcarbaldehyd werden 0,10 g (0,6 mmol) *trans*-4-Azidomethylcyclohex-1-ylmethanol in 4 ml trockenem DMSO gelöst und nach Zugabe von 0,21 g (0,77 mmol) IBX (1-Hydroxy-1,2-benziodoxol-3(1H)-on-1-oxid) 5 Stunden bei Raumtemperatur gerührt. Nun gibt man Diethylether und NaHCO₃-Lösung zu und trennt die organische Phase ab. Diese wird erneut mit NaHCO₃-Lösung und Wasser gewaschen und über MgSO₄ getrocknet. Das Lösungmittel wird im Vakuum abgedampft. Ausbeute: 75 mg (76%).
MS: m/z 167 (M⁺); IR: (NaCl): 2927, 2856, 2097, 1723, 1452. ¹H NMR (CDCl₃, 360 MHz) δ (ppm): 1.01-1.12 (m, 2H, CH₂-CH₂-CH-CHO); 1.24-1.35 (m, 2H, CH₂-CH₂-CH-CHO); 1.49-1.60 (m, 1H, CH); 1.90-1.95 (m, 2H, CH₂-CH₂-CH-CHO); 2.03-2.07 (m, 2H, CH₂-CH₂-CHCHO); 2.15-2.24 (m, 1 H, CHCHO); 3.18 (d, J=6.8 Hz, 2H, CH₂N₃); 9.63 (d, J=1.4 Hz, 1 H, CHO). ¹³C NMR (CDCl₃, 90 MHz) δ (ppm): 204.0, 57.5, 50.0, 41.0, 37.3, 29.8, 29.2, 25.3.

Die Synthese von trans-4-(4-Azidomethylcyclohexylmethyl)-1-(2-methoxyphenyl)piperazin beginnt mit dem Lösen von 0,39 g (2,3 mmol) trans-4-Azidomethylcyclohex-1-ylcarbaldehyd und 0,56 g (2,9 mmol) 2-Methoxyphenylpiperazine in 15 ml Dichlomethan und der Zugabe von 0,74 g (3,5 mmol) Natriumtriacetoxyborhydrid. Nach 23 Stunden Reaktion bei Raumtemperatur wird das Gemisch mit NaHCO₃-Lösung gewaschen, die organische Phase eingeengt und mit Flashchromatographisch (EtOAc-Benzin : 1-1) gereinigt.
Ausbeute: 0,78 g (97%).
IR: (NaCl): 2919, 2851, 2812, 2095, 1500, 1450, 1240. ¹H NMR (CDCl₃, 360 MHz) δ (ppm): 0.87-1.05 (m, 4H, CH₂-CH₂); 1.47-1.50 (m, 2H, CH); 1.80-1.91 (m, 4H, CH₂-CH₂); 2.21 (d, J=7.1 Hz, 2H, CH₂Npip); 2.59 (m, 4H, pip); 3.08 (m, 4H, pip); 3.14 (d, J=6.4 Hz, 2H, CH₂N₃); 3.86 (s, 3H, CH₃O); 6.84-7.01 (m, 4H, Phenyl).

Die Synthese von trans-4-(4-Azidomethylcyciohexylmethyl)-1-(2,3-dichlorphenyl)piperazin gelingt unter gleichen Bedingungen.
Ausbeute: 0,80 g (85%).
IR: (NaCl): 2930, 2818, 2801, 2096, 1577, 1448. ¹H NMR (CDCl₃, 360 MHz) δ (ppm): 0.87-1.06 (m, 4H, CH₂-CH₂); 1.44-1.59 (m, 2H, CH); 1.81-1.90 (m, 4H, CH₂-CH₂); 2.21 (d, J=7.1 Hz, 2H, CH₂Npip); 2.57 (m, 4H, pip); 3.05 (m, 4H, pip); 3.14 (d, J=6.4 Hz, 2H, CH₂N₃); 6.92-6.97 (m, 1H, Phenyl); 7.10-7.16 (m, 4H, Phenyl). ¹³C NMR (CDCl₃, 90 MHz) δ (ppm): 151.4, 134.0, 127.5, 127.4, 124.4, 117.5, 65.4, 58.0, 53.8, 51.4, 38.4, 35.0, 31.1, 30.3.

Die Aminkomponente trans-4-(4-Aminomethylcyclohex-1-ylmethyl)-1-(2-methoxyphenyl)piperazin wird hergestellt durch Vorlage einer Lösung von 0,40 g (1,2 mmol) trans-4-(4-Azidomethylcyclohexylmethyl)-1-(2-methoxyphenyl)piperazin in 10 ml Methanol und Zugabe von 0,10 g Pd/C 10%. Die Suspension wird unter H₂-Atmosphäre 23 Stunden bei Raumtemperatur gerührt. Dann wird das Lösungsmittel im Vakuum abgedampft und mit Flashchromatographie (CH₂Ch₂-CH₃OH-NEtMe₂: 90-8-2) gereinigt.
Ausbeute: 0,14 g (39%) (leicht gelbliches ÖI).
MS: 317 m/z (M⁺); IR: (NaCl): 3382, 2912, 2842, 2811, 1500, 1240, 747. ¹H NMR (CDCl₃, 360 MHz) δ (ppm): 0.87-1.05 (m, 4H, CH₂-CH₂); 1.25-1.30 (m, 1 H, CH); 1.45-1.56 (m, 1H, CH); 1.81-1.91 (m, 4H, CH₂-CH₂);2.21 (d, J=7.1 Hz, 2H, H₂N-CH₂); 2.55 (d, J=6.4 Hz, 2H, CH₂Npip); 2.59 (m, 4H, pip); 3.08 (m, 4H, pip); 3.86 (s, 3H, CH₃O); 6.84-7.01 (m, 4H, Phenyl). ¹³C NMR (CDCl₃, 90 MHz) δ (ppm): 152.3, 141.5, 122.7, 120.9, 118.1, 111.1, 65.7, 55.3, 53.9, 50.7, 48.7, 35.3, 31.4, 30.9, 30.4.

Zur Herstellung von trans-4-(4-Aminomethylcyclohex-1-ylmethyl)-1-(2,3-dichlorphenyl)piperazn wird zu einer Lösung von 0.20 g (0,52 mmol) trans-4-(4-Azidomethylcyclohexylmethyl)-1-(2,3-dichlorphenyl)piperazin in 25 ml trockenem THF 1,05 ml LiAlH₄-Lösung (1 M in THF) gegeben und 8 Stunden unter Rückfluss erhitzt. Die Lösung wird im Vakuum abgedampft und durch Flashchromatographie (CH₂Cl₂-CH₃OH-NEtMe₂: 90-8-2) gereinigt.
Ausbeute: 0,13 g (36%) (leicht gelbliches Öl).
MS: 355 m/z (M⁺), 357 ((M+2)⁺), 359 ((M+4)⁺); IR: (NaCl): 3375, 2913, 2843, 2817, 1577, 1448, 778. ¹H NMR (CDCl₃, 360 MHz) δ (ppm): 0.85-0.98 (m, 4H, CH₂-CH₂); 1.19-1.31 (m, 1 H, CH); 1.43-1.52 (m, 1 H, CH); 1.80-1.88 (m, 4H, CH₂-CH₂); 2.19 (d, J=7.1 Hz, 2H, H₂N-CH₂); 2.53-2.56 (m, 6H, pip, CH₂Npip); 3.06-3.08 (m, 3H, pip); 3.17-3.20 (m, 1H, pip); 6.94-6.96 (m, 1 H, Phenyl), 7.10-7.15 (m, 2H, Phenyl).

### SYNTHESE DER BEISPIELVERBINDUNGEN

### Beispiel 1:

### N-4-(4-(2-Methoxyphenyl)piperazin-1-yl)butylindolizin-2-ylcarbamid

0,019 g Indolizin-2-carbonsäure (0,12 mmol) werden in 4 ml trockenem Methylenchlorid gelöst. Es werden 0,07 ml (0,42 mmol) trockenes DIPEA zugegeben. Anschließend wird 0,042 g (0,13 mmol) des in 0.5 ml trockenem DMF gelösten TBTU bei 0° C langsam zugetropft und 15 Minuten bei Raumtemperatur gerührt. Die Reaktionslösung wird erneut auf 0°C gekühlt und eine Lösung von 0.034 g (0,13 mmol) 4-(4-(2-Methoxyphenyl)-piperazin-1-yl)butylamin in 4 ml trockenem Methylenchlorid bei 0°C zugetropft. Nach 1 Stunde wird der Reaktionsansatz in CH₂Cl₂ aufgenommen und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt.
Ausbeute: 39 mg (81%).
Smp.: 143°C; MS: m/z 406 (M⁺); IR (NaCl): 2933; 2819; 1631; 1558; 1500; 1242; 1029; 750. ¹H NMR (CDCl₃, 360 MHz) δ (ppm): 1.67-1.69 (m, 4H, CH₂-CH₂); 2.50 (t, J=6.9 Hz, 2H, CH₂N); 2.65-2.70 (m, 4H, pip); 3.08-3.11 (m, 4H, pip); 3.47-3.52 (m, 2H, CH₂NHCO); 3.86 (s, 3H, OCH₃); 6.39 (br t, J=5.1 Hz, 1H, NHCO); 6.49-6.53 (m, 1 H, H-6); 6.59 (s, 1 H, H-1); 6.68 (ddd, J=1.1 Hz, J=6.6 Hz, J=9.1 Hz, 1 H, H-7); 6.84-6.87 (m, 1 H, H-arom); 7.91-7.02 (m, 3H, H-arom); 7.33 (d, J=9.1 Hz, 1H, H-8); 7.74-7.75 (m, 1H, H-3); 7.87 (dd, J=7.1 Hz, J=0.9 Hz, 1H, H-5). ¹³C NMR (CDCl₃, 90 MHz) δ (ppm): 164.9; 152.2; 141.1; 132.8; 125.4; 123.9; 122.9; 120.9; 119.8; 118.2; 113.9; 111.8; 111.2; 97.1; 89.3; 58.1; 55.3; 53.4; 50.4; 39.3; 27.6; 24.2.
C H N (%): C₂₄H₃₀N₄O₂ x 0.5 H₂O
Ber.: C 69,37; H 7,52; N 13,48 Gef.: C 69,07; H 7,30; N 13,46.

### Beispiel 2:

### N-4-(4-(3-Chlor-2-methoxyphenyl)piperazin-1-yl)butylindolizin-2-ylcarbamid

### Synthese analog zu Beispiel 1.

### Ausbeute: 41 mg (75%).

Smp.: 157°C. MS: m/z 440 (M⁺), 442 ((M+2)⁺), 444 ((M+4)⁺). IR (NaCl): 3321; 2936; 2811; 1626; 1554; 1525; 1250; 1142; 739. ¹H NMR (CDCl₃, 360 MHz) δ (ppm): 1.66-1.68 (m, 4H, CH₂-CH₂); 2.48 (t, J=6.9 Hz, 2H, CH₂Npip); 2.62-2.66 (m, 4H, pip); 3.11-3.18 (m, 4H, pip); 3.47-3.52 (m, 2H, CH₂NHCO); 3.86 (s, 3H, OCH₃); 6.35 (br t, J=5.0 Hz, 1H, NHCO); 6.49-6.53 (m, 1 H, H-6); 6.58 (br s, 1 H, H-1); 6.69 (ddd, J=9.1 Hz, J=6.6 Hz, J=1.1 Hz, 1 H, H-7); 6.77 (dd, J=8.0 Hz, J=1.8 Hz, 1 H, H-arom); 6.94 (dd, J=8.0 Hz, 1 H, H-arom); 7.00 (dd, J=8.0 Hz, J=1.8 Hz, 1 H, H-arom); 7.33 (d, J=9.1 Hz, 1 H, H-8); 7.74-7.75 (m,1H, H-3); 7.85 (dd, J=7.1 Hz, J =0.9 Hz, 1H, H-5). ¹³C NMR (CDCl₃, 90 Mhz) δ (ppm): 164.9; 148.6; 146.5; 132.8; 128.7; 125.4; 124.6; 123.9; 123.3; 119.8; 118.2; 117.0; 113.9; 111.8; 97.0; 58.9; 58.1; 53.7; 50.0; 39.4; 27.6; 24.2.
C H N (%): C₂₄H₂₉ClN₄O₂. 0.3 H₂O
Ber.: C 64,49; H 6,69; N 12,53; Gef.: C 64,57; H 6,72; N 12,46.

### Beispiel 3:

### N-4-(4-(2,3-Dichlorphenyl)piperazin-1-yl)butylindolizin-2-ylcarbamid

### Synthese analog zu Beispiel 1.

### Ausbeute: 30 mg (57%).

Smp.: 179°C. MS: m/z 444 (M⁺), 446 ((M+2)⁺), 448 ((M+4)⁺). IR (NaCl): 3427; 2925; 2852; 1631; 1529; 1244; 1043; 731. ¹H NMR: (CDCl₃, 360 MHz) δ (ppm): 1.64-1.70 (m, 4H,CH₂-CH₂); 2.50 (t, J=6.9 Hz, 2H, CH₂Npip); 2.63-2.69 (m, 4H, pip); 3.04-3.08 (m, 4H, pip); 3.47-3.52 (m, 2H, CH₂NHO); 6.33 (br t, J= 5.1 Hz, 1 H, NHCO); 6.49-6.53 (m, 1 H, H-6); 6.58 (s, 1H, H-1); 6.69 (ddd, J=9.1 Hz, J=6.6 Hz, J=1.1 Hz, 1H, H-7); 6.92 (dd, J=7.3 Hz, J=2.3 Hz, 1H, H-arom); 7.10-7.17 (m, *2*H, H-arom); 7.33 (d, J=9.1 Hz, 1H, H-8); 7.75-7.76 (m, 1 H, H-3); 7.87 (dd, J=7.1 Hz, J=1.0 Hz, 1H, H-5). ¹³C NMR (CDCl₃, 90 MHz) δ (ppm): 164.9; 151.2; 134.0; 132.9; 127.5; 127.4; 125.4; 124.6; 123.9; 119.8; 118.6; 118.3; 113.9; 111.8; 96.9; 58.0; 53.3; 51.1; 39.4; 27.7; 24.3.

### Beispiel 4:

### N-4-(4-(2,3-Difluorphenyl)piperazin-1-yl)butylindolizin-2-ylcarbamid

### Synthese analog zu Beispiel 1.

### Ausbeute: 46 mg (93%).

Smp.: 170°C. MS: m/z 412 (M⁺). IR (NaCl): 3316; 2946; 2812; 1626; 1556; 1502; 1266; 1142; 767. ¹H NMR (CDCl₃, 360 MHz) δ (ppm): 1.62-1.70 (m, 4H, CH₂-CH₂); 2.46 (t, J= 6.9 Hz, 2H, CH₂Npip); 2.61-2.64 (m, 4H, pip); 3.11-3.13 (m, 4H, pip); 3.46-3.52 (m, 2H, CH₂NHCO); 6.29 (br t, J= 5.0 Hz, 1 H, NHCO); 6.49-6.54 (m, 1H, H-6); 6.57 (s, 1 H, H-1); 6.63-6.71 (m, 2H, H-arom, H-7); 6.74-6.80 (m, 1H, H-arom); 6.91-6.98 (m, 1 H, H-arom); 7.33 (d, J=9.0 Hz, 1 H, H-8); 7.75 (d, J=1.2 Hz, 1 H, H-3); 7.85 (dd, J=6.9 Hz, J=1.1 Hz, 1H, H-5). ¹³C NMR (CDCl₃, 90 MHz) δ (ppm): 164.9:151.5 (dd, ¹J_{C-C}=10.4 Hz; ¹J_{C-F}=244.1 Hz, 1C, PhenylC-2); 143.9 (dd, ¹J_{C-C}=13.9 Hz, ¹J_{C-F}=246.9 Hz, 1C, Phenyl C-3); 141.9 (dd; ³J=5.5 Hz; ⁴J=2.1 Hz, 1C, PhenylC1); 132.9; 125.4; 123.5 (dd, ³J=8.3 Hz, ⁴J=4.9 Hz, 1C. PhenylC-4); 123.9; 119.8; 118.2; 117.0; 113.9; 111.8; 109.9; 96.9; 58.0; 53.2; 50.4; 39.4; 27.7; 24.3.

### Beispiel 5:

### N-4-(4-(2,3-Dihydrobenzofuran-7-yl)piperazin-yl)butylindolizin-2-ylcarbamid

### Synthese analog zu Beispiel 1.

### Ausbeute: 47 mg (94%).

Smp.: 159 °C. MS: m/z 418 (M⁺). IR (NaCl): 3323; 2941; 2817; 1634; 1557; 1267; 1146; 753. ¹H NMR (CDCl₃, 360 MHz) δ (ppm): 1.66-1.68 (m, 4H, CH₂-CH₂); 2.48 (t, J= 6.9 Hz, 2H, CH₂Npip); 2.67 (m, 4H, pip); 3.17-3.22 (m, 6H, O-CH₂-CH₂,pip); 3.46-3.51 (m, 2H, CH₂NHCO); 4.59 (t, J=8.8 Hz, 2H, O-CH₂-CH₂); 6.38 (br t, J= 4.8 Hz, 1 H, NHCO); 6.50 (ddd, J=6.9 Hz, J=6.6 Hz, J=1.2 Hz, 1H, H-6); 6.59 (s, 1 H, H-1); 6.66-6.70 (m, 2H, H-arom, H-7); 6.77-6.81 (m, 1 H, H-arom); 6.86 (dd, J=7,3 Hz, J=1.1 Hz, 1H, H-arom); 7.33 (d, J=9.0 Hz, 1 H, H-8); 7.75 (dd, J=2.1 Hz, J=1.1 Hz, 1 H, H-3); 7.86 (dd, J=7.1 Hz, J=1.1 Hz, 1H, H-5). ¹³C NMR (CDCl₃, 90 MHz) δ (ppm): 164.9; 151.1; 136.2; 132.8; 127.5; 125.4; 123.9; 121.0; 119.8; 118.2; 118.1; 115.6; 113.9; 111.8; 97.1; 71.0; 58.1; 53.2; 49.3; 39.4; 30.1; 27.6; 24.2.

### Beispiel 10:

### N-4-(4-(2-Methoxyphenyl)piperazin-1-yl)butyl-3-brompyrazolo[1,5-a]pyridin-2-ylcarbamid

### Synthese analog zu Beispiel 39.

### Ausbeute: 149 mg (74% über 2 Reaktionsschritte).

Smp.: 108°C. IR (NaCl): 3413, 3326, 2938, 2817, 1668, 1542, 1500, 1240. ¹H NMR (CDCl₃, 360 MHz) δ (ppm): 1.65-1.74 (m, 4H, CH₂-CH₂); 2.45-2.49 (m, 2H, CH₂Npip); 2.67 (m, 4H, pip); 3.10 (m, 4H, pip); 3.50-3.55 (m, 2H, CH₂NHCO); 3.86 (s, 3H, CH₃O); 6.84-7.01 (m, 5H, Phenyl, H-6); 7.21-7.25 (m, 1 H, H-5); 7.30 (br s, 1 H, NHCO); 7.60 (br d, J=8.9 Hz, 1H, H-4); 8.33 (d, J=7.1 Hz, 1H, H-7). ¹³C NMR (CDCl₃, 90 MHz) δ (ppm): 161.0, 152.3, 143.3, 141.4, 140.1, 128.6, 124.6, 122.8, 121.0, 118.2, 118.0, 114.5, 111.2, 85.0, 58.2, 55.3, 53.5, 50.6, 39.2, 27.6, 24.4.
C H N (%):C₂₃H₂₈BrN₅O₂
Ber.: C 56.79; H 5.80; N 14.40; Gef.: C 56.71; H 5.91; N 14.44.

### Beispiel 11:

### N-4-(4-(2-Methoxyphenyl)piperazin-9-yl)butyl-3-chlorpyrazolo[1,5-a]pyridin-2-ylcarbamid

### Synthese analog zu Beispiel 39.

### Ausbeute: 55 mg (61 % über 2 Reaktionsschritte).

Smp.: 121°C. MS: m/z 441 (M⁺), 443 ((M+2)⁺); IR (NaCl): 3332, 2937, 2815,1668, 1635, 1545, 1500, 1240. ¹H NMR (CDCl₃, 360 MHz) δ (ppm): 1.65-1.74 (m, 4H, CH₂-CH₂); 2.45-2.49 (m, 2H, CH₂Npip); 2.67 (m, 4H, pip); 3.10 (m, 4H, pip); 3.50-3.56 (m, 2H, CH₂NHCO); 3.86 (s, 3H, CH₃O); 6.84-6.89 (m, 1H, H-6); 6.90-7.01 (m, 4H, Phenyl); 7.20-7.27 (m, 2H, NHCO, H-5); 7.61 (br d, J=8.9 Hz, 1H, H-4); 8.31 (d, J=7.1 Hz, 1 H, H-7). ¹³C NMR (CDCl₃, 90 MHz) δ (ppm): 160.5, 152.3, 141.9, 141.3, 138.6, 123.5, 124 2, 122.8, 121.0, 118.2, 117.2, 114.4, 111.2, 101.0, 58.1, 55.3, 53.4, 50.5, 39.1, 27.6, 24.4.

### Beispiel 12:

### N-4-(4-(2-Methoxyphenyl)piperazin-1-yl)butyl-5-methylpyrazolo[1,5-a]pyridin-2-ylcarbamid

### Synthese analog zu Beispiel 39.

### Ausbeute: 60 mg (50% über 2 Reaktionsschritte).

Smp.: 119°C. MS: m/z 421 (M⁺), IR (NaCl): 3411, 3336, 2937, 2871, 1662, 1500, 1240. ¹H NMR (CDCl₃, 360 MHz) δ (ppm): 1.65-1.72 (m, 4H, CH₂-CH₂); 2.34 (s, 3H, CH₃); 2.44-2.48 (m, 2H, CH₂Npip); 2.66 (m, 4H, pip); 3.11 (m, 4H, pip); 3.49-3.54 (m, 2H, CH₂NHCO); 3.86 (s, 3H, CH₃O); 6.66 (d, J=7.1 Hz, 1 H, H-6); 6.85-7.01 (m, 5H, Phenyl, H-3); 7.25 (br s, 1 H, NHCO); 7.33 (s, 1 H, H-4); 8.23 (d, J=7.1Hz, 1H, H-7).
C H N (%):C₂₄H₃₁N₅O₂
Ber.: C 68.38; H 7.41; N 16.61; Gef.: C 67.99; H 7.51; N 16.69.

### Beispiel 13:

### N-4-(4-(2-Methoxyphenyl)piperazin-1-yl)butyl-6-brompyrazolo[1,5-a]pyridin-2-ylcarbamid

### Synthese analog zu Beispiel 39.

### Ausbeute: 85 mg (84% über 2 Reaktionsschritte).

Smp.: 104°C. MS: 485 m/z (M⁺), 487 ((M+2)⁺); IR (NaCl): 3320, 2937,2815,1662, 1552, 1502, 1240, ¹H NMR (CDCl₃, 360 MHz) δ (ppm): 1.65-1.73 (m, 4H,-CH₂-CH₂); 2.45-2.49 (m, 2H, CH₂Npip); 2.67 (m, 4H, pip); 3.12 (m, 4H, pip); 3.49-3.55 (m, 2H, CH₂NHCO); 3.86 (s, 3H, CH₃O); 6.84-7.01 (m, 4H, Phenyl); 7.07 (s, 1 H, H-3); 7.20-7.23 (m, 1 H, H-5); 7.28 (br s, 1 H, NHCO); 7.49 (d, J=9.6 Hz, 1H, H-4); 8.51 (br s, 1 H, H-7). ¹³C NMR (CDCl₃, 90 MHz) ö (ppm): 161.7, 152.3, 148.5, 141.3, 139.7, 128.6, 127.4, 122.9, 120.9, 119.6, 118.1, 111.2, 108.1, 98.8, 58.1, 55.3, 53.4, 50.5, 39.2, 27.6, 24.3.
C H N (%):C₂₃H₂₈BrN₅O₂
Ber.: C 56.79; H 5.80; N 14.40; Gef.: C 56.39; H 5.94; N 1429.

### Beispiel 14:

### N-4-(4-(2-Methoxyphenyl)piperazin-1-yl)butyl-3-methoxycarbonylpyrazolo[1,5-a]pyridin-2-ylcarbamid

0,12 g (0,5 mmol) Pyrazolo[1,5-*a*]pyridin-2,3-dicarbonsäuredimethylester, 0,24 g (1,0 mmol) 1-(4-Aminobutyl)-4-(2-Methoxyphenyl)piperazin und 4,0 mg (0,08 mmol) Natriumcyanid werden mit 2 ml Methanol in einem Druckrohr gemischt und 62 Stunde bei 50°C gerührt. Anschließend wird das Lösungsmittel im Vakuum abgedampft um das Produkt zu erhalten.
Ausbeute 0,21 g (89%).
Smp.: 149°C. IR (NaCl): 3286, 2940, 2815, 1683, 1660, 1502, 1444, 1240, 750.¹H NMR (CDCl₃, 360 MHz) δ (ppm): 1.67-1.77 (m, 4H, CH₂-CH₂); 2.47-2.51 (m, 2H, CH₂Npip); 2.67 (m, 4H, pip); 3.08 (m, 4H, pip); 3.56-3.61 (m, 2H, CH₂NHCO); 3.85 (s, 3H, CH₃O); 4.00 (s, 3H, CH₃O); 6.84-7.01 (m, 4H, Phenyl) 7.03-7.07 (m, 1 H, H-6), 7.45-7.50 (m, 1 H, H-5), 8.15 (br d, J=8.9 Hz, 1 H, H-4), 8.64 (br d, J=6.7 Hz, 1H, H-7), 9.98 (br s, 1H, NHCO).¹³C NMR (CDCl₃, 90 MHz) δ (ppm): 165.4, 160.5, 152.3, 150.1, 142.3, 141.4, 129.7, 128.3, 122.8, 121.0, 120.5, 118.2, 1,15.1, 111.2, 100.6, 58.3, 55.3, 53.4, 52.2, 50.5, 39.7, 27.3, 24.3.
C H N (%):C₂₅H₃₁N₅O₄0.5 H₂O
Ber.: C 63.27; H 6.80; N 14.76; Gef.: C 62.94; H 6.73; N 14.74.

### Beispiel 15:

### N-4-(4-(2,3-Dichlorphenyl)piperazin-1-yl)bu-yl-3-methoxycarbonylpyrazolo[1,5-a]pyridin-2-ylcarbamid

Synthese analog zu Beispiel 14 und zusätzliche Reinigung durch Flashchromatographie (CH₂Cl₂-MeOH: 95-5).
Ausbeute: 0,20 g (78%)
Smp: 149.°C. MS: m/z 503 (M⁺), 505 ((M+2)⁺), 507 ((M+4)⁺). IR (NaCl): 3471, 3280, 3097, 2944, 2819, 1685, 1660, 1577, 1238. ¹H NMR (CDCl₃, 360 MHz) δ (ppm): 1.67-1.80 (m, 4H, CH₂-CH₂); 2.49 (t, J=7.1 Hz, 2H, CH₂Npip); 2.65 (m, 4H, pip); 3.05 (m, 4H, pip); 3.57-3.62 (m, 2H, CH₂NHCO); 4.01 (s, 3H, CH₃O); 6.91-6.94 (m, 1H, Phenyl), 7.04-7.08 (m, 1 H, H-6), 7.10-7.16 (m, 2H, Phenyl), 7.46-7.51 (m, 1 H, H-5), 8.14-8.17 (m, 1 H, H-4), 8.63-8.66 (m, 1 H, H-7), 10.03 (br s, 1 H, NHCO). ¹³C NMR (CDCl₃, 90 MHz) δ (ppm): 165.5, 160.5, 151.3, 150.0, 142.3, 133.9, 129.7, 128.3, 127.5, 127.4, 124.4, 120.5, 118.5, 115.1, 100.6, 58.2, 53.2, 52.2, 51.3, 39.7, 27.3, 24.3.
C H N (%):C₂₄H₂₁N₅O₃
Ber.: C 57.15; H 5.40; N 13.88; Gef.: C 57.00; H 5.34; N 13.86.

### Beispiel 16:

### Trans-N-(4-(4-(2-Methoxyphenyl)piperazin-1-yl)methylcyclohex-1-yl)methylpyrazolo[1,5-a]pyridin-2-ylcarbamid

0,025 g (0,15 mmol) Pyrazolo[1,5-*a*]pyridin-2-carbonsäure, 0,026 g (0,17 mmol) HOBt und 0,035g (0,17 mmol) N.N'-Dicyctohexyicarbodiimid werden mit 3 ml trockenem Methylenchlorid gemischt und 0.5 Stunden bei Raumtemperatur gerührt. Anschließend wird eine Lösung von 0,054 (0,16 mmol) trans-4-(4-Aminomethylcyclohex-1-yl)-1-(2-methoxyphenyl)piperazin in 2.5 ml Methylenchlorid zugetropft und 18 Stunden bei Raumtemperatur gerührt. Der ausfallende Feststoff wird abfiltriert und die Lösung im Vakuum abgedampft. Reinigung durch Flashchromatographie (CH₂Cl₂-MeOH: 95-5).
Ausbeute: 64 mg (90%).
Smp. 149°C. MS: m/z 461 (M⁺); IR (NaCl): 3419, 2917, 2845, 2813, 1667, 1635, 1551, 1500, 1240, 734. ¹H NMR (CDCl₃, 360 MHz) δ (ppm): 0.90-1.10 (m, 4H, CH₂-cyclohex); 1.53-1.62 (m, 2H, CH-cyclohex); 1.88 (t, J=10.7Hz, 4H, CH₂-cyclohex); 2.32 (d, J=6.7 Hz, 2H, CH₂Npip); 2.72 (m, 4H, pip); 3.13 (m, 4H, pip); 3.32-3.36 (m, 2H, CH₂NHCO); 3.85 (s, 3H, CH₃O); 6.82-7.02 (m, 5H, Phenyl, H-6), 7.05 (br s, 1 H, H-3), 7.13-7.16 (m, 2H, H-5, NHCO), 7.58 (br d, J=8.8 Hz, 1H, H-4), 8.38 (br d, J=7.1 Hz, 1 H, H-7). ¹³C NMR (CDCl₃, 90 MHz) δ (ppm): 162.1, 152.2, 148.0, 141.3, 141.1, 128.4, 123.6, 123.0, 121.0, 119.2, 118.2, 113.5, 111.1, 97.9, 65.3, 55.3, 53.7, 50.1, 45.3, 38.3, 34.7, 31.2, 30.4.

### Beispiel 17:

### Trans-N-(4-(4-(2,3-Dichlorphenyl)piperazin-1-yl)methylcyclohex-1-yl)methylpyrazolo[1,5-a]pyridin-2-ylcarbamid

### Synthese analog zu Beispiel 16.

### Ausbeute: 13 mg (16%).

Smp.: 138°C. MS: m/z 499 (M⁺), 501 ((M+2)⁺), 503 ((M+4)⁺). IR (NaCl): 2920, 2844, 1669, 1635,1557, 1448, 1239. ¹H NMR (CDCl₃, 360 MHz) δ (ppm): 0.83-1.11 (m, 4H, CH₂-cyclohex); 1.50-1.61 (m, 2H, CH-cyclohex); 1.85-1.90 (m, 4H, CH₂-cyclohex); 2.17-2.22 (m, 2H, CH₂Npip); 2.50-2.58 (m, 4H, pip); 3.05-3.19 (m, 4H, pip); 3.36 (t, J=6.4 Hz, 2H, CH₂NHCO); 6.82-6.87 (m, 1 H, H-6); 6.92-6.98 (m, 1 H, Phenyl), 7.06 (br s, 1H, H-3); 7.10-7.18 (m, 4H, Phenyl, H-5, NHCO); 7.59 (br d, J=9.2 Hz, 1H, H-4); 8.38 (br d, J=7.1 Hz, 1 H, H-7).¹³C NMR (CDCl₃, 90 MHz) ö (ppm): 162.1, 151.5, 148.1, 141.4, 134.0, 128.4, 127.4, 124.4, 123.6, 119.3, 118.5, 113.5, 98.0, 91.6, 65.4, 53.7, 51.3, 45.4, 38.5, 35.1, 31.2, 30.5. C H N (%):C₂₆H₃₁Cl₂N₅O₂
Ber.: C 62.40; H 6.24; N 13.99; Gef.: C 62.55; H 6.25; N 13.53.

### Beispiel 22:

### Trans-N-(4-(4-(2-Methoxyphenyl)piperazin-1-yl)methylcyclohex-1-yl)methylpyrazolo[1,5-a]pyridin-3-ylcarbamid

Synthese analog zu Beispiel 16.
Ausbeute: 41 mg (66%).
Smp.:76°C. MS: m/z 461 (M⁺); IR (NaCl): 3313, 2916, 2844, 2813, 1637, 1627, 1556, 1531, 1499, 1240, 749. ¹H NMR (CDCl₃, 360 MHz) δ (ppm): 0.89-1.09 (m, 4H, CH₂-cyclohex); 1.50-1.62 (m, 2H, CH-cylohex); 1.88 (t, J=10.8 Hz, 4H, CH₂-cyclohex); 2.25 (d, J=7.1 Hz, 2H, CH₂Npip); 2.64 (m, 4H, pip); 3.10 (m, 4H, pip); 3.31-3.35 (m, 2H, CH₂NHCO); 3.85 (s, 3H, CH₃O); 5.94 (m, 1 H, NHCO); 6.84-7.01 (m, 5H, Phenyl, H-6), 7.32-7.37 (m, 1 H, H-5), 8.14 (br s, 1H, H-2), 8.31 (br d, J=8.8 Hz, 1H, H-4), 8.48 (br d, J=7.1 Hz, 1H, H-7). ¹³C NMR(CDCl₃, 90 MHz) δ (ppm): 163.3, 152.3, 141.4, 140.6, 140.1, 128.8, 126.3, 122.8, 121.0, 119.7, 118.2, 113.5, 111.1,106.9, 65.4, 55.3, 53.9, 50.5, 45.5, 38.5,35.0,31.2,30.6.

### Beispiel 23:

### Trans-N-(4-(4-(2,3-Dichlorphenyl)piperazin-1-yl)methylcyclohex-1-yl)methylpyrazolo[1,5-a]pyridin-3-ylcarbamid

Synthese analog zu Beispiel 16.
Ausbeute: 42 mg (51 %).
Smp.: 68°C. MS: m/z 499 (M⁺), 501 ((M+2)⁺), 503 ((M+4)⁺). IR (NaCl): 3308,2920,2847, 1637, 1555, 1530, 1449,1272, 1240, 745.¹H NMR (CDCl₃, 360 MHz) δ (ppm): 0.88-1.10 (m, 4H, CH₂-cyclohex); 1.45-1.61 (m, 2H, CH-cyclohex); 1.87-1.91 (m, 4H, CH₂-cyclohex); 2.17-2.23 (m, 2H, CH₂Npip); 2.54-2.58 (m, 4H, pip); 3.06-3.19 (m, 4H, pip); 3.32-3.36 (m, 2H, CH₂NHCO); 5.90 (s, 1 H, NHCO); 6.82-6.87 (m, 1H, H-6); 6.90-6.97 (m, 1 H, Phenyl), 7.11-7.18 (m, 2H, Phenyl); 7.34-7.37 (m, 1 H, H-5); 8.13 (s, 1 H, H-2); 8.32 (br d, J=8.9 Hz, 1H, H-4); 8.48 (br d, J=7.1 Hz, 1H, H-7). ¹³C NMR (CDCl₃, 90 MHz) δ (ppm): 163.3, 151.4, 140.6, 140.1, 133.6, 128.8,127.4, 126.3, 124.4, 123.5, 119.0, 115.9, 113.6, 106.9, 65.4, 53.7, 51.3, 45.5, 38.6, 35.1, 31.2, 30.6.

### Beispiel 28:

### N-4-(4-(2-Methoxyphenyl)piperazin-1-yl)butyl-3-brompyrazolo[1,5-a]pyridin-5-ylcarbamid

Synthese analog zu Beispiel 39.
Ausbeute: 59 mg (59% über 2 Reaktionsschritte).
Smp.: 172°C. IR (NaCl): 3316, 2939, 2817, 1648, 1546, 1500, 1240. ¹H NMR (CDCl₃, 360 MHz) δ (ppm): 1.69-1.78 (m, 4H, CH₂-CH₂); 2.49-2.53 (m, 2H, CH₂Npip); 2.68 (m, 4H, pip); 3.04 (m, 4H, pip); 3.50-3.55 (m, 2H, CH₂NHCO); 3.84 (s, 3H, CH₃O); 6.76-7.01 (m, 4H, Phenyl); 7.20-7.25 (m, 2H, NHCO, H-6); 7.88 (s, 1 H, H-4); 7.98 (s, 1 H, H-2); 8.42-8.44 (m, 1H, H-7). ¹³C NMR (CDCl₃, 90 MHz) δ (ppm): 165.4, 152.2, 142.7, 140.9, 137.1, 131.2, 129.1, 123.0, 120.9,118.1, 115.5,111.1, 111.0, 86.4, 58.0, 55.3, 53.4, 50.4, 40.2, 27.3, 24.4.
C H N (%):C₂₃H₂₈BrN₅O₂
Ber.: C 56.79; H 5.80; N 14.40; Gef.: C 56.67; H 5.86; N 14.21.

### Beispiel 29:

### N-4-(4-(2-Methoxyphenyl)piperazin-1yl)butyl-3-chlorpyrazolo[1,5-a]pyridin-5-ylcarbamid

Synthese analog zu Beispiel 39.
Ausbeute: 62 mg (54% über 2 Reaktionsschritte).
Smp.: 155°C. MS: m/z 441 (M⁺), 443 ((M+2)⁺); IR (NaCl): 3307, 2940, 2817,1647, 1546, 1500, 1240. ¹H NMR (CDCl₃, 360 MHz) δ (ppm): 1.67-1.77 (m, 4H, CH₂-CH₂); 2.49-2.52 (m, 2H, CH₂Npip); 2.67 (m, 4H, pip); 3.04 (m, 4H, pip); 3.49-3.54 (m, 2H, CH₂NHCO); 3.84 (s, 3H, CH₃O); 6.77-7.01 (m, 4H, Phenyl); 7.21 (d, J=7.4 Hz, 1H, H-6); 7.18-7.22 (m, 1 H, NHCO); 7.91 (s, 1H, H-4); 7.95 (s, 1 H, H-2); 8.40 (d, J=7.4 Hz, 1H, H-7). ¹³C NMR (CDCl₃, 90 MHz) δ (ppm): 165.4, 152.2, 141.0, 140.6, 135.6, 130.7, 129.1, 123.0, 121.0, 118.1, 114.9, 111.2, 110.9, 102.5, 58.0, 55.3, 53.5, 50.4, 40.2, 27.4, 24.5.
C H N (%):C₂₃H₂₈ClN₅O₂·0.2H₂O
Ber.: C 62.00; H 6.42; N 15.72; Gef.: C 61.66; H 6.41; N 15.72.

### Beispiel 30

### N-5-(4-(2-Methoxyphenyl)piperazin-1-yl)pentylpyrazolo[1 ,5-a]pyridin-5-ylcarbamid

Synthese analog zu Beispiel 16.
Ausbeute: 63 mg (85%) (wachsartig).
IR (NaCl): 3309, 2938, 2832, 1650, 1546, 1502, 1243. ¹H NMR (CDCl₃, 360 MHz) ö (ppm): 1.42-1.50 (m, 2H, CH₂-CH₂-CH₂); 1.58-1.73 (m, 4H, CH₂-CH₂-CH₂); 2.45 (t, J=7.45 Hz, 2H, CH₂Npip); 2.68 (m, 4H, pip); 3.12 (m, 4H, pip); 3.47-3.52 (m, 2H, CH₂NHCO); 3.86 (s, 3H, CH₃O); 6.35 (br s, 1H, NHCO); 6.67 (br d, J=2.1 Hz, 1 H, H-3); 6.85-7.02 (m, 4H, Phenyl); 7.12 (br d, J=7.1 Hz, 1 H, H-6), 8.01 (s, 1 H, H-4), 8.01 (d, J=2.1 Hz, 1H, H-2), 8.49 (br d, J=7.1 Hz, 1H, H-7). ¹³C NMR (CDCl₃, 90 MHz) δ (ppm): 155.6, 152.2, 142.6, 141.2, 139,0, 129.7, 128.6, 122.9, 121.0,118.2,117.4, 111.1, 109.6, 99.3, 58.4, 55.3, 53.4, 50.4, 40.1, 29.4, 26.3, 24.8.

### Beispiel 39:

### N-4-(4-(2-Methoxyphenyl)piperazin-1-yl)butyl-4,5,6,7-tetrahydropyrazolo[1,5-a]pyridin-2-ylcarbamid

0,050 g (0,3 mmol) 4,5,6,7-Tetrahydropyrazolo[1,5-*a*]pyridin-2-carbonsäure werden in 2 ml trockenem Toluol gelöst. Es werden 80 µl (0,9 mmol) Oxalylchlorid zugegeben und bis zur einsetzenden Gasentwicklung auf 40°C enwärmt. Anschließend rührt man zunächst 1 Stunde bei Raumtemperatur, danach 3,5 Stunden bei 60°C. Das Lösungsmittel wird im Vakuum-eingedampft und der Rückstand mit 2 ml abs. Methylenchlorid versetzt. Das Säurechlorid wird unter Rühren bei 0°C zu einer Lösung aus 0,36 mmol 4-(4-Aminobutyl)-1-(2-methoxyphenyl)piperazin (0,095 g) in 2 ml abs. Methylenchlorid hinzugefügt. Die Lösung wird langsam auf Raumtemperatur erwärmt und 1 Stunde gerührt. Nach Zugabe von NaHCO₃-Lösung wird mit Methylenchlorid extrahiert, die organische Phase mit MgSO₄ getrocknet und im Vakuum abgedampft. Die Reinigung erfolgt durch FlashChromatographie an Kieselgel (CH₂Cl₂-MeOH:95-5).
Ausbeute: 93 mg (75% über 2 Reaktionschritte).
Smp.:62°C. MS: 411 m/z (M⁺); IR (NaCl): 3355, 2929, 2852, 1862, 1531, 1240. ¹H NMR (CDCl₃, 360 MHz) δ (ppm): 1.61-1.65 (m, 4H, CH₂-CH₂); 1.83-1.90 (m, 2H, H-5); 2.01-2.08 (m, 2H, H-6); 2.44 (t, J=6.7Hz, 2H, CH₂Npip); 2.65 (m, 4H, pip); 2.81 (t, J=6.4 Hz, 2H, H-4); 3.10 (m, 4H, pip); 3.41-3.47 (m, 2H, CH₂NHCO); 3.86 (s, 3H, CH₃O); 4.09-4.12 (m, 2H, H-7); 6.49 (s, 1 H, H-3), 6.84-7.00 (m, 4H, Phenyl). ¹³C NMR (CDCl₃, 90 MHz) δ (ppm): 162.4, 152.3, 145.9, 141.4, 140.7, 122.8, 121.0, 118.2, 111.2, 103.6, 58.2, 55.3, 53.4, 50.6, 48.2, 38.9, 27.7, 24.4, 23.3, 22.6, 20.3.

### Beispiel 40:

### (±)-N-4-(4-(2-Methoxyphenyl)piperazin-1-yl)butyl-5-methyl-4,5,6,7-tetrahydropyrazolo[1,5-a]pyridin-2-ylcarbamid

Synthese analog zu Beispiel 39.
Ausbeute: 107 mg (91 % über 2 Reaktionsschritte).
Smp.: 65°C. MS: 425 m/z (M⁺); IR (NaCl): 3343, 2937, 2815, 1662, 1533, 1500, 1240. ¹H NMR (CDCl₃, 360 MHz) δ (ppm): 1.12 (d, 3H, J=6.4 Hz, CH₃); 1.60-1.67 (m, 4H, CH₂-CH₂); 1.68-1.78 (m, 1H, H-5); 1.96-2.08 (m, 2H, H-6);2.36 (dd,J=16.Hz, J=10.3 Hz, 1H, H-4); 2.44 (t, J=6.7 Hz, 2H, CH₂Npip); 2.65 (m, 4H, pip); 2.93 (dd, J=16.3 Hz, J=5.0 Hz, 1 H, H-4); 3.10 (m, 4H, pip); 3.41-3.46 (m, 2H, CH₂NHCO); 3.86 (s, 3H, CH₃O); 3.98-4.06 (m, 1 H, H-7); 4.19-4.25 (m, 1 H, H-7); 6.47 (s, 1 H, H-3), 6.84-7.01 (m, 4H, Phenyl). ¹³C NMR (CDCl₃, 90 MHz) δ (ppm): 162.4, 152.3, 146.2, 141.4, 140.7, 122.8, 120.9, 118.2, 111.2, 103.5, 58.2, 55.3, 53.4, 50.6, 47.5, 38.9, 31.2, 30.8, 27.7, 27.1, 24.3, 20.8.

### Beispiel 49:

### N-4-(Chroman-8-yl)piperazin-1-yl)butylindolizin-2-ylcarbamid

Synthese analog zu Beispiel 1.
Ausbeute: 30 mg (69%).
Smp.: 75°C. MS: m/z 432 (M⁺). IR (NaCl): 3321; 2935; 2873; 2817; 1636; 1558; 1266; 1143; 754. ¹H NMR (CDCl₃, 360 MHz) δ (ppm): 1.67-1.69 (m, 4H, CH₂-CH₂); 1.96-2.03 (m, 2H, O-CH₂-CH₂-CH₂); 2.51-2.55 (m, 2H, CH₂Npip); 2.70-2.74 (m, 4H, pip); 2.77-2.80 (m, 2H, O-CH₂-CH₂-CH₂); 3.10-3.14 (m, 4H, pip); 3.46-3.51 (m, 2H, CH₂NHCO); 4.23-4.26 (m, 2H, O-CH₂-CH₂-CH₂); 6.38 (br t, J=4.1 Hz, 1H, NHCO); 6.51 (ddd, J= 6.9 Hz, J= 6.6 Hz, J=1.2 Hz, 1H, H-6); 6.59 (s, 1H, H-1); 6.68 (ddd, 1 H, J=9.0 Hz, J=6.6 Hz, J=0.9 Hz, H-7); 6.71-6.80 (m, 3H, H-arom); 7.32 (d, J=9.0 Hz, 1H, H-8); 7.76 (d, J=1.1 Hz, 1H, H-3); 7.86 (dd, J=7.1 Hz, J=1.1 Hz, 1 H, H-5). ¹³C NMR (CDCl₃, 90 MHz) δ (ppm): 164.9; 147.6; 140.6; 132.8; 125.4; 124.0; 123.9; 122.7; 119.9; 119.8; 118.2; 115.9; 113.9; 111.8; 97.2; 66.5; 58.0; 53.4; 50.3; 39.2; 27.5; 25.1; 24.0; 22.7; 21.0.

### Beispiel 69:

### N-4-(4-(2-Methoxyphenyl)piperazin-1-yl)butylindolizin-1-ylcarbamid

Zur Synthese werden 0,039 g Indolizin-1-carbonsäure (0,24 mmol) in 6 ml trockenem Methylenchlorid gelöst. Dann werden 0,14 ml (0,84 mmol) trockenes DIPEA zugegeben und anschließend wird 0,084 g (0,26 mmol) des in 0,5 ml trockenem DMF gelösten TBTU bei 0° C langsam zugetropft und 30 Minuten bei Raumtemperatur gerührt. Die Reaktionslösung wird erneut auf 0 °C gekühlt und eine Lösung von 0,071 g (0,27 mmol) 4-(4-(2-Methoxyphenyl)piperazin-1-yl)butylamin in 4 ml trockenem Methylenchlorid zugetropft. Nach 1 Stunde Rühren bei 0°C wird der Reaktionsansatz mit Methylenchlorid verdünnt und mit gesättigter NaHO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt
Ausbeute: 59 mg (61 %) farbloser Feststoff.
Smp.: 54-56 °C. MS: m/z 407 (M⁺). IR (NaCl): 3414; 3339; 2934; 2817; 1634; 1500; 1241; 1028; 749. ¹H NMR (CDCl₃, 360 MHz) δ (ppm): 1.64-1.72 (m, 4H, CH₂-CH₂); 2.52-2.54 (m, 2H, CH₂N); 2.68-2.76 (m, 4H, pip); 3.06-3.16 (m, 4H, pip); 3.51-3.56 (m, 2H, CH₂NHCO); 6.08-6.14 (m, 1 H, NHCO); 6.61-6.66 (m, 1H); 6.85-6.87 (m, 1 H); 6.90-7.02 (m, 5H, H-arom); 7.20 (d, J=2.7 Hz, 1 H); 7.93 (d, J=7.2 Hz, 1 H); 8.32 (d, J=8.9 Hz, 1 H).

### Beispiel 70:

### N-4-(4-(2-Methoxyphenyl)piperazin-1-yl)butyl-5,6,7,8-tetrahydroindolizin-2-ylcarbamid

0,020 g Tetrahydroindolizin-2-carbonsäure (0,12 mmol) werden wie für Beispiel 69 beschrieben umgesetzt und durch Flashchromatographie (SiO₂; CH₂Cl₂-CH₃OH:98-2) gereinigt. '
Ausbeute: 29 mg (59%).
Smp.: 51-53 °C; MS: m/z 411 (M⁺); IR (NaCl): 3325; 2938; 2817;1629; 1500; 1241; 1028; 750. ¹H NMR (CDCl₃, 360 MHz) δ (ppm): 1.59-1.66 (m, 4H,CH₂-CH₂); 1.76-1.83 (m, 2H, H-7); 1.88-1.97 (m, 2H, H-6); 2.44 (t, J=6.8 Hz, 2H, CH₂N); 2.62-2.69 (m, 4H, pip); 2.73 (dd, J=6.2 Hz, 2H, H-8); 3.07-3.13 (m, 4H, pip); 3.38-3.43 (m, 2H, CH₂NHCO); 3.86 (s, 3H, OCH₃); 3.92 (dd, J=6.0 Hz, 2H, H-5); 5.88 (br t, J=5.4 Hz, 1 H, NHCO); 6.00 (dd, J=0.9 Hz, J=0.9 Hz, 1H, H-1); 6.83-7.01 (m, 4H, H-arom); 7.05 (d, J=1.8 Hz, 1H, H-3).

### Beispiel 71:

### N-4-(4-(2,3-Dichlorphenyl)piperazin-1-yl)butyl-5,6,7,8-tetrahydroindolizin-2-ylcarbamid

0,040 g Tetrahydroindolizin-2-carbonsäure (0,24 mmol) werden wie für Beispiel 69 beschrieben umgesetzt und durch Flashchromatographie (SiO₂; CH₂Cl₂-CH₃OH:98-2) gereinigt
Ausbeute: 49 mg (45%).
Smp.: 64-66 °C; MS: m/z 448 (M⁺), 450 (M⁺+2); IR (NaCl): 3329; 2940; 2863; 2822; 1627; 1243,755. ¹HNMR (CDCl₃, 360 MHz) ö (ppm): 1.64-1.71 (m, 4H, CH₂-CH₂); 1.81-1.85 (m, 2H, H-7); 1.93-1.97 (m, 2H, H-6); 2.64 (t, J=6.8 Hz,2H, CH₂N); 2.75 (dd, J=6.4 Hz, 2H, H-8); 2.78-2.85 (m, 4H, pip); 3.13-3.17 (m, 4H, pip); 3.42-3.46 (m, 2H, CH₂NHCO); 3.94 (dd, J=6.0 Hz, 2H, H-5); 6.03 (br t, J=5.3 Hz, 1 H, NHCO); 6.06 (br s, 1 H, H-1); 6.98 (dd, J=1.7 Hz, J=7.7 Hz, 1 H, H-arom); 7.09 (d, J=1.9 Hz, 1 H, H-3); 7.15-7.20 (m, 2H, H-arom).

### Beispiel 72:

### N-4-(4-(2-Methoxyphenyl)piperazin-1-yl)butyl-1-cyano-2-methyl-indolizin-3ylcarbamid

Die nach Methode A7 erhaltene 1-Cyano-2-methylindolizin-3-carbonsäure (0,031 g (0,19 mmol)) werden wie für Beispiel 69 beschrieben umgesetzt und durch Flashchromatographie (SiO₂; CH₂Cl₂-CH₃OH:98-2) gereinigt
Ausbeute: 60 mg (71 %).
Smp.: 63-65 °C; MS: m/z 445 (M⁺); IR (NaCl): 3347; 2939; 2817; 2211; 1638; 1512; 1500; 1241; 1027; 750. ¹H NMR (CDCl₃, 360 MHz) δ (ppm): 1.64-1.76 (m, 4H.CH₂-CH₂); 2.48 (t, J=6.9 Hz, 2H, CH₂N); 2.59-2.69 (m, 4H, pip); 2.63 (s, 3H, CH₃); 2.98-3.07 (m, 4H, pip); 3.49-3.56 (m, 2H, CH₂NHCO); 3.85 (s, 3H, OCH₃); 6.29 (br t, J=3.5 Hz, 1 H, NHCO); 6.83-6.93 (m, 4H, H-arom, H-6); 6.96-7.01 (m, 1H, H-arom); 7.20 (ddd, J=1.0 Hz, J=6.8 Hz, J=8.9 Hz,1 H, H-7); 7.59 (ddd, J=1.2 Hz, J=1.2 Hz, J=8.9 Hz, 1 H, H-8); 9.34 (ddd, J=1.0 Hz, J=1.0 Hz, J=7.2 Hz, 1H, H-5).

### Beispiel 73:

### N-4-(4-Phenylpiperazin-1-yl)butylpyrazolo[1,5-a]pyridin-2-ylcarbamid

Zu 0,039 g in 6 ml trockenem Methylenchlorid gelöstem Pyrazolo[1,5-*a*]pyridin-2-carbonsäure (0,24 mmol) werden 0,14 ml (0,84 mmol) trockenes DIPEA gegeben. Danach werden 0,042 g (0,13 mmol) in 0.5 ml trockenem DMF gelösten TBTU bei 0°C langsam zugetropft und 30 Minuten bei Raumtemperatur gerührt. Die Reaktionslösung wird erneut auf 0 °C gekühlt und eine Lösung von 0,065 g (0,28 mmol) 4-(4-Phenylpiperazin-1-yl)butylamin (hergestellt nach den Vorschriften für Amine des Typs C2) in 4 ml trockenem Methylenchlorid zugetropft. Nach 1 Stunde wird der Reaktionsansatz in mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt.
Ausbeute: 64 mg (71 %) weißer Feststoff.
Smp.: 164-166 °C; MS: m/z 377 (M⁺); IR (NaCl): 3380; 2936; 2819; 1655; 1633; 1547; 1503; 1241; 764; 749. ¹H NMR (CDCl₃, 360 MHz) δ (ppm): 1.68-1.75 (m, 4H, CH₂-CH₂); 2.47 (t, J=7.0 Hz, 2H, CH₂N); 2.61-2.65 (m, 4H, pip); 3.20-3.25 (m, 4H, pip); 3.50-3.55 (m, 2H, CH₂NHCO); 6.81-6.87 (m, 2H, H-arom, H-6);6,91-6.94(m, 2H. H-arom); 7.05 (d, J=0.9 Hz, 1H, H-3); 7.13 (ddd, J=1.0 Hz, J=6.7 Hz, J=8.9 Hz, 1H, H-5); 7.22-7.28 (m, 3H, H-arom, NHCO); 7.58 (br d, J=9.0 Hz, 1H, H-4); 8.34 (br d, J=1.0 Hz, J=7.2 Hz, 1 H, H-7).

### Beispiel 74:

### N-4-(4-(2-Methylphenyl)piperazin-1-yl)butylpyrazolo[1,5-a]pyridin-2-ylcarbamid

Synthese analog zu Beispiel 73.
Ausbeute: 75 mg (80 %) gelblicher Feststoff.
Smp.: 99-101 °C; MS: m/z 391 (M⁺); IR (NaCl): 3412; 2937; 2855; 2812; 1663; 1552; 1492; 1227; 1042; 764. ¹H NMR (CDCl₃, 360 MHz) δ (ppm): 1.66-1.77 (m, 4H, CH₂-CH₂); 2.32 (s, 3H, CH₃); 2.50 (t, J=7.0 Hz, 2H, CH₂N); 2.56-2.73 (m, 4H, pip); 2.94-3.03 (m, 4H, pip); 3.53-3.60 (m, 2H, CH₂NHCO); 6.87 (dd, J=6.2 Hz, J=6.2 Hz, 1H, H-6); 6.99 (dd, J=7.2 Hz, J=7.2 Hz, 1 H, H-5); 7.03-7.05 (m, 1 H, H-arom); 7.07 (br s, 1 H, H-3); 7.14-7.21 (m, 3H, H-arom); 7.31 (br t, J=5.7 Hz, 1H, NHCO); 7.61 (br d, J=9.0 Hz, 1H, H-4); 8.39 (br d, J=6.8 Hz, 1H, H-7). ¹³C NMR (CDCl₃, 90 MHz) δ (ppm): 162.2; 151.5; 148.1; 141.3; 132.6; 131.0; 128.4; 126.5; 123.6; 123.1; 119.3; 119.0; 113.5; 97.9; 58.1; 53.4; 51.7; 39.1; 27.6; 24.3; 17.8.

### Beispiel 75:

### N-4-(4-(2-Biphenyl)piperazin-1-yl)butylpyrazolo[1,5-alpyddin-2-ylcalbamid

Synthese analog zu Beispiel 73.
Ausbeute: 83 mg (71 %) farbloses Öl.

MS: m/z 454 (M⁺). IR (NaCl): 3412; 3331; 2939; 2814; 1665; 1635; 1551; 1225; 1146; 1045; 741. ¹H NMR (CDCl₃, 360 MHz) δ (ppm): 1.56-1.68 (m, 4H, CH₂-CH₂); 2.36-2.39 (m, 6H, CH₂N, pip); 2.86-2.89 (m, 4H, pip); 3.46-3.50 (m, 2H, CH₂NHCO); 6.84 (ddd, J=1.1 Hz, J=6.8 Hz, J=6.8 Hz, 1 H, H-6); 7.01-7.07 (m, 3H, H-arom, H-3); 7.13 (dd, J=6.8 Hz, J=8.9 Hz, 1H, H-5); 7.22-7.29 (m, 4H, H-arom I, NHCO); 7.37-7.40 (m, 2H, H-arom); 7.58 (d, J=8.9 Hz, 1 H, H-4); 7.61-7.63 (m , 2H, H-arom); 8.34 (d, J=6.4 Hz, 1 H, H-7).

### Beispiel 76:

### N-4-(4-(2-Ethoxyphenyl)piperazin-1-yl)butylpyrazolo[1,5-a]pyridin-2-ylcarbamid

Synthese analog zu Beispiel 73.
Ausbeute: 24 mg (47 %) gelbes Öl.
Smp.: 118-120 °C; MS: m/z 422 (M⁺); IR (NaCl): 3411; 2935; 2815; 11663; 1552; 1500; 1241; 1043; 748. ¹H NMR (CDCl₃, 360 MHz) δ (ppm): 1.45 (t, J=6.9 Hz, 3H, O-CH₂-CH₃); 1.66-1.75 (m, 4H, CH₂-CH₂); 2.53 (t, J=6.4 Hz, 2H, CH₂N); 2.68-2.76 (m, 4H, pip); 3.09-3.22 (m, 4H, pip); 3.50-3.56 (m, 2H, CH₂NHCO); 4.06 (q, J=6.9 Hz, 2H, O-CH₂-CH₃); 6.82-6.86 (m, 2H, H-arom); 6.88-6.92 (m, 2H, H-arom, H-6); 6.94-6.98 (m, 1 H, H-arom); 7.05 (br s,1 H, H-3); 7.12-7.15 (m, 1 H, H-5); 7.30 (br t, J=4.2 Hz, 1H, NHCO); 7.58 (br d, J=9.1 Hz, 1H, H-4); 8.36 (d, J=6.8 Hz, 1 H, H-7). ¹³C NMR (CDCl₃, 90 MHz) δ (ppm): 162.2; 151.6; 148.1; 141.3; 141.2; 128.4; 123.6; 122.8; 121.0; 119.2; 118.2; 113.5; 112.6; 97.9; 63.6; 58.2; 53.5; 50.3; 39.1; 27.6; 24.1; 14.9.

### Beispiel 77:

### N-4-(4-(2-Benzyloxyphenyl)piperazin-1-yl)butylpyrazolo[1,5-a]pyridin-2-ylcarbamid

Synthese analog zu Beispiel 73.
Ausbeute: 86 mg (74 %) farbloses Öl.
MS: m/z 483 (M⁺). IR (NaCl): 3411; 2934; 2814; 1664; 1635; 1551; 1241; 1146; 1016; 750. ¹H NMR (CDCl₃, 360 MHz) δ (ppm): 1.64-1.74 (m, 4H, CH₂-CH₂); 2.46 (br t, J= 6.9 Hz, 2H, CH₂N); 2.61-2.67 (m, 4H, pip); 3.13-3.21 (m, 4H, pip); 3.50-3.55 (m,2H, CH₂NHCO); 5.13 (s, 2H, -CH₂-O); 6.83 (ddd, J=1.3 Hz, J=6.9 Hz, J=6.9 Hz, 1H, H-6); 6.93-7.96 (m, 4H, H-arom-CH₂); 7.05 (d, J=0.9 Hz, 1 H, H-3); 7.13 (ddd, J=1.0 Hz, J=6.7 Hz, J=8.9 Hz, 1 H, H-5); 7.25-7.33 (m, 2H. H-arom-CH₂, NHCO); 7.36-7.40 (m, 2H, H-arom); 7.43-7.46 (m, 2H, H-arom); 7.58 (d, J=8.9 Hz, 1 H, H-4); 8.35 (dd, J=1.1 Hz, J=7.0 Hz, 1 H, H-7).

### Beispiel 78:

### N-4-(4-(2-Methylmercaptophenyl)piperazin-1-yl)butylpyrazolo[1,5-a]pyridin-2-ylcarbamid

Synthese analog zu Beispiel 73.
Ausbeute: 72 mg (71 %) farbloses Öl.
Smp.: 50-52 °C;MS: m/z 423 (M⁺); IR (NaCl): 3412; 2940; 2816; 1664; 1636; 1552; 1519; 1259; 119; 1046; 752. ¹H NMR (CDCl₃, 360 MHz) ö (ppm): 1.66-1.75 (m, 4H, CH₂-CH₂); 2.41 (s, 3H, CH₃); 2.55 (t, J=6.9 Hz, 2H, CH₂N); 2.68-2.77 (m, 4H, pip); 3.03-3.10 (m, 4H, pip); 3.51-3.56 (m, 2H, CH₂NHCO); 6.84 (ddd, J=1.4 Hz, J=6.8 Hz, J=6.8 Hz, 1 H, H-6); 7.03-7.06 (m 1H, H-arom); 7.05 (d, J=0.7 Hz, 1 H, H-3); 7.07-7.12 (m, 3H, H-arom); 7.13 (ddd, J=1.1 Hz, J=6.8 Hz, J=8.9 Hz, 1 H, H-5); 7.30 (br t, J=5.0 Hz, 1 H, NHCO); 7.58 (d, J=8.9 Hz, 1H, H-4); 8.36 (dd, J=1.0 Hz, J=7.2 Hz, 1H, H-7).

### Beispiel 79:

### N-4-(4-(2-Fluorphenyl)piperazin-1-yl)butylpyrazolo[1,5 a]pyridin-2-ylcarbamid

Synthese analog zu Beispiel 73.
Ausbeute: 76 mg (80 %) weißer Feststoff.
Smp.: 98-100 °C; MS: m/z 395 (M⁺); IR (NaCl): 3414; 2940; 2853; 2819; 1664; 1636; 1552; 1501; 1239; 1039; 753. ¹H NMR (CDCl₃, 360 MHz) δ (ppm): 1.61-1.78 (m, 4H, CH₂-CH₂); 2.47 (t, J=7.0 Hz, 2H, CH₂N); 2.61-2.67 (m, 4H, pip); 3.10-3.16 (m, 4H, pip); 3.50-3.56 (m, 2H, CH₂NHCO); 6.84 (ddd, J=1.4 Hz, J=6.9 Hz, J=6.9 Hz, 1 H, H-6); 6.87-7.07 (m, 4H, H-arom); 7.05 (d, J=0.7 Hz, 1 H, H-3); 7.13 (ddd, J=1.1 Hz, J=6.8 Hz, J=8.9 Hz, 1H, H-5); 7.28 (br t, J=5.9 Hz, 1 H, NHCO); 7.58 (br d, J=8.9 Hz, 1 H, H-4); 8.35 (br dd, J=1.1 Hz, J=7.0 Hz, 1 H, H-7). ¹³C NMR (CDCl₃, 90 MHz) δ (ppm): 162.1; 155.8 (d, J=245, Fluorphenyl); 148.1; 141.3; 140.2 (d, J=8.6 Hz, Fluorphenyl); 128.4; 124.4 (d, J=4.0 Hz, Fluorphenyl); 123.6; 122.3 (d, J=7.9 Hz, Fluorphenyl); 119.2; 118.5 (d, J=3.3 Hz, Fluorphenyl); 116.1 (d, J=21 Hz, Fluorphenyl); 113.5; 97.9; 58.1; 55.6; 53.4; 50.5; 39.1; 27.6; 24.3.

### Beispiel 80:

### N-4-(4-(2-Trifluormethylphenyl)piperazin-1-yl)butylpyrazolo[1,5-a]pyridin-2-ylcarbamid

Synthese analog zu Beispiel 73.
Ausbeute: 83 mg (78 %) farbloses Öl.
MS: m/z 445 (M⁺). IR (NaCl): 3414; 3337; 2939; 2817; 1656; 1636; 1553; 1259; 1140; 1036; 766. ¹H NMR (CDCl₃, 360 MHz) δ (ppm): 1.67-1.74 (m, 4H, CH₂-CH₂); 2.52 (br t, J= 6.7 Hz, 2H, CH₂N); 2.55-2.60 (m, 4H, pip); 2.98-3.03 (m, 4H, pip); 3.51-3.56 (m, 2H, CH₂NHCO); 6.84 (ddd, J=1.4 Hz, J=6.9 Hz, J=6.9 Hz, 1H, H-6); 7.05 (d, J=0.7 Hz, 1 H, H-3); 7.14 (ddd, J=1.1 Hz, J=6.8 Hz, J=8.9 Hz, 1 H, H-5); 7.18-7.23 (m, 1 H, H-arom); 7.29 (br t, J=4.7 Hz, 1 H, NHCO); 7.35-7.38 (m 1 H, H-arom); 7.47-7.52 (m, 1 H, H-arom I); 7.57-7.63 (m, 2H, H-arom, H-4); 8.36 (dd, J=0.9 Hz, J=7.0 Hz, 1 H, H-7).

### Beispiel 81:

### N-4-(4-(2-Cyanophenyl)piperazin-1yl)butylpyrazolo[1,5-a]pyridin-2-ylcarbamid

Synthese analog zu Beispiel 73, wobei die Aminkomponente 4-(4-Phenylpiperazin-1-yl)butylamin nach den Vorschriften für Amine des Typs C1 hergestellt wurde.
Ausbeute: 62 mg (64 %) farbloser Feststoff.
Smp.:144-146 °C. MS: m/z 402 (M⁺). IR (NaCl): 3411; 2933; 2818; 2219; 1662; 1635; 1553; 1515; 1258; 1144; 1038; 761. ¹H NMR (CDCl₃, 360 MHz) ö (ppm): 1.62-1.75 (m, 4H, CH₂-CH₂); 2.49 (br t, J= 7.0 Hz, 2H, CH₂N); 2.66-2.69 (m, 4H, pip); 3.24-3.27 (m, 4H, pip); 3.50-3.56 (m, 2H, CH₂NHCO); 6.84 (ddd, J=1.2 Hz, J=6.9 Hz, J=6.9 Hz, 1 H, H-6); 6.96-7.01 (m, 2H, H-arom); 7.05 (d, J=0.9 Hz, 1H, H-3); 7.14 (ddd, J=1.0 Hz, J=6.7 Hz, J=8.9 Hz, 1 H, H-5); 7.27 (br t, J=3.9 Hz, 1 H, NHCO); 7.44-7.49 (m 1 H, Phenyl); 7.54-7.57 (m, 1 H, H-arom); 7.58 (d, J=8.9 Hz, 1 H, H-4); 8.36 (dd, J=0.9 Hz, J=7.0 Hz, 1 H, H-7).

### Beispiel 82:

### N-4-(4-(2-Nitrophenyl)piperazin-1yl)butylpyrazolo[1,5-a]pyridin-2-ylcarbamid

Synthese analog zu Beispiel 73.
Ausbeute: 61 mg (60 %) oranges Öl.
MS: m/z 422 (M⁺); IR (NaCl): 3410; 2935; 2818; 1635; 1553; 1516; 1341; 1231; 764; 752. ¹H NMR (CDCl₃, 360 MHz) δ (ppm): 1.60-1.78 (m, 4H, CH₂-CH₂); 2.47 (t, J=7.0 Hz, 2H, CH₂N); 2.60-2.63 (m, 4H, pip); 3.08-3.12 (m, 4H, pip); 3.50-3.55 (m, 2H, CH₂NHCO); 6.84 (ddd, J=1.4 Hz, J=6.9 Hz, J=6.9 Hz, 1H, H-6); 7.00-7.04 (m, 1 H, H-arom); 7.05 (d, J=0.9 Hz, 1 H, H-3); 7.11-7.16 (m, 2H, H-arom, H-5); 7.26 (br t, J=5.2 Hz, 1 H, NHCO); 7.42-7.48 (m, 1H, H-arom); 7.57-7.60 (m, 1 H, H-arom); 7.74 (dd, J=1.6 Hz, J=7.9 Hz, 1 H, H-4); 8.36 (dd, J=0.9 Hz, J=7.0 Hz, 1H, H-7).

### Beispiel 83:

### N-4-(4-(4-Methoxyphenyl)piperazin-1-yl)butylpyrazolo[1,5-a]pyridin-2-ylcarbamid

Synthese analog zu Beispiel 73.
Ausbeute: 24 mg (47 %) weißer Feststoff.
Smp.: 152-154 °C; MS: m/z 407 (M⁺); IR (NaCl): 3356; 2928; 2853; 2816;1653; 1634; 1550; 1512; 1243; 1033; 756. ¹H NMR (CDCl₃, 360 MHz) δ (ppm): 1.62-1.76 (m, 4H, CH₂-CH₂); 2.47 (t, J=6.8 Hz, 2H, CH₂N); 2.60-2.66 (m, 4H, pip); 3.08-3.16 (m, 4H, pip); 3.49-3.57 (m, 2H, CH₂NHCO); 3.77 (s, 1H, O-CH₃); 6.81-6.85 (m, 3H, H-arom, H-6); 6.86-6.92 (m, 2H, H-arom); 6.94-6.98 (m, 1 H, H-arom); 7.05 (d, J=0.9 Hz, 1 H, H-3); 7.13 (ddd, J=1.0 Hz, J=6.7 Hz, J=9.0 Hz, 1H, H-5); 7,27 (br t, J=4.8Hz, 1H, NHCO); 7.58 (ddd, J=1.2 Hz, J=1.2 Hz, J=9.0 Hz, 1 H, H-4); 8.34 (br dd, J=1.1 Hz, J=7.0 Hz, 1 H, H-7). ¹³C NMR (CDCl₃, 90 MHz) δ (ppm): 162.1; 153.8; 148.1; 145.8; 141.3; 128.4; 123.6; 119.3; 118.1; 114.4; 113.5; 97.9; 58.1; 55.6; 53.4; 50.5; 39.1; 27.6; 24.3.

### Beispiel 84:

### N-4-(4-(3-Chlor-2-methoxyphenyl)piperazin-1-yl)butylpyrazolo[1,5-a]pyridin-2-ylcarbamid

Synthese analog zu Beispiel 73.
Ausbeute: 36 mg (68%) farbloses Öl.
MS: m/z 440 ((M+2)⁺), 442 ((M+4)⁺). IR (NaCl): 2929; 2853; 2819; 1663; 1635; 1250; 743. ¹H NMR (CDCl₃, 360 MHz) δ (ppm): 1.68-1.71 (m, 4H,CH₂-CH₂); 2.47 (t, J=7.0 Hz, 2H, CH₂N); 2.61-2.64 (m, 4H, pip); 3.14-3.17 (m, 4H, pip); 3.50-3.55 (m, 2H, CH₂NHCO); 3.86 (s, 3H, OCH₃); 6.79 (dd, J=1.8 Hz, J=7.9 Hz, 1 H, H-arom); 6.84 (ddd, J=1.2 Hz, J=6.9 Hz, J=6.9 Hz, 1 H, H-6); 6.92-6.96 (m, 1 H, H-arom); 6.99 (dd, J=1.6 Hz, J=7.9 Hz, 1 H, H-arom); 7.05 (d, J=0.9 Hz, 1 H, H-3); 7.14 (ddd, J=1.1 Hz, J=6.8 Hz, H=8.9 Hz, 1H, H-5); 7.29 (br t, J=4.8 Hz, 1H, NHCO); 7.58 (br d, J=8.9 Hz, 1H, H-4); 8.35 (br dd, J=1.1 Hz, J=7.0 Hz, 1H, H-7).¹³C NMR (CDCl₃, 90 Mhz) δ (ppm): 162.4; 148.8; 141.4; 128.4; 124.8; 123.7; 119.3; 117.3; 113.6; 97.8; 59.3; 57.8; 53.4; 49.2; 38.6; 27.4; 21.0.

### Beispiel 85:

### N-4-(4-(2,3-Dimethylphenyl)piperazin-1-yl)butylpyrazolo[1,5-a]pyridin-2-ylcarbamid

Synthese analog zu Beispiel 73.
Ausbeute: 75 mg (77 %) weißer Feststoff.

Smp: 140-143 °C. MS: m/z 405 (M⁺). IR (NaCl): 3410; 2926; 2853; 1658; 1634; 1553; 1241; 1145; 769. ¹H NMR (CDCl₃, 360 MHz) δ (ppm): 1.67-1.74 (m, 4H, CH₂-CH₂); 2.21 (s, 3H, CH₃); 2.26 (s, 3H, CH₃); 2.51 (br t, J= 7.0 Hz, 2H, CH₂N); 2.62-2.72 (m, 4H, pip); 2.93-2.94 (m, 4H, pip); 3.52-3.54 (m, 2H, CH₂NHCO); 6.84 (ddd, J=1.2 Hz, J=6.9 Hz, J=6.9 Hz, 1 H, H-6); 6.85-7.92 (m, 2H, H-arom); 7.05-7.07 (m, 2H, H-arom, H-3); 7.14 (ddd, J=0.8 Hz, J=6.8 Hz, J=8.7 Hz, 1 H, H-5); 7.29 (br t, J=4.9 Hz, 1 H, NHCO); 7.58 (d, J=9.1 Hz, 1 H, H-4); 8.36 (dd, J=0.8 Hz, J=6.8 Hz, 1 H, H-7).

### Beispiel 86:

### N-4-(4-(2,3-Dihydrobenzofuran-7-yl)piperazin-1-yl)butylpyrazolo[1,5-a]pyridin-2-ylcarbamid

Synthese analog zu Beispiel 73, wobei die Aminkomponente 4-(4-(2,3-Dihydrobenzofuran-7-yl)piperazin-1-yl)butylamin nach den Vorschriften für Amine des Typs C3 hergestellt wurde.
Ausbeute: 72 mg (71 %) farbloses Öl.
Smp.: 60-62 °C. MS: m/z 419 (M⁺). IR (NaCl): 3411; 2939; 2817; 1662; 1636; 1553; 1256; 1146; 1012; 753. ¹H NMR (CDCl₃, 360 MHz) δ (ppm): 1.66-1.73 (m, 4H, CH₂-CH₂); 2.54 (t, J= 6.8 Hz, 2H, CH₂N); 2.69-2.74 (m, 4H, pip); 3.17-3.22 (m, 6H, O-CH₂-C-H-2,pip); 3.49-3.55 (m, 2H, CH₂NHCO); 4.59 (t, J=8.9 Hz, 2H, O-CH₂-CH₂); 6.68-6.70 (m, 1 H, H-arom); 6.77-6.87 (m, 3H, H-arom, H-6); 7.04 (d, J=0.9 Hz, 1 H, H-3); 7.13 (ddd, J=1.0 Hz, J=6.8 Hz, J=8.9 Hz, 1 H, H-5); 7.29 (br t, J=5.0 Hz, 1 H, NHCO); 7.58 (d, J=8.9 Hz,1H, H-4); 8.36 (dd, J=0.9 Hz, J=7.0 Hz, 1H, H-7).

### Beispiel 87:

### N-4-(Chroman-8-yl)piperazin-1-yl)butylpyrazolo[1,5-a]pyridin-2-ylcarbamid

Synthese analog zu Beispiel 73, wobei die Aminkomponente 4-(4-(Chroman-8-yl)piperazin-1-yl)butylamin nach den Vorschriften für Amine des Typs C3 hergestellt wurde.
Ausbeute: 40 mg (38 %) farbloses Öl.
MS: m/z 434 (M⁺). IR (NaCl): 3397; 2926; 2853; 1634; 1556; 1259; 750. ¹H NMR (CDCl₃, 360 MHz) δ (ppm): 1.70-1.78 (m, 4H, CH₂-CH₂); 1.97-2.02 (m, 2H, O-CH₂-CH₂-CH₂); 2.60-2.65 (m, 2H, CH₂N); 2.79 (t, J= 6.4 Hz, 2H, O-CH₂-CH₂-CH₂); 2.80-2.87 (m, 4H, pip); 3.14-3.20 (m, 4H, pip); 3.5.1-3.54 (m, 2H, CH₂NHCO); 4.24-4.26 (m, 2H, O-CH₂-CH₂-CH₂); 6.72-6.78 (m, 3H, H-arom); 6.85 (ddd, J=6.8 Hz, J=6.8 Hz, J=1.1 Hz, 1 H, H-6); 7.05 (br s, 1 H, H-3); 7.14 (ddd, J=0.8 Hz, J=6.8 Hz, J=8.7 Hz, 1 H, H-5); 7.31 (br t, J=5.3 Hz, 1 H, NHCO); 7.58 (d, J=9.0 Hz, 1 H, H-4); 8.37 (dd, J=0.8 Hz, J=7.2 Hz, 1 H, H-7).

### Beispiel 88:

### N-4-(4-(2,4-Dimethoxyphenyl)piperazin-1-yl)butylpyrazolo[1,5-a]pyridin-2-ylcarbamid

Synthese analog zu Beispiel 73.
Ausbeute: 74 mg (71 %) farbloses Öl.
MS: m/z 437 (M⁺); IR (NaCl): 3412; 2937; 2817; 1662; 1635; 1552; 1509; 1257; 1208; 1034; 750. ¹H NMR (CDCl₃, 360 MHz) δ (ppm): 1.68-1.74 (m, 4H, CH₂-CH₂); 2.53 (t, J=4.4 Hz, 2H, CH₂N); 2.68-2.76 (m, 4H, pip); 3.04-3.11 (m, 4H, pip); 3.50-3.55 (m, 2H, CH₂NHCO); 3.77 (s, 3H, OCH₃); 3.33 (s, 3H, OCH₃); 6.40-6.43 (m, 1H, H-arom); 6.47-6.48 (m, 1H, H-arom); 6.82-6.87 (m, 2H, H-arom, H-6); 7.05 (d, J=0.7 Hz, 1 H, H-3); 7.13 (ddd, J=1.0 Hz, J=6.8 Hz, J=8.9 Hz, 1 H, H-5); 7.28 (br t, J=5.2 Hz, 1 H, NHCO); 7.58 (d, J=9.0 Hz, 1H, H-4); 8.36 (dd, J=0.9 Hz, J=7.0 Hz, 1H, H-7).

### Beispiel 89:

### N-5-(4-(2-Methoxyphenyl)piperazin-1-yl)pentyl-4,5,6,7-tetrahydropyrazolo[1,5-a]pyridin-2-ylcarbamid

Synthese analog zu Beispiel 39.
Ausbeute: 40 mg (78%).
MS: 426 m/z (M⁺); ¹H NMR (CDCl₃, 360 MHz) δ (ppm): 1.37-1.46 (m, 2H, CH₂-CH₂-CH₂); 1.52-1.67 (m, 4H, CH₂-CH₂-CH₂); 1.82-1.89 (m, 2H, H-5); 2.01-2.08 (m, 2H, H-6);2.41 (t, J=7.7 Hz, 2H, CH₂N); 2.61-2.68 (m, 4H, pip); 2.81 (t, J=6.4 Hz, 2H, H-4); 3.06-3.13 (m, 4H, pip); 3.38-3.45 (m, 2H, CH₂NHCO); 3.86 (s, 3H, CH₃O); 4.09-4.16 (m, 2H, H-7); 6.49 (s, 1H, H-3), 6.81-7.01 (m, 5H, H-arom, NHCO).

### Beispiel 90:

### N-4-(4-(2-Methoxyphenyl)piperazin-1-yl)butyl-6-chlorimidazo[1,2-a]pyridin-2-ylcarbamid

Von *Ambinter, Paris (F)* erworbene 0,024 g 6-Chlorimidazo[1,2-*a*]pyridin-2-carbonsäure (0,12 mmol) werden in 4 ml trockenem Methylenchlorid gelöst und 0,07 ml (0,42 mmol) trockenes DIPEA zugegeben. Anschließend werden 0,042 g (0,13 mmol) in 0.5 ml trockenem DMF gelöstes TBTU bei 0° C langsam zugetropft und 30 Minuten bei Raumtemperatur gerührt. Die Reaktionslösung wird erneut auf 0 °C gekühlt und eine Lösung von 0,034 g (0,13 mmol) 4-(4-(2-Methoxyphenyl)piperazin-1-yl)butylamin in 4 ml trockenem Methylenchlorid zugetropft. Nach 1 Stunde rühren wird der Reaktionsansatz mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt.
Ausbeute: 40 mg (76 %) weißer Feststoff.
Smp.: 116-119 °C; MS: m/z 441 (M⁺); IR (NaCl): 3404; 2939; 2819; 1658; 1567; 1499; 1241; 1027; 751. ¹H NMR (CDCl₃, 360 MHz) ö (ppm): 1.61-1.73 (m, 4H,CH₂-CH₂); 2.47 (t, J=7.0 Hz, 2H, CH₂N); 2.63-2.71 (m, 4H, pip); 3.07-3.15 (m, 4H, pip); 3.48-3.53 (m, 2H, CH₂NHCO); 3.86 (s, 3H, OCH₃); 6.84-7.01 (m, 4H, H-arom); 7.20 (dd, J=1.9 Hz, J=9.7 Hz, 1H, H-7); 7.46 (br t, J=5.9 Hz, 1H, NHCO); 7.49 (ddd, J=0.7 Hz, J=0.7 Hz, J=9.7 Hz, 1 H, H-8); 8.09 (d, j=0.7 Hz, 1H, H-3); 8.20 (dd, J=0.9 Hz, J=2.0 Hz, 1H, H-5). ¹³C NMR (CDCl₃, 90 MHz) ö (ppm): 162.2; 152.3; 142.8; 141.4; 141.2; 127.5; 124.2; 122.9; 121.7; 121.0; 118.5; 114.3; 111.3; 58.2; 55.4; 53.5; 50.6; 39.1; 27.7; 24.3.

### Beispiel 91:

### N-4-(4-(2,3-Dichlorphenyl)piperazin-1-yl)butyl-6-chlotimidazo[1,2-a]pyridin-2yl-carbamid

Synthese analog zu Beispiel 90.
Ausbeute: 77 mg (67 %) weißer Feststoff.
Smp.: 135-135 °C; MS: m/z 480 (M⁺); 482 (M⁺+2); IR (NaCl): 3401; 2930; 2820; 1655; 1567; 1449; 1241; 732. ¹H NMR (CDCl₃, 360 MHz) δ (ppm): 1.68-1.75 (m, 4H,CH₂-CH₂); 2.54 (t, J=7.2 Hz, 2H, CH₂N); 2.67-2.76 (m, 4H, pip); 3.09-3.15 (m, 4H, pip); 3.52-3.56 (m, 2H, CH₂NHCO); 6.97 (dd, J=1.9 Hz, J=7.2 Hz, 1H, H-arom); 7.14-7.18 (m, 2H, H-arom); 7.23 (dd, J=1.9 Hz, J=9.4 Hz, 1 H, H-7); 7.50 (br t, J=5.7 Hz, 1H, NHCO); 7.51 (d, J=9.8 Hz, 1 H, H-8); 8.13 (br s, 1H, H-3); 8.22 (d, J=1.1 Hz, 1H, H-5).

### Beispiel 92:

### N-4-(4-(2-Methoxyphenyl)piperazin-1-yl)butyl-6-chlor-2-methylimidazo[1,2-a]pyridin-3-ylcarbamid

Von *Butt Park Ltd., Camelford (UK)* erworbene 0,025 g 6-Chlor-2-methylimidazo[1,2-*a*]pyridin-3-carbonsäure (0,12 mmol) werden in 4 ml trockenem Methylenchlorid gelöst und 0,07 ml (0,42 mmol) trockenes DIPEA zugegeben. Anschließend werden 0,042 g (0,13 mmol) in 0.5 ml trockenem DMF gelöstes TBTU bei 0° C langsam zugetropft und 15 Minuten bei Raumtemperatur gerührt. Die Reaktionslösung wird erneut auf 0 °C gekühlt und eine Lösung von 0,034 g (0,13 mmol) 4-(4-(2-Methoxyphenyl)piperazin-1-yl)butylamin in 4 ml trockenem Methylenchlorid zugetropft. Nach 1 Stunde wird der Reaktionsansatz mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt.
Ausbeute: 45 mg (82%).
Smp.: 118-120 °C; MS: m/z 455 (M⁺); IR (NaCl): 2937; 2818; 1635; 1594; 1498; 1241; 1028; 751. ¹H NMR (CDCl₃, 360 MHz) ö (ppm): 1.66-1.77 (m, 4H,CH₂-CH₂); 2.50 (t, J=6.9 Hz, 2H, CH₂N); 2.65-2.73 (m, 4H, pip); 2.70 (s, 3H, CH₃); 3.04-3.11 (m, 4H, pip); 3.52-3.56 (m, 2H, CH₂NHCO); 3.85 (s, 3H, OCH₃); 6.15 (br t, J=4.9 Hz, 1 H, NHCO); 6.84-6.86 (m, 1H, H-arom); 6.88-6.91 (m, 2H, H-arom); 6.95-7.01 (m, 1H, H-arom); 7.28 (dd, J=9.4 Hz, J=1.9 Hz, 1 H, H-7); 7.49 (d, J=9.4 Hz, 1H, H-8); 9.45 (d, J=1.1 Hz, 1H, H-5). ¹³C MMR (CDCl₃, 90 MHz) δ (ppm): 161.3; 152.3; 145.5; 144.2; 141.2; 128.1; 126.0; 123.0; 121.4; 121.0; 118.2; 116.7; 116.2; 111.2; 58.1; 55.3; 53.4; 50.4; 39.5; 27.8; 24.3; 16.6.

### Beispiel 93:

### N-4-(4-(2-Methoxyphenyl)piperazin-1yl)butylimidazo[1,2-a]pyridin-6-ylcarbamid

0,020 g Imidazo[1,2-*a*]pyridin-6-carbonsäure (0,12 mmol) werden in 4 ml trockenem Methylenchlorid gelöst und 0,07 ml (0,42 mmol) trockenes DIPEA zugegeben. Anschließend werden 0,042 g (0,13 mmol) in 0.5 ml trockenem DMF gelöstes TBTU bei 0°C langsam zugetropft und 30 Minuten bei Raumtemperatur gerührt. Die Reaktionslösung wird erneut auf 0 °C gekühlt und eine Lösung von 0,034 g (0,13 mmol) 4-(4-(2-Methoxyphenyl)piperazin-1-yl)butylamin in 4 ml trockenem Methylenchlorid zugetropft. Nach 1 Stunde wird der Reaktionsansatz mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt.
Ausbeute: 5 mg (10 %) gelbes Öl.
MS: m/z 408 (M⁺); IR (NaCl): 2933; 2826; 1656; 1553; 1500; 1241; 1027; 750. ¹H NMR (CDCl₃, 360 MHz) δ (ppm): 1.68-1.76 (m, 4H,CH₂-CH₂); 2.52 (t, J=6.6 Hz, 2H, CH₂N); 2.67-2.74 (m, 4H, pip); 3.05-3.15 (m, 4H, pip); 3.49-3.52 (m, 2H, CH₂NHCO); 3.85 (s, 3H, OCH₃); 6.84-6.93 (m, 3H, H-arom); 6.97-7.02 (m, 1H, H-arom); 7.04 (br t, J=6.4 Hz, 1 H, NHCO); 7.44 (dd, J=1.5 Hz, J=9.4 Hz, 1H, H-3); 7.61 (d, J=9.4 Hz, 1H, H-2); 7.65 (br s, 1H, H-7); 7.69 (d, J=1.1 Hz, 1H, H-8); 8.82 (br s, 1 H, H-5).

### Beispiel 94:

### N-4-(4-(2,3-Dichlorphenyl)piperazin-1-yl)butyl[1,2,4]triazolo[1,5-a]pyridin-2-ylcarbamid

0,01 g [1,2,4]Triazolo[1,5-*a*]pyridin-2-carbonsäure (0,06 mmol) werden in 3 ml trockenem Methylenchlorid gelöst und 0,035 ml (0,12 mmol) trockenes DIPEA zugegeben. Anschließend werden 0,022 g (0,07 mmol) in 0.5 ml trockenem DMF gelöstes TBTU bei 0°C langsam zugetropft und 30 Minuten bei Raumtemperatur gerührt. Die Reaktionslösung wird erneut auf 0 °C gekühlt und eine Lösung von 0,03 g (0,1 mmol) 4-(4-(2,3-Dichlorphenyl)piperazin-1-yl)butylamin in 4 ml trockenem Methylenchlorid bei 0 °C zugetropft. Nach 1 Stunde wird der Reaktionsansatz mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:97-3) gereinigt.
Ausbeute: 20 mg (74%) weißer Feststoff.
Smp.: 134-135 °C; MS: m/z 446 ((M+2)⁺), 448 ((M+4)⁺). IR (NaCl): 2941; 2820; 1676; 1637; 1241; 734. ¹H NMR (CDCl₃, 360 MHz) δ (ppm): 1.67-1.77 (m, 4H,CH₂-CH₂); 2.55 (t, J=7.0 Hz, 2H, CH₂N); 2.69-2.75 (m, 4H, pip); 3.10-3.14 (m, 4H, pip); 3.56-3.61 (m, 2H, CH₂NHCO); 6.96-6.99 (m, 1 H, H-6); 7.10-7.17 (m, 3H, H-arom); 7.60-7-66 (m, 2H, H-7, NHCO); 7.77-7.80 (m, 1H, H-8); 8.66-8.69 (m, 1 H, H-5).

### Beispiel 95:

### N-4-(4-(2,3-Dichlorphenyl)piperazin-1-yl)pyrazolo[1,5-b]pyridazin-2-ylcarbamid

0,019 g Pyrazolo[1,5-*b*]pyridazin-2-carbonsäure (0,12 mmol) werden in 5 ml trockenem Methylenchlorid gelöst und 0,07 ml (0,42 mmol) trockenes DIPEA zugegeben. Anschließend werden 0,042 g (0,13 mmol) in 0.5 ml trockenem DMF gelöstes TBTU bei 0°C langsam zugetropft und 30 Minuten bei Raumtemperatur gerührt. Die Reaktionslösung wird erneut auf 0 °C gekühlt und eine Lösung von 0,085 g (0,28 mmol) 4-(4-(2,3-Dichlorphenyl)piperazin-1-yl)butylamin in 4 ml trockenem Methylenchlorid bei 0 °C zugetropft. Nach 1 Stunde wird der Reaktionsansatz mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:97-3) gereinigt.
Ausbeute: 39 mg (72%) weiβer Feststoff.
Smp.: 122-124 °C; MS: m/z 446 ((M+2)⁺), 448 ((M+4)⁺). IR (NaCl): 2934; 2821; 1656; 1242; 725. ¹H NMR (CDCl₃, 360 MHz) δ (ppm): 1.69-1.74 (m, 4H,CH₂-CH₂); 2.59 (t, J=6.9 Hz, 2H, CH₂N); 2.74-2.80 (m, 4H, pip); 3.11-3.16 (m, 4H, pip); 3.51-3.57 (m, 2H, CH₂NHCO); 6.98 (dd, J=2.5 Hz, J=7.0 Hz, 1H, H-arom); 7.05 (dd, J=4.5 Hz, J=9.1 Hz, 1H, H-5); 7.13-7.16 (m, 2H, H-arom); 7.19 (s, 1H, H-3); 7.47 (br t, J=5.4 Hz, 1 H, NHCO); 8.03 (dd, J=2.0 Hz, J=9.1 Hz, 1 H, H-4); 8.36 (dd, J=1.9 Hz, J=4.4 Hz, 1 H, H-6).

### Beispiel 96:

### N-4-(4-(2-Methoxyphenyl)piperazin-1-yl)butyl-6 chlorimidazo[1,2-b]pyridazin-2-ylcarbamid

Von *Maybridge, Tintangel (UK)* erworbene 0,047 g 6-Chlorimidazo[1,2-b]pyridazin-2-carbonsäure (0,24 mmol) werden in 6 ml trockenem Methylenchlorid gelöst und 0,14 ml (0,84 mmol) trockenes DIPEA zugegeben. Anschließend werden 0,084 g (0,26 mmol) in 0.5 ml trockenem DMF gelöstes TBTU bei 0°C langsam zugetrepft und 15 Minuten bei Raumtemperatur gerührt. Die Reaktionslösung wird auf 0 °C gekühlt und eine Lösung von 0,071 g (0,27 mmol) 4-(4-(2-Methoxyphenyl)piperazin-1-yl)butylamin in 4 ml trockenem Methylenchlorid zugetropft. Nach 1 Stunde wird der Reaktionsansatz mit Mthylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt
Ausbeute: 82 mg (77%).
Smp.: 120-123 °C; MS: m/z 442 (M⁺); IR (NaCl): 3407; 2931; 2851; 2817; 1655; 1499; 1241; 1028; 751. ¹H NMR (CDCl₃, 360 MHz) δ (ppm): 1.64-1.74 (m, 4H,CH₂-CH₂); 2.50 (t, J=7.0 Hz, 2H, CH₂N); 2.65-2.73 (m, 4H, pip); 3.09-3.17 (m, 4H, pip); 3.49-3.55 (m, 2H, CH₂NHCO); 3.86 (s, 3H, OCH₃); 6.84-7.02 (m, 4H, H-arom); 7.11 (d, J=9.5 Hz, 1 H, H-8); 7.48 (br t, J=5.3 Hz, 1 H, NHCO); 7.84 (d, J=9.5 Hz, 1 H, H-7); 8.43 (d, J=0.7 Hz, 1H, H-3).

### Beispiel 97:

### N-4-(4-(2-Methoxyphenyl)piperazin-1-yl)butyl-6-chlor-2-phenylimidazo[1,2-b]pyridazin-3-ylcarbamid

Von *Butt Park Ltd., Camelford (UK)* erworbene 0,033 g 6-Chlor-2-phenylimidazo[1,2-*b*]pyridazin-3-carbonsäure (0,12 mmol) werden in 4 ml trockenem Methylenchlorid gelöst und 0,07 ml (0,42 mmol) trockenes DIPEA zugegeben. Anschließend werden 0,042 g (0,13 mmol) in 0.5 ml trockenem DMF gelöstes TBTU bei 0° C langsam zugetropft und 30 Minuten bei Raumtemperatur gerührt. Die Reaktionslösung wird erneut auf 0°C gekühlt und eine Lösung von 0,034 g (0,13 mmol) 4-(4-(2-Methoxyphenyl)piperazin-1-yl)butylamin in 4 ml trockenem Methylenchlorid zugetropft. Nach 1 Stunde wird der Reaktionsansatz mit Methylenchlorid verdünnt und mit gesättigter NaHCO₃-Lösung sowie Wasser gewaschen. Nach Trocknung der organischen Phase mit MgSO₄ wird das Lösungsmittel abgedampft und durch Flashchromatographie (SiO₂; CH₂Cl₂-CH₃OH:98-2) gereinigt
Ausbeute: 41 mg (66 %) weißer Feststoff.
Smp.: 58-62 °C; MS: m/z 518 (M⁺), 520 (M+2)⁺; IR (NaCl): 3348; 2929; 2816; 1656; 1554; 1499; 1241; 1027; 751. ¹H NMR (CDCl₃, 360 MHz) δ (ppm): 1.66-1.74 (m, 4H,CH₂-CH₂); 2.41-2.50 (m, 2H, CH₂N); 2.58-2.66 (m, 4H, pip); 2.98-3.09 (m, 4H, pip); 3.53-3.60 (m, 2H, CH₂NHCO); 3.85 (s, 3H, OCH₃); 6.82-6.93 (m, 4H, H-arom); 6.96-7.01 (m, 1H, H-arom); 7.21 (d, J=9.4 Hz, 1 H, H-8); 7.35-7.48 (m, 3H, H-arom); 7.89-7.95 (m, 2H, H-arom); 8.01 (d, J=9.4 Hz, 1H, H-7); 8.51 (br t, J=4.8 Hz, 1 H, NHCO); 8.58 (br t, J=5.3 Hz, 1 H, NH).

### ERSTELLUNG VON SUBSTANZBIBLIOTHEKEN DURCH FESTPHASENUNTERSTÜTZTE SYNTHESE:

Die Erstellung einer Substanzbibliothek von Dopaminrezeptor-affinen Liganden durch festphasenunterstützte Synthese basiert auf Methoden, die in unserer Arbeitsgruppe entwickelt wurden und bereits beschrieben sind (Löber, S., et al. *Org. Lett.* **2003,** *5*, 1753-1755). Grundlage dieser Arbeit ist die Entwicklung neuartigen BAL-Linker, die zur Synthese des FIMT-Harzes (Formyl-Indolyl-Methyl-Triazol) führte. Mit Hilfe dieses funktionalisierten Polystyrols konnten zwei Bibliotheken (Bibliothek 1 und Bibliothek 2) potenter Liganden synthetisiert werden:

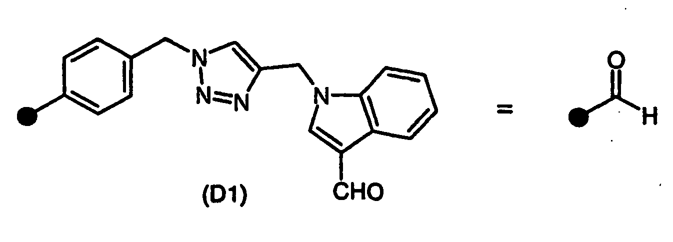

Nachfolgend sind die einzelnen Reaktionsschritte der festphasenunterstützten Synthese von N-(ω-(4-(2,3-Dichlorphenyl)piperazin-1-yl)alkylheteroarylcarbamiden (Bibliothek 1 mit Beispiel 6-9, 18-21, 24-27, 31-38, 41-44) beschrieben.

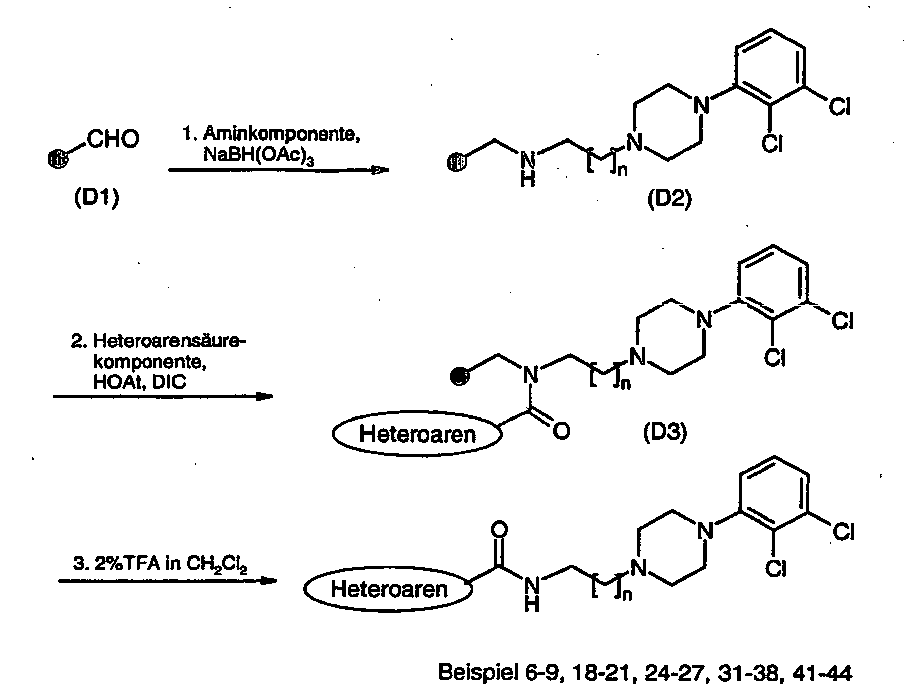

Analog dieser Synthesesequenz wurden in Bibliothek 2 die Verbindungen Beispiel 13 sowie Beispiel 50-68 hergestellt.

### Herstellung der polymergebundenen Verbindung D2 (Schritt 1):

### Polymergebundenes ω-(4-(2,3-Dichlorphenyl)piperazin-1-yl)alkylamin

0,100 g (1.043 mmol/g) FIMT-Harz (D1), 4 eq NaBH(AcO)₃ und eine Lösung von 4 eq der Aminkomponent in 5 ml trockenem Methylenchlorid werden 24 Stunden bei Raumtemperatur in einem Teflon-Reaktionsgefäß (PLS Organic Synthesiser; Rotation: 320/min) geschüttelt. Danach wird das Harz abfiltriert und je 3x folgenden Waschschritten unterworfen: Methanol, Methanol-0,1 N HCl (9-1), Triethylamin (2%-ig in Methylenchlorid) und Methylenchlorid. Das Harz wird nach dem letzten Waschvorgang im Zuge der Filtration getrocknet.

### Herstellung der polymergebundenen Verbindung D3 (Schritt 2):

### Polymergebundenes N-(ω-(4-(2,3-Dichlorphenyl)piperazin-1-y)lalky)heteroarencarbamid

Das erhaltene Harz wird in 5 ml Methylenchlorid-DMF:9-1 suspendiert und mit 4 Eq Säure, 4 eq HOAt sowie 4.5 eq N,N'-Diisopropylcarbodiimid versetzt. Der Reaktionsansatz wird 48 Stunden bei Raumtemperatur geschüttelt, danach wird abfiltriert und je 3x gewaschen mit DMF, Methanol sowie Dichlormethan und nach dem letzten Waschschritt in der Fritte getrocknet.

### Abspaltung der polymergebundenen Zielverbindungen Beispiel 6-9, 18-21, 24-27, 31-38, 41-44 sowie 13 und 50-68 (Schritt 3):

### N-(ω-(4-(2,3-Dichlorphenyl)piperazin-1-y)lalky)heteroarencarbamid

Das erhaltene Harz wird mit 5 ml einer Lösung von 2%-iger Trifluoressigsäuren in Methylenchlorid versetzt und 2 Stunden bei Raumtemperatur geschüttelt. Das Harz wird filtriert, je 2x mit 3 ml Methanol und anschließend Methylenchlorid gewaschen. Filtrat und Waschflüssigkeiten werden vereint, die Lösungsmittel evapuriert und dabei das Produkt erhalten.

### Charakterisierung der festphasensynthetisierten Substanzen:

Die analytische Überprüfung der durch festphasenunterstützte Synthese erhaltenen Produkte erfolgte durch LC/MS-Analytik an einem Chromatographiesystem der Firma Agilent (Binary Gradient System in Kombination mit der ChemStation- Software) und der massenspektrometrischen Bestimmung mit Hilfe eines Bruker Esquire 2000 ion-trap-Massenspektrometers (Ionisierung in einer APCI-Quelle).
Die chromatographische Trennung wurde durchgeführt auf einer Zorbax SB-C18-Säule (4.6 mm ID x 250 mm, 5 µm) mit einem Fließmittelsystem MeOH/0.1 N aq.HCO₂H im Gradienten von 50/50 auf 90/10 bei einer Flussrate von 0.5 ml/min. Die Detektion erfolgte mittels Agilent UV/VIS-Detektor bei 254 nm.

### VERBINDUNGEN DER SUBSTANZBIBLIOTHEKEN 1 und 2:

Alle Verbindungen der Substanzbibliotheken 1 und 2 wurden wie oben besprochen analytisch charakterisiert und in einem biologischen Screeningverfahren untersucht. Die vielversprechendsten Testverbindungen wurden anschließend einer detaillierten spektroskopischen Untersuchung unterworfen und durch weitergehende Rezeptorbindungsversuche charakterisiert.

### VERBINDUNGEN VON BIBLIOTHEK 1:

### Beispiel 6:

### N-2-(4-(2,3-Dichlorphenyl)piperazin-1-yl)ethylpyrazolo[1,5-a]pyridin-2-ylcarbamid

Molekulargewicht (MG) (berechnet): 418,33; Masse (MS) (gefunden): 418,7 (M+1); Retentionszeit (T_{R} in [min]) :11,2.

### Beispiel 7:

### N-3-(4-(2,3-Dichlorphenyl)piperazin-1-yl)propylpyrazolo[1,5-a]pyridin-2-ylcarbamid

MG (ber): 432,36; MS (gef): 432,4; T_{R}: 12,0 min.

### Beispiel 8:

### N-4-(4-(2,3-Dichlorphenyl)piperazin-1-yl)butylpyrazolo[1,5-a]pyridin-2-ylcarbamid Trifluoressigsäuresalz

MG (ber): 446,68; MS (gef): 446,5; T_{R}: 6,1 min.
IR (NaCl): 3410, 3318, 2954, 2849, 1778, 1670, 1635, 1555, 1514, 1452, 1198. ¹H NMR (CDCl₃, 360 MHz) δ (ppm): 1.74-1.80 (m, 2H, CH₂-CH₂-Npip); 1.89-1.95 (m, 2H, CONH-CH₂CH₂); 3.06-3.12 (m, 2H, CH₂Npip); 3.20-3.41 (m, 6H, pip); 3.52-3.58 (m, 2H, CH₂NHCO); 3.69-3.72 (m, 2H, pip); 5.30 (br s, 1 H, HNpip); 6.86-6.89 (m, 1 H, Phenyl); 6-97-7.00 (m, 1 H, H-6), 7.05 (s, 1 H, H-3); 7.14-7.27 (m, 3H, Phenyl, H-5), 7.42 (s, 1 H, HNCO), 7.60 (d, J=8.2 Hz, 1 H, H-4), 8.49 (d, J=6.7 Hz, 1 H, H-7).

### Beispiel 9:

### N-5-(4-(2,3-Dichlorphenyl)piperazin-1-yl)pentylpyrazolo[1,5-a]pyridin-2-ylcarbamid Trifluoressigsäuresalz

MG (ber): 460,41; MS (gef): 460,0; T_{R}: 9,1 min.

IR (NaCl): 3407, 3326, 2948, 1773, 1671, 1558, 1514, 1451, 1199. ¹H NMR (CDCl₃, 360 MHz) δ (ppm): 1.46-1.54 (m, 2H, (CH₂CH₂CH₂); 1.67-1.75 (m, 2H, CH₂-CH₂-Npip); 1.84-1.93 (m, 2H, CH₂Npip); 3.04-3.13 (m, 4H, CONH-CH₂CH₂, pip); 3.28-3.42 (m, 4H, pip); 3.49-3.54 (m, 2H, CH₂NHCO); 3.70-3.74 (m, 2H, pip); 6.24 (br s, 1 H, HNpip); 6.86-6.89 (m, 1 H, Phenyl); 6-97-7.00 (m, 1 H, H-6), 7.06 (s, 1 H, H-3); 7.13-7.26 (m, 3H, Phenyl, H-5), 7.30 (s, 1 H, HNCO), 7.60 (d, J=7.8 Hz, 1 H, H-4), 8.39 (d, J=6.4 Hz, 1 H, H-7).

### Beispiel 18:

### N-2-(4-(2,3-Dichlorphenyl)piperain-1-yl)ethylpyrazolo(1,5-a]pyridin-3-ylcarbamid

MG (ber): 418,33; MS (gef): 418,7; T_{R}: 11,0 min.

### Beispiel 19:

### N-3-(4-(2,3-Dichlorphenyl)piperazin-1-yl)propylpyrazolo[1,5-a]pyridin-3-ylcarbamid

MG (ber): 432,36; MS (gef): 432,2; T_{R}: 8,4 min.

### Beispiel 20:

### N-4-(4-(2,3-Dichlorphenyl)piperazin-1-yl)butylpyrazolo[1,5-a]pyridin-3-ylcarbamid

MG (ber): 446,68; MS (gef): 446,1; T_{R}: 6,5 min.

### Beispiel 21:

### N-5-(4-(2,3-Dichlorphenyl)piperazin-1-yl)pentylpyrazolo[1,5-a]pyridin-3-ylcarbamid

MG (ber): 460,41; MS (gef): 460,1 ; T_{R}: 14,3 min.

### Beispiel 24:

### N-2-(4-(2,3-Dichlorphenyl)piperazin-1-yl)ethylpyrazolo[1,5-a]pyridin-5-ylcarbamid

MG (ber): 418,33; MS (gef): 418,5; T_{R}: 11,0 min.

### Beispiel 25:

### N-3-(4-(2,3-Dichlorphenyl)piperazin-1-yl)propylpyrazolo[1,5-a]pyridin-5-ylcarbamid

MG (ber): 432,36; MS (gef): 431,9; T_{R}: 9,9 min.

### Beispiel 26:

### N-4-(4-(2,3-Dichlorphenyl)piperazin-1-yl)butylpyrazolo[1,5-a]pyridin-5-ylcarbamid Trifluoressigsäuresalz

MG (ber): 446,68; MS (gef): 446,0; T_{R}: 9,8 min.

IR (NaCl): 3325, 2961, 2851, 1779, 1671, 1631, 1548, 1452, 1201. ¹H NMR (CDCl₃, 360 MHz) δ (ppm): 1.74-1.85 (m, 2H, CH₂CH₂Npip); 1.95-2.06 (m, 2H, CONHCH₂CH₂); 3.03-3.12 (m, 2H, CH₂Npip); 3.14-3.23 (m, 2H, pip); 3.28-3.44 (m, 4H, pip); 3.51-3.70 (m, 4H, CH₂NHCO, pip); 6.70 (br s, 1 H, HNpip); 6.97-6.99 (m, 1H, H-3); 6.99-7.01 (m, 1 H, Phenyl); 7.17-7.23 (m, 2H, Phenyl); 7.24-7.26 (m, 1 H, H-6), 7.65 (br s, 1 H, HNCO); 8.01 (s, 1H, H-4), 8.14-8.27 (m, 1H, H-2), 8.45-8.57 (m, 1H, Hz, H-7).

### Beispiel 27:

### N-5-(4-(2,3-Dichlorphenyl)piperazin-1-yl)pentylpyrazolo[1,5-a]pyridin-5ylcarbamid Trifluoressigsäuresalz

MG (bec): 460,41; MS (gef): 460,0; T_{R}: 11,2.min.
IR (NaCl): 3326,2948.2861.1778,1672,1631,1548,1452,1200. ¹H NMR(CDCl₃, 360 MHz) δ (ppm): 1.48-1.59 (m, 2H, CH₂CH₂CH₂); 1.66-1.77 (m, 2H, CH₂CH₂Npip); 1.85-1.97 (m, 2H, CONHCH₂CH₂); 3.02-3.19 (m, 4H, CH₂Npip, pip); 3.26-3.35 (m, 2H, pip); 3.36-3.46 (m, 3H, pip); 3.47-3.58 (m, 1 H, pip); 3.64-3.75 (m, 2H, CH₂NHCO); 6.73 (br s, 1H, HNpip); 6.96-6.98 (m, 1 H, H-3); 6.98-7.00 (m, 1 H, Phenyl); 7.17-7.24 (m, 4H, Phenyl, H-6, HNCO); 8.00-8.80 (m, 3H, H-4, H-2, H-7).

### Beispiel 31:

### N-2-(4-(2,3-Dichlorphenyl)piperazin-1-yl)ethylpyrazolo[1,5-a]pyridin-6-ylcarbamid

MG (ber): 418,33; MS (gef): 417,9; T_{R}: 7,3 min.

### Beispiel 32:

### N-3-(4-(2,3-Dichlorphenyl)piperazin-1-yl)propylpyrazolo[1,5-a]pyridin-6-ylcarbamid

MG (ber): 436,39; MS (gef): 431,9; T_{R}: 8,6 min.

### Beispiel 33:

### N-4-(4-(2,3-Dichlorphenyl)piperazin-1-yl)butylpyrazolo[1,5-a]pyridin-6-ylcarbamid

MG (ber): 446,68; MS (gef): 446,3; T_{R}: 7,4 min.

### Beispiel 34:

### N-5-(4-(2,3-Dichlorphenyl)piperazin-1-yl)pentylpyrazolo[1,5-a]pyridin-6-ylcarbamid

MG (ber): 460,41; MS (gef): 460,0; T_{R}: 6,0 min.

### Beispiel 35:

### N-2-(4-(2,3-Dichlorphenyl)piperazin-1-yl)ethyl-4,5,6,7-tetrahydropyrazolo[1,5-a]pyridin-2-ylcarbamid

MG (ber): 422,36; MS (gef): 422,4; T_{R}: 7,2 min.

### Beispiel 36:

### N-3-(4-(2,3-Dichlorphenyl)piperazin-1-yl)propyl-4,5,6,7-tetrahydropyrazolo[1,5-a]pyridin-2-ylcarbamid

MG (ber): 436,39; MS (gef): 436,4; T_{R}: 6,3 min.

### Beispiel 37:

### N-4-(4-(2,3-Dichlorphenyl)piperazin-1-yl)butyl-4,5,6,7-tetrahydropyrazolo[1,5-a]pyridin-2-ylcarbamid Trifluoressigsäuresalz

MG (ber): 450,42; MS (gef): 450,5; T_{R}: 6,2 min.

IR (NaCl): 3407, 3328, 2956, 2867, 1776, 1669, 1631,1578, 1534,1451, 1197, ¹H NMR (CDCl₃, 360 MHz) δ (ppm): 1.69-1.73 (m, 2H, CH₂CH₂Npip); 1.84-1.89 (m, 4H, CONH-CH₂CH₂, H-5); 2.02-2.08 (m, 2H, H-6); 2.80-2.84 (m, 2H, H-4); 3.07-3.12 (m, 2H, CH₂Npip); 3.19-3.31 (m, 4H, pip); 3.38-3.48 (m, 4H, pip); 3.68-3.72 (m, 2H, CH₂NHCO); 4.11-4.15 (m, 2H, H-7); 6.50 (s, 1 H, H-3); 6.96-6.98 (m, 1 H, Phenyl); 7.16-7.26 (m, 3H, Phenyl, HNCO), 8.27 (br s, 1 H, HNpip).

### Beispiel 38:

### N-5-(4-(2,3-Dichlorphenyl)piperazin-yl)pentyl-4,5,6,7 tetrahydropyrazolo[1,5-a]pyridin-2-ylcarbamid Trifluoressigsäuresalz

MG (ber): 464,44; MS (gef): 464,1; T_{R}: 6,4 min.

IR (NaCl): 3412, 3327, 2951, 2865, 1779, 1669, 1631, 1578, 1533, 1452, 1198. ¹H NMR (CDCl₃, 360 MHz) δ (ppm): 1.43-1.49 (m, 2H, CH₂CH₂CH₂); 1.63-1.67 (m, 2H, CH₂CH₂Npip); 1.81-1.90 (m, 4H, CONHCH₂CH₂, H-5); 2.03-2.05 (m, 2H, H-6); 2.80-2.83 (m, 2H, H-4); 3.03-3.12 (m, 4H, pip); 3.30 (t, J=11.7 Hz, 2H,CH₂Npip); 3.38-3.45 (m, 4H, pip); 3.69-3.73 (m, 2H, CH₂NHCO); 4.11-4.14 (m, 2H, H-7); 6.50 (s, 1H, H-3); 6.98-7.00 (m, 1 H, Phenyl); 7.08 (br s, 1 H, HNCO); 7.17-7.27 (m, 2H, Phenyl), 7.83 (br s, 1 H, HNpip).

### Beispiel 41:

### N-2-(4-(2,3-Dichlorphenyl)piperazin-1-yl)ethyl-4,5,6,7-tetrahydropyrazolo[1,5-a]pyridin-3-ylcarbamid

MG (ber): 422,36; MS (gef): 421,9; T_{R}:6,6 min.

### Beispiel 42:

### N-3-(4-(2,3-Dichlorphenyl)piperazin-1-yl)propyl-4,5,6,7-tetrahydropyrazolo[1,5-a]pyridin-3-ylcarbamid

MG (ber): 450,42; MS (gef): 436,4; T_{R}: 5,9 min.

### Beispiel 43:

### N-4-(4-(2,3-Dichlorphenyl)piperizin-1-yl)butyl-4,5,6,7-tetrahydropyrazolo[1,5-a]pyridin-3-ylcarbamid

MG (ber): 450,42; MS (gef): 450,5; T_{R}: 6,2 min.

### Beispiel 44:

### N-5-(4-(2,3-Dichlorphenyl)piperazin-1-yl)pentyl-4,5,6,7-tetrahydropyrazolo[1,5-a]pyridin-3-ylcarbamid

MG (ber): 464,44; MS (gef): 464,1; T_{R}: 10,8 min.

### VERBINDUNGEN VON BIBLIOTHEK 2:

### Beispiel 13:

### N-4-(4-(2-Methoxyphenyl)piperazin-1-yl)butyl-6-brompyrazolo[1,5-a]pyridin-2-ylcarbamid

MG (ber): 486,42; MS (gef): 487,7; T_{R}: 19,3 min.

Die spektroskopischen Daten von Beispiel 13 sind außerdem auf Seite 63 beschrieben.

### Beispiel 50:

### N-4-(4-(2,3-Dichlorphenyl)piperazin-1-yl)butyl-5-methoxypyrazolo[1,5-a]pyridin-2-ylcarbamid

MG (ber): 476,41; MS (gef): 476,6; T_{R}: 17,2 min.

### Beispiel 51:

### N-4-(4-(2-Methoxyphenyl)piperazin-1-yl)butyl-5-methoxypyrazolo[1,5-a]pyridin-2-ylcarbamid

MG (ber): 437,55; MS (gef): 438,2; T_{R}: 18,5 min.

### Beispiel 52:

### N-4-(4-(2,3-Dichlorphenyl)piperazin-1-yl)butyl-5-trifluormethylpyrazolo[1,5-a]pyridin-2-ylcarbamid

MG (ber): 514,38; MS (gef): 514,8; T_{R}: 18,2 min.

### Beispiel 53:

### N-4-(4-(2-Methoxyphenyl)piperazin-1 yl)butyl-5-trifluormethylpyrazolo[1,5-a]pyridin 2-ylcarbamid

MG (ber): 475,52; MS (gef): 476,2; T_{R}: 19,7 min.

### Beispiel 54:

### N-4-(4-(2,3-Dichlorphenyl)piperazin-1-yl)butyl-6-brompyrazolo[1,5-a]pyrldin-2-ylcarbamid

MG (ber): 525,28; MS (gef): 526,0; T_{R}: 17,9 min.

### Beispiel 55:

### N-4-(4-(2,3-Dichlorphenyl)piperazin-1 yl)butyl-6-chlorpyrazolo[1,5-a]pyridin-2-ylcarbamid

MG (ber): 480,82; MS (gef): 481,8; T_{R}: 17,9 min.

### Beispiel 56:

### N-4-(4-(2-Methoxyphenyl)piperazin-1 yl)butyl 6-chlorpyrazolo[1,5-a]pyridin-2-ylcarbamid

MG (ber): 441,96; MS (gef): 442,4; T_{R}: 19,1 min.

### Beispiel 57:

### N-4-(4-(2,3-Dichlorphenyl)piperazin-1-yl)butyl-6-fluorpyrazolo[1,5-a]pyridin-2-ylcarbamid

MG (ber): 464,37; MS (gef): 464,5; T_{R}: 17,4 min.

### Beispiel 58:

### N-4-(4-(2-Methoxyphenyl)piperazin-1-yl)butyl-6-fluorpyrazolo[1,5-a]pyridin-2-ylcarbamid

MG (ber): 425,51; MS (gef): 426,2; T_{R}: 18,4 min.

### Beispiel 59:

### N-5-(4-(2,3-Dichlorphenyl)piperazin-1-yl)pentyl-5-methoxypyrazolo[1,5-a]pyridin-2-ylcarbamid

MG (ber): 490,43; MS (gef): 490,7; T_{R}: 17,5 min.

### Beispiel 60:

### N-5-(4-(2-Methoxyphenyl)piperazin-yl)pentyl-5-methoxypyrazolo[1,5-a]pyridin-2-ylcarbamid

MG (ber): 451,57; MS (gef): 452,4; T_{R}: 18,7 min.

### Beispiel 61:

### N-5-(4-(2,3-Dichlorphenyl)piperazin-1yl)pentyl-5-trifluormethylpyrazolo[1,5-a]pyridin 2-ylcarbamid

MG (ber): 528,4; MS (gef): 529,5; T_{R}: 18,1 min.

### Beispiel 62:

### N-5-(4-(2-Methoxyphenyl)piperazin-1-yl)pentyl-5-trifluomethylpyrazolo[1,5-a]pyridin-2-ylcarbamid

MG (ber): 484,54; MS (gef):.490,2;.T_{R}: 19,8 min.

### Beispiel 63:

### N-5-(4-(2,3-Dichlorphenyl)piperazin-1-yl)pentyl-6-brompyrazolo[1,5-a]pyridin-2-ylcarbamid

MG (ber): 539,30; MS (gef): 540,0; T_{R}: 18,0 min.

### Beispiel 64:

### N-5-(4-(2-Methoxyphenyl)piperazin-1-yl)pentyl-6-brompyrazolo[1,5-a]pyridin-2-ylcarbamid

MG (ber): 500,44; MS (gef): 501,7; T_{R}: 19,4 min.

### Beispiel 65:

### N-5-(4-(2,3-Dichlorphenyl)piperazin-1-yl)pentyl-6-chlorpyrazolo[1,5a]pyridin-2-ylcarbamid

MG (ber): 494,84; MS (gef): 495,8; T_{R}: 17,9 min.

### Beispiel 66:

### N-5-(4-(2-Methoxyphenyl)piperazin-1-yl)pentyl-6-chlorpyrazolo[1,5-a]pyridin-2-ylcarbamid

MG (ber): 455,98; MS (gef): 456,4; T_{R}: 19,2 min.

### Beispiel 67:

### N-S-(4-(2,3-Dichlorphenyl)piperazin-1-yl)pentyl-6-fluorpyrazolo[1,5-a]pyrldin-2-ylcarbamid

MG (ber): 478,34; MS (gef): 478,7; T_{R}: 17,4 min.

### Beispiel 68:

### N-5-(4-(2-Methoxyphenyl)piperazin-1-yl)pentyl-6-fluorpyrazolo[1,5-a]pyridin-2-ylcarbamid

MG (ber): 439,53; MS (gef): 440,2; T_{R}:18,6 min.

### BIOLOGISCHE AKTIVITÄT

Die biologischen Aktivitäten der erfindungsgemäßen Verbindungen wurden in Radioligandbindungsuntersuchungen ermittelt. Alle Radioligandexperimente wurden nach von uns beschriebenen Methoden durchgeführt (Hübner, H. et al. J. Med. Chem. 2000, 43, 756-762). Für die Messung der Affinitäten zu den Rezeptoren der D2-Familie kamen Membranhomogenate von chinesischen Hamster-Ovarialzellen (CHO-Zellen) zum Einsatz, die jeweils den humanen D2long-, den humanen D2short- (Hayes, G. et al. Mol. Endocrinol. 1992, 6, 920-926), den humanen D3- (Sokoloff, P. et al. Eur. J. Pharmacol. 1992, 225, 331-337) oder den humanen D4.4-Rezeptorsubtyp (Asghari, V. J. Neurochem. 1995, 65, 1157-1165) stabil exprimierten. Prinzipiell erfolgten die Bindungsassays durch Inkubation der Rezeptorhomogenate mit dem Radioligand [³H]Spiperone und der zu untersuchenden Verbindung in verschiedenen Konzentrationen. Die Ermittlung der Affinitäten zum D1-Rezeptor erfolgte mit nativen Membranhomogenaten, gewonnen aus dem Striatum des Schweines, und dem D1-selektiven Radioliganden [³H]SCH 23390.

Die biologische Testung der durch festphasenunterstützte Synthese gewonnenen Substanzen erfolgte zuerst nach einem Screeningansatz. Dabei wurde die Fähigkeit der Testverbindungen untersucht, bei drei representativen Konzentrationen den Radioliganden von der Bindungsstelle des jeweiligen Rezeptorsubtyps zu verdrängen. Für die aussichtsreichsten Kandidaten wurden anschließend in umfassenden Dosis-WirkungsUntersuchungen die Bindungseigenschaften zu den verschiedenen Dopamin-Rezeptorsubtypen ermittelt sowie exemplarisch die Affinitäten zu den 5-HT1A-, 5-HT2-und α1-Rezeptoren festgestellt.

Die Messung der Bindungsstärken der Verbindungen zu den Serotonin-Rezeptorsubtypen 5-HT1A und 5-HT2 wurden nach von uns beschriebenen Methoden (Heindl, C. et al. Tetrahedron: Asymmetry 2003, 14, 3141-3152) durchgeführt. Dazu inkubierten wir Cortex-Membranpräparationen des Schweines mit den Radioliganden [³H]8-OH-DPAT (für 5-HT1 A) oder [³H]Ketanserin (5-HT2) und den Verbindungen in verschiedenen Konzentrationen. In gleicher Weise wurde die Affinität der Testverbindungen zum α1-Rezeptor des Schweins untersucht, wobei porcine Cortex-Membranpräparationen und der α1-selektive Radiollgand [³H]Prazosin eingesetzt wurden.

Alle im Dopaminrezeptor-Bindungsassay untersuchten Verbindungen zeigten gute bis sehr gute Affinitäten zu den Dopamin-Rezeptoren mit einer klaren Bindungspräferenz zu den Subtypen der D2-Familie. Unabhängig von der Partialstruktur ist dabei immer eine deutliche Selektivität zum D3-Rezeptor zu erkennen, der bei allen getesten Verbindungen mit Ki-Werten zwischen 0.1 und ca 200 nM gebunden wurde.

Untersuchungen zur Bestimmung der intrinsischen Aktivität der Beispielverbindungen wurden in einem Mitogenese-Assay in Anlehnung an die Literatur (Hübner, H. et al. J. Med. Chem. 2000, 43, 4563-4569*;* Bettinetti, L. et al. J. Med. Chem. 2002, 45, 4594-4597) durchgeführt. Dabei wurden verschieden Konzentrationen der zu untersuchenden Verbindung mit D3-Rezeptor exprimierenden Zellen inkubiert und anschließend die rezeptorvermittelte Stimulation der Mitogeneserate durch Einbau des radioaktiven Markers [³H]Thymidin gemessen. Agonistische, partialagonistische oder antagonistische Effekte wurden im Vergleich zur Wirkung des vollen Agonisten Quinpirol ermittelt.

Bei diesem Test zeigen die untersuchten Verbindung unterschiedliche intrinsische Effekte am D3-Rezeptor. So wiesen einige Beispiele keinen [³H]Thymidin-Einbau und sind somit als Antagonisten einzuordnen. Andere Verbindungen zeigen eine Stimulation des Rezeptors im Bereich von 11% - 35% und sind eher als schwach partialagonistisch zu bezeichnen, wohingegen eine dritte Gruppe von Substanzen mit einer intrinsischen Aktivität von 36-50% als Partialagonisten einzuordnen ist.

## Patentansprüche

1. Verbindungen der allgemeinen Formel I, 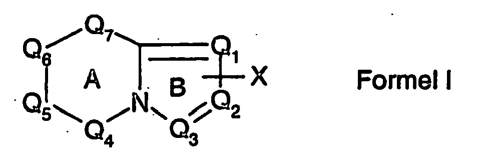 in der bedeuten:
A ist ein gesättigter oder aromatischer 6-gliedriger Ring;
B ist ein aromatischer 5-gliedriger Ring;
das aus A+B gebildete Heteroaren weist insgesamt höchstens drei ringbildende N-Atome und genau eine Gruppe X als Substituenten auf;
Q1, Q2 und Q3 sind jeweils unabhängig voneinander N, CH oder C-R1;
Q4 ist N-R, CH-R1' oder C-R1 R1';
Q5, Q6 und Q7 sind unabhängig voneinander CH-R1' oder C-R1R1';
R1 ist jeweils ausgewählt aus der Gruppe Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino;
R1' fehlt, wenn Ring A aromatisch ist oder ist Wasserstoff, wenn Ring A gesättigt ist;
R fehlt, wenn Ring A aromatisch ist oder ist ausgewählt aus Wasserstoff, Alkyl, Phenyl, Alkylcarbonyl, Phenylcarbonyl, Phenylalkyl und Phenylsulfonyl, wenn Ring A gesättigt ist;
X ist eine an ein C-Atom eines aromatischen Rings A oder B gebundene Gruppe der allgemeinen Formel X1 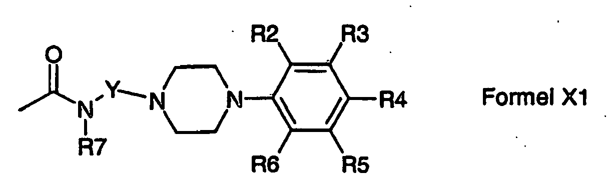 wobei gilt:
Y ist eine unverzweigte, gesättigte oder ungesättigte Kohlenwasserstoffkette mit 2-5 Kohlenstoffatomen oder eine Kette -(CH2)ₒ-Z-(CH2)ₚ, worin Z ausgewählt ist aus den Resten Cyclopentyl, Cyclohexyl und Cycloheptyl, wobei o und p jeweils unabhängig voneinander den Wert 0, 1, 2 oder 3 haben und wobei die Summe aus o und p höchstens 3 ist;
R2, R3, R4, R5 und R6 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Phenylalkyloxy, Halogen, Trifluormethyl, Alkylcarbonyl, Phenylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino, wobei zwei benachbarte Reste R2, R3, R4, R5 und R6 auch gemeinsam mit den C-Atomen des Phenylrings an die sie gebunden sind, einen sauerstoffhaltigen 5-, 6- oder 7-gliedrigen Ring bilden können;
R7 ist Wasserstoff, Alkyl oder Phenylalkyl;
in Form der freien Base, deren physiologisch akzeptable Salze sowie möglicher Enantiomere und Diastereomere,
mit der Maßgabe, daß ausgenommen sind,
(a) Verbindungen, in denen der Heterocyclus ein Pyrazolo[1,5-a]pyridin ist, das als einzigen Substituenten die Gruppe X, aber keinen Substituenten R1 trägt, wobei für X gilt: R2 = Methoxy; R3, R4, R5, R6 und R7 sind jeweils Wasserstoff und
(i) Y = Ethylen, n-Propylen oder n-Butylen oder
(ii) Y = n-Pentylen und X ist in 2- oder 3-Position mit dem Pyrazolo[1,5-a]pyridin-Kern verknüpft
(b) Die Verbindung N-4-(4-(2-Chlorphenyl)piperazin-1-yl)butyl-7-methylpyrazolo[1,5-a]pyridin-3-ylcarbamid.

2. Verbindungen nach Anspruch 1, ausgewählt aus der Gruppe 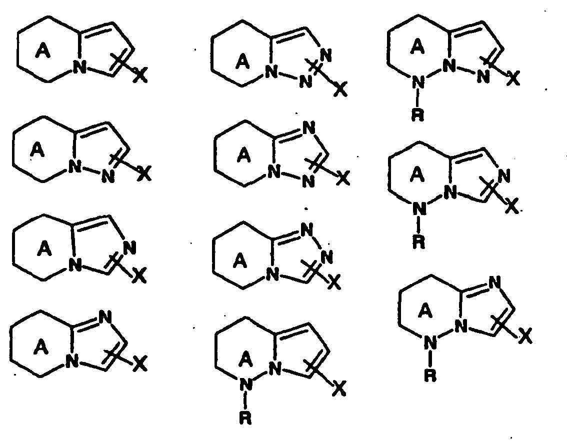 wobei gilt:
der Ring A ist jeweils gesättigt oder aromatisch;
die ringbildenden C-Atome der Ringe A und B können jeweils unabhängig voneinander mit R1 substituiert sein;
R, R1 und X haben die Bedeutung wie in Anspruch 1 beschrieben.

3. Verbindungen nach einem der vorhergehenden Anspruche, worin Y eine Gruppe -(CH₂)ₙ- mit n= 4 oder 5 darstellt.

4. Verbindungen nach einem der vorhergehenden Ansprüche, wobei R7 Wasserstoff ist.

5. Verbindungen nach einem der vorhergehenden Ansprüche mit der allgemeinen Formel II 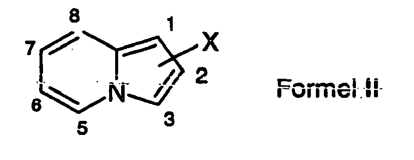 in der bedeuten:
der Substituent X ist mit einer beliebigen Position 1-3 und 5-8 des Indolizins verknüpft und stellt eine Gruppe mit der allgemeinen Formel X1 dar wie in den vorherigen Ansprüchen beschrieben;
das Indolizin kann in den Positionen 1-3 und 5-8 außer X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

6. Verbindungen nach Anspruch 5, wobei X mit der 1-, 2-, oder 3-Position des Indolizins verknüpft ist.

7. Verbindungen nach einem der Ansprüche 5-6, wobei X eine Gruppe der allgemeinen Formel X2 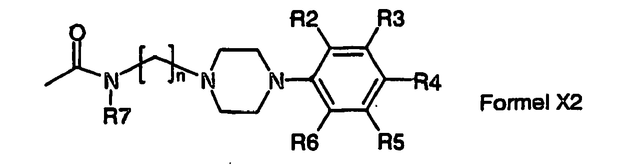 darstellt, in der gilt:
n ist 4 oder 5;
R2, R3, R4, R5, R6 und R7 sind Reste, wie in Anspruch 1 beschrieben.

8. Verbindungen nach einem der Ansprüche 5-7, wobei wenigstens einer der beiden Substituenten R2 und R3 ein Halogenatom oder eine Methoxygruppe darstellt oder wobei R2 und R3 gemeinsam mit dem Phenylrest, an den sie gebunden sind ein Chroman oder Dihydrobenzofuran bilden, während R4 Wasserstoff repräsentiert.

9. Verbindungen nach einem der Ansprüche 1-4, mit der allgemeinen Formel III 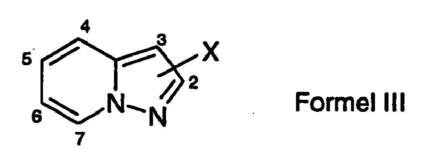 in der bedeuten:
der Substituent X ist mit einer beliebigen Position 2-7 des Pyrazolo[1,5-a]pyridins verknüpft und stellt eine Gruppe mit der allgemeinen Formel X1 dar, wie in einem der Ansprüche 1, 3 oder 4 beschrieben;
das Pyrazolo[1,5-a]pyridin kann in den Positionen 2-7 außer X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

10. Verbindungen nach Anspruch 9, wobei die Gruppe X mit den Positionen 2, 5 oder 6 des Pyrazolo[1,5-a]pyridins verbunden ist.

11. Verbindungen nach einem der Ansprüche 9-10, wobei das Pyrazolo[1,5-a]pyridin in Position 5 einen Methoxy- oder Trifluormethyl-Rest und/oder in Position 6 ein Halogenatom trägt.

12. Verbindungen nach einem der Ansprüche 9-11, wobei X eine Gruppe der allgemeinen Formel X2 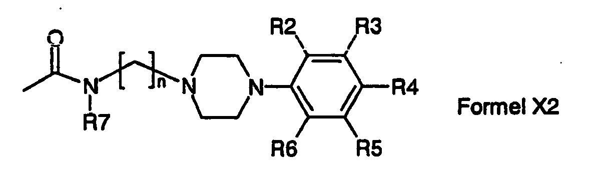 darstellt, in der gilt:
n ist 4 oder 5;
R2, R3, R4, R5, R6 und R7 sind Reste, wie in Anspruch 1 beschrieben.

13. Verbindungen nach einem der Ansprüche 9-12, wobei R4 Wasserstoff repräsentiert und die Substituenten R2 und R3 ausgewählt sind aus der Gruppe Halogen, Alkyl, Alkyloxy, Phenylalkyloxy, Alkylthio, Trifluoromethyl, Cyano oder Nitro oder wobei die beiden Substituenten R2 und R3 gemeinsam einen Chroman oder Dihydrobenzofuranring bilden.

14. Verbindungen nach einem der Ansprüche 1-4 mit der allgemeinen Formel IV 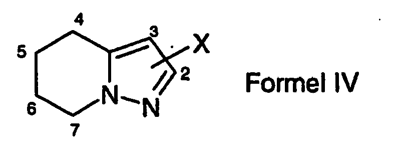 in der bedeuten:
der Substituent X ist mit den Positionen 2 oder 3 des 4,5,6,7-Tetrahydropyrazolo[1,5-a]pyridins verknüpft und stellt eine Gruppe mit der allgemeinen Formel X1 dar wie in einem der Ansprüche 1, 3 oder 4 beschrieben;
das 4,5,6,7-Tetrahydropyrazolo[1,5-a]pyridin kann in den Positionen 2-7 außer der Gruppe X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

15. Verbindungen nach einem der Ansprüche 13-14, wobei X eine Gruppe der allgemeinen Formel X2 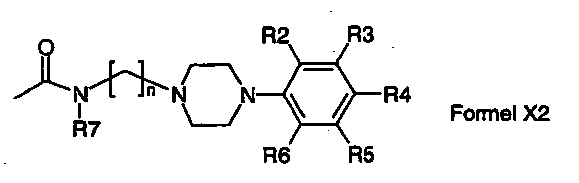 darstellt, in der gilt:
n ist 4 oder 5;
R2, R3, R4, R5, R6 und R7 sind Reste, wie in Anspruch 1 beschrieben.

16. Verbindungen nach einem der Ansprüche 14-15, wobei R4 Wasserstoff repräsentiert und wobei mindestens einer der Substituenten R2 und R3 ein Halogenatom oder eine Methoxygruppe ist.

17. Verbindungen nach einem der Ansprüche 1-4 mit der allgemeinen Formel V 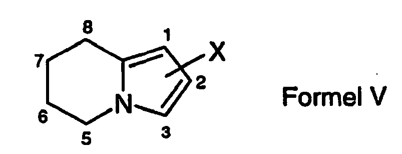 in der bedeuten:
der Substituent X ist mit den Positionen 1, 2 oder 3 des 5,6,7,8-Tetrahydroindolizins verknüpft und stellt eine Gruppe mit der allgemeinen Formel X1 dar wie in einem der Ansprüche 1, 3 oder 4 beschrieben;
das 5,6,7,8-Tetrahydroindolizin kann in den Positionen 1-3 und 5-8 außer der Gruppe X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkytsulfonylamino.

18. Verbindungen nach Anspruch 17, wobei X eine Gruppe der allgemeinen Formel X2 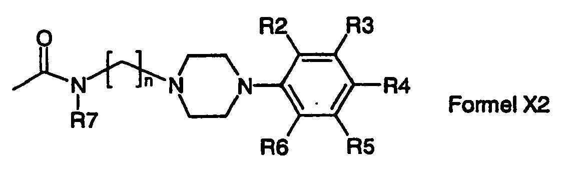 darstellt, in der gilt:
n ist 4 oder 5;
R2, R3, R4, R5, R6 und R7 sind Reste, wie in Anspruch 1 beschrieben.

19. Verbindungen nach einem der Ansprüche 17-18, wobei R4 Wasserstoff repräsentiert und mindestens einer der Substituenten R2 und R3 ein Halogenatom oder eine Methoxygruppe ist.

20. Verbindungen nach einem der Ansprüche 1-4 mit der allgemeinen Formel VI 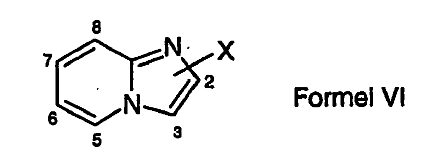 in der bedeuten:
der Substituent X ist mit den Positionen 2-3 oder 5-8 des Heteroarenkerns verknüpft und stellt eine Gruppe mit der allgemeinen Formel X1 dar wie in einem der Ansprüche 1, 3 oder 4 beschrieben;
der Heteroarenkem der Formel VI kann in den Positionen 2-3 und 5-8 außer der Gruppe X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

21. Verbindungen nach Anspruch 20, wobei X eine Gruppe der allgemeinen Formel X2 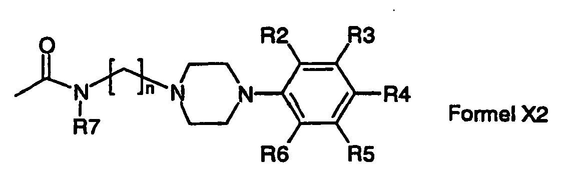 darstellt, in der gilt
n ist 4 oder 5;
R2, R3, R4, R5, R6 und R7 sind Reste, wie in Anspruch 1 beschrieben.

22. Verbindungen nach einem der Ansprüche 20-21, wobei R4 Wasserstoff repräsentiert und mindestens einer der Substituenten R2 und R3 ein Halogenatom oder eine Methoxygruppe ist.

23. Verbindungen nach einem der Ansprüche 1-4 mit der allgemeinen Formel VII 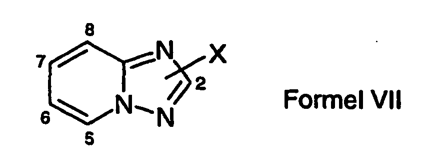 in der bedeuten:
der Substituent X ist mit den Positionen 2 oder 5-8 des Heteroarenkerns verknüpft und stellt eine Gruppe mit der allgemeinen Formel X1 dar wie in einem der Ansprüche 1, 3 oder 4 beschrieben;
der Heteroarenkern der Formel VII kann in den Positionen 2 und 5-8 außer der Gruppe X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

24. Verbindungen nach Anspruch 23, wobei X eine Gruppe der allgemeinen Formel X2 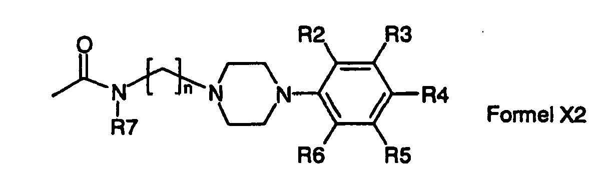 darstellt, in der gilt
n ist 4 oder 5;
R2, R3, R4, R5, R6 und R7 sind Reste, wie in Anspruch 1 beschrieben.

25. Verbindungen nach einem der Ansprüche 23-24, wobei R4 Wasserstoff repräsentiert und mindestens einer der Substituenten R2 und R3 ein Halogenatom ist.

26. Verbindungen nach einem der Ansprüche 1-4 mit der allgemeinen Formel VIII 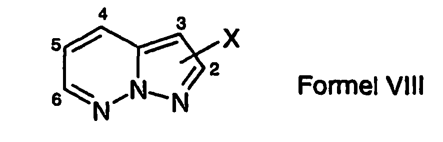 in der bedeuten:
der Substituent X ist mit den Positionen 2-6 des Heteroarenkerns verknüpft. und stellt eine Gruppe mit der allgemeinen Formel X1 dar wie in einem der Ansprüche 1, 3 oder 4 beschrieben;
der Heteroarenkern der Formel VIII kann in den Positionen 2-6 außer der Gruppe X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

27. Verbindungen nach Anspruch 26, wobei X eine Gruppe der allgemeinen Formel X2 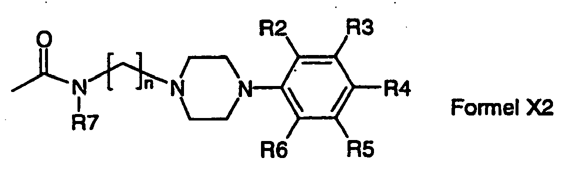 darstellt, in der gilt
n ist 4 oder 5;
R2, R3, R4, R5, R6 und R7 sind Reste, wie in Anspruch 1 beschrieben.

28. Verbindungen nach einem der Ansprüche 26-27, wobei R4 Wasserstoff repräsentiert und mindestens einer der Substituenten R2 und R3 ein Halogenatom ist.

29. Verbindungen nach einem der Ansprüche 1-4 mit der allgemeinen Formel IX 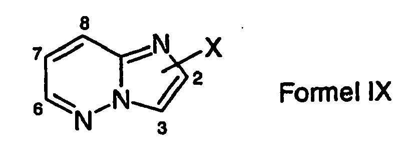 in der bedeuten:
der Substituent X ist mit den Positionen 2-3 und 6-8 des Heteroarenkerns verknüpft und stellt eine Gruppe mit der allgemeinen Formel X1 dar wie in einem der Ansprüche 1, 3 oder 4 beschrieben;
der Heteroarenkern der Formel IX kann in den Positionen 2-3 und 6-8 außer der Gruppe X noch einen oder mehrere weitere Substituenten R1 tragen, die ausgewählt sind aus Hydroxy, Alkyl, Alkyloxy, Alkylthio, Alkenyl, Alkinyl, Phenyl, Phenoxy, Halogen, Trifluormethyl, Alkylcarbonyl, Phenylcarbonyl, Alkyloxycarbonyl, Cyano, Nitro, Amino, Carboxy, Sulfo, Sulfamoyl, Sulfonylamino, Alkylaminosulfonyl und Alkylsulfonylamino.

30. Verbindungen nach Anspruch 29, wobei X eine Gruppe der allgemeinen Formel X2 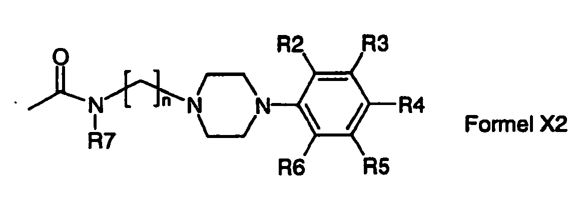 darstellt, in der gilt:
n ist 4 oder 5;
R2, R3, R4, R5, R6 und R7 sind Reste, wie in Anspruch 1 beschrieben.

31. Verbindungen nach einem der Ansprüche 29-30, wobei R4 Wasserstoff repräsentiert und mindestens einer der Substituenten R2 und R3 ein Methoxyrest oder ein Halogenatom ist.

32. Verbindung, ausgewählt aus
N-4-(4-(2-Methoxyphenyl)piperazin-1-yl)butylindolizin-1-ylcarbamid
N-4-(4-(2-Methoxyphenyl)piperazin-1-yl)butylindolizin-2-ylcarbamid
N-4-(4-(3-Chlor-2-methoxyphenyl)piperazin-1-yl)butylindolizin-2-ylcarbamid
N-4-(4-(2,3-Dichlorphenyl)piperazin-1-yl)butylindolizin-2-ylcarbamid
N-4-(4-(2,3-Difluorophenyl)piperazin-1-yl)butylindolizin-2-ylcarbamid
N-4-(4-(2,3-Dihydrobenzofuran-7-yl)piperazin-1-yl)butylindolizin-2-ylcarbamid
N-4-(4-(Chroman-8-yl)piperazin-1-yl)butyindolizin-2-2-ylcarbamid
N-4-(4-(2-Methoxyphenyl)piperazin-1-yl)butyl-5,6,7,8-tetrahydroindolizin-2-ylcarbamid
N-4-(4-(2,3-Dichlorphenyl)piperazin-1-yl)butyl-5,6,7,8-tetrahydrolndolizin-2-ylcarbamid
N-4-(4-(2-Methoxyphenyl)piperazin-1-yl)butyl-1-cyano-2-methylindolizin-3-ylcarbamid
N-2-(4-(2,3-Dichlorphenyl)piperazin-1-yl)ethylpyrazolo[1,5-*a*]pyridin-2-ylcarbamid
N-3-(4-(2,3-Dichlorphenyl)piperazin-1-yl)propylpyrazolo[1,5-*a*]pyridin-2-ylcarbamid
N-4-(4-Phenylpiperazin-1-yl)butylpyrazolo[1,5-*a*]pyridin-2-ylcarbamid
N-4-(4-(2-Methylphenyl)piperazin-1-yl)butylpyrazolo[1,5-*a*]pyridin-2-ylcarbamid
N-4-(4-(2-Biphenyl)piperazin-1-yl)butylpyrazolo[1,5-*a*]pyridin-2-ylcarbamid
N-4-(4-(2-Ethoxyphenyl)piperazin-1-yl)butylpyrazolo[1,5-*a*]pyridin-2-ylcarbamid
N-4-(4-(2-Benzyloxyphenyl)piperazin-1-yl)butylpyrazolo[1,5-*a*]pyridin-2-ylcarbamid
N-4-(4-(2-Methylmercaptophenyl)piperazin-1-yl)butylpyrazoto[1,5-*a*]pyridin-2-ylcarbamid
N-4-(4-(2-Fluorphenyl)piperazin-1-yl)butylpyrazolo[1,5-*a*]pyridin-2-ylcarbamid
N-4-(4-(2-Trifluormethylphenyl)piperazin-1-yl)butylpyrazolo[1,5-*a*]pyridin-2-ylcarbamid
N-4-(4-(2-Cyanophenyl)piperazin-1-yl)butylpyrazolo[1,5-*a*]pyridin-2-ylcarbamid
N-4-(4-(2-Nitrophenyl)piperazin-1-yl)butylpyrazolo[1,5-*a*]pyridin-2-ylcarbamid
N-4-(4-(4-Methoxyphenyl) piperazin-1-yl)butylpyrazolo[1,5-*a*]pyridin-2-ylcarbamid
N-4-(4-(3-Chlor-2-methoxyphenyl)piperazin-1-yl)butylpyrazolo[1,5-*a*]pyridin-2-ylcarbamid
N-4-(4-(2,3-Dichlorphenyl)piperazin-1-yl)butylpyrazolo[1,5-*a*]pyridin-2-ylcarbamid
N-4-(4-(2,3-Dimethylphenyl)piperazin-1-yl)butylpyrazolo[1,5-*a*]pyridin-2-ylcarbamid
N-4-(4-(2,3-Dihydrobignzofuran-7-yl)piperazin-1-yl)butylpyrazolo[1,5-*a*]pyridin-2-ylcarbamid
N-4-(4-(Chroman-8-yl)piperazin-1-yl)butylpyrazolo[1,5-*a*]pyridin-2-ylcarbamid
N-4-(4-(2,4-Dimethoxyphenyl)piperazin-1-yl)butylpyrazolo[1,5-*a*]pyridin-2-ylcarbamid
N-4-(4-(2-Methoxyphenyl)piperazin-1-yl)butyl-3-brompyrazolo[1,5-*a*]pyridin-2-ylcarbamid
N-4-(4-(2-Methoxyphenyl)piperazin-1-yl)butyl-3-chlorpyrazolo[1,5-*a*]pyridin-2-ylcarbamid
N-4-(4-(2,3-Dichlorphenyl)piperazin-1-yl)butyl-5-methoxypyrazolo[1,5-*a*]pyridin-2-ylcarbamid
N-4-(4-(2-Methoxyphenyl)piperazin-1-yl)butyl-5-methoxypyrazolo[1,5-alpyridin-2-ylcarbamid
N-4-(4-(2-Methoxyphenyl)piperazin-1-yl)butyl-5-methylpyrazolo[1,5-*a*]pyridin-2-ylcarbamid
N-4-(4-(2,3-Dichlorphenyl)piperazin-1-yl)butyl-5-trifluormethylpyrazolo[1,5-*a*]pyridin-2-ylcarbamid
N-4-(4-(2-Methoxyphenyl)piperazin-1-yl)butyl-5-trifluormethylpyrazolo[1,5-*a*]pyridin-2-ylcarbamid
N-4-(4-(2,3-Dichlorphenyl)piperazin-1-yl)butyl-6-brompyrazolo[1,5-*a*]pyridin-2-ylcarbamid
N-4-(4-(2-Methoxyphenyl)piperazin-1-yl)butyl-6-brompyrazolo[1,5-*a*]pyridin-2-ylcarbamid
N-4-(4-(2,3-Dichlorphenyl)piperazin-1-yl)butyl-6-chlorpyrazolo[1,5-*a*]pyridin-2-ylcarbamid
N-4-(4-(2-Methoxyphenyl)piperazin-1-yl)butyl-6-chlorpyrazolo[1,5-*a*]pyridin-2-ylcarbamid
N-4-(4-(2,3-Dichlorphenyl)piperazin-1-yl)butyl-6-fluorpyrazolo[1,5-*a*]pyridin-2-ylcarbamid
N-4-(4-(2-Methoxyphenyl)piperazin-1-yl)butyl-6-fluorpyrazolo[1,5-*a*]pyridin-2-ylcarbamid
N-4-(4-(2-Methoxyphenyl)piperazin-1-yl)butyl-3-methoxycarbonylpyrazolo[1,5-*a*]pyridin-2-ylcarbamid
N-4-(4-(2,3-Dichlorphenyl)piperazin-1-yl)butyl-3-methoxycarbonylpyrazolo[1,5-*a*]pyridin-2-ylcarbamid
N-5-(4-(2,3-Dichlorphenyl)piperazin-1-yl)pentylpyrazolo[1,5-*a*]pyridin-2-ylcarbamid
N-5-(4-(2,3-Dichlorphenyl)piperazin-1-yl)pentyl-5-methoxypyrazolo[1,5-*a*]pyridin-2-ylcarbamid
N-5-(4-(2-Methoxyphenyl)piperazin-1-yl)pentyl-5-methoxypyrazolo[1,5-*a*]pyridin-2-ylcarbamid
N-5-(4-(2,3-Dichlorphenyl)piperazin-1-yl)pentyl-5-trifluormethylpyrazolo[1,5-*a*]pyridin-2-ylcarbamid
N-5-(4-(2-Methoxyphenyl)piperazin-1-yl)pentyl-5-trifluormethylpyrazolo[1,5-*a*]pyridin-2-ylcarbamid
N-5-(4-(2,3-Dichlorphenyl)piperazin-1-yl)pentyl-6-brompyrazolo[1,5-*a*]pyridin-2-ylcarbamid
N-5-(4-(2-Methoxyphenyl)piperazin-1-yl)pentyl-6-brompyrazolo[1,5-*a*]pyridin-2-ylcarbamid
N-5-(4-(2,3-Dichlorphenyl)piperazin-1-yl)pentyl-6-chlorpyrazolo[1,5-*a*]pyridin-2-ylcarbamid
N-5-(4-(2-Methoxyphenyl)piperazin-1-yl)pentyl-6-chlorpyrazolo[1,5-*a*]pyridin-2-ylcarbamid
N-5-(4-(2,3-Dichlorphenyl)piperazin-1-yl)pentyl-6-fluorpyrazolo[1,5-*a*]pyridin-2-ylcarbamid
N-5-(4-(2-Methoxyphenyl)piperazin-1-yl)pentyl-6-fluorpyrazolo[1,5-*a*]pyridin-2-ylcarbamid
*trans*-N-(4-((4-(2-Methoxyphenyl)piperazin-1-yl)methyl)cyclohex-1-yl)methylpyrazolo[1,5-*a*]pyridin-2-ylcarbamid
*trans*-N-(4-((4-(2,3-Dichlorphenyl)piperazin-1-yl)methyl)cyclohex-1-yl)methylpyrazolo[1,5-*a*]pyridin-2-ylcarbamid
N-2-(4-(2,3-Dichlorphenyl)piperazin-1-yl)ethylpyrazolo[1,5-*a*]pyridin-3-ylcarbamid
N-3-(4-(2,3-Dichlorphenyl)piperazin-1-yl)propylpyrazolo[1,5-*a*]pyridin-3-ylcarbamid
N-4-(4-(2,3-Dichlorphenyl)piperazin-1-yl)butylpyrazolo[1,5-*a*]pyridin-3-ylcarbamid
N-5-(4-(2,3-Dichlorphenyl)piperazin-1-yl)pentylpyrazolo[1,5-*a*]pyridin-3-ylcarbamid
*trans*-N-(4-((4-(2-Methoxyphenyl)piperazin-1-yl)methyl)cyclohex-1-yl)methylpyrazolo[1,5-*a*]pyridin-3-ylcarbamid
*trans*-N-(4-((4-(2,3-Dichlorphenyl)piperazin-1-yl)methyl)cyclohex-1-yl)methylpyrazolo[1,5-*a*]pyridin-3-ylcarbamid
N-2-(4-(2,3-Dichlorphenyl)piperazin-1-yl)ethylpyrazolo[1,5-*a*]pyridin-5-ylcarbamid
N-3-(4-(2,3-Dichlorphenyl)piperazin-1-yl)propylpyrazolo[1,5-*a*]pyridin-5-ylcarbamid
N-4-(4-(2,3-Dichlorphenyl)piperazin-1-yl)butylpyrazolo[1,5-*a*]pyridin-5-ylcarbamid
N-5-(4-(2,3-Dichlorphenyl)piperazin-1-yl)pentylpyrazolo[1,5-*a*]pyridin-5-ylcarbamid
N-4-(4-(2-Methoxyphenyl)piperazin-1-yl)butyl-3-brompyrazolo[1,5-*a*]pyridin-5-ylcarbamid
N-4-(4-(2-Methoxyphenyl)piperazin-1-yl)butyl-3-chlorpyrazolo[1,5-*a*]pyridin-5-ylcarbamid
N-5-(4-(2-Methoxyphenyl)piperazin-1-yl)pentylpyrazolo[1,5-*a*]pyridin-5-ylcarbamid
N-2-(4-(2,3-Dichlorphenyl)piperazin-1-yl)ethylpyrazolo[1,5-*a*]pyridin-6-ylcarbamid
N-3-(4-(2,3-Dichlorphenyl)piperazin-1-yl)propylpyrazolo[1,5-*a*]pyridin-6-ylcarbamid
N-4-(4-(2,3-Dichlorphenyl)piperazin-1-yl)butylpyrazolo[1,5-*a*]pyridin-6-ylcarbamid
N-5-(4-(2,3-Dichlorphenyl)piperazin-1-yl)pentylpyrazolo[1,5-*a*]pyridin-6-ylcarbamid
N-2-(4-(2,3-Dichlorphenyl)piperazin-1-yl)ethyl-4,5,6,7-tetrahydropyrazolo[1,5-*a*]pyridin-2-ylcarbamid
N-3-(4-(2,3-Dichlorphenyl)piperazin-1-yl)propyl-4,5,6,7-tetrahydropyrazolo[1,5-*a*]pyridin-2-ylcarbamid
N-4-(4-(2,3-Dichlorphenyl)piperazin-1-yl)butyl-4,5,6,7-tetrahydropyrazolo[1,5-*a*]pyridin-2-ylcarbamid
N-4-(4-(2-Methoxyphenyl)piperazin-1-yl)butyl-4,5,6,7-tetrahydropyrazolo[1,5-*a*]pyridin-2-ylcarbamid
N-4-(4-(2-Methoxyphenyl)piperazin-1-yl)butyl-5-methyl-4,5,6,7-tetrahydropyrazolo[1,5-*a*]pyridin-2-ylcarbamid
N-5-(4-(2-Methoxyphenyl)piperazin-1-yl)pentyl-4,5,6,7-tetrahydropyrazolo[1,5-*a*]pyridin-2-ylcarbamid
N-5-(4-(2,3-Dichlorphenyl)piperazin-1-yl)pentyl-4,5,6,7-tetrahydropyrazolo[1,5-*a*]pyridin-2-ylcarbamid
N-2-(4-(2,3-Dichlorphenyl)piperazin-1-yl)ethyl-4,5,6,7-tetrahydropyrazolo[1,5-*a*]pyridin-3-ylcarbamid
N-3-(4-(2,3-Dichlorphenyl)piperazin-1-yl)propyl-4,5,6,7-tetrahydropyrazolo[1,5-*a*]pyridin-3-ylcarbamid
N-4-(4-(2,3-Dichlorphenyl)piperazin-1-yl)butyl-4,5,6,7-tetrahydropyrazolo[1,5-*a*]pyridin-3-ylcarbamid
N-5-(4-(2,3-Dichlorphenyl)piperazin-1-yl)pentyl-4,5,6,7-tetrahydropyrazolo[1,5-*a*]pyridin-3-ylcarbamid
N-4-(4-(2-Methoxyphenyl)piperazin-1-yl)butyl-6-chlorimidazo[1,2-*a*]pyridin-2-ylcarbamid
N-4-(4-(2,3-Dichlorphenyl)piperazin-1-yl)butyl-6-chlorimidazo[1,2-*a*]pyridin-2-ylcarbamid
N-4-(4-(2-Methoxyphenyl)piperazin-1-yl)butyl-6-chlor-2-methylimidazo[1,2-*a*]pyridin-3-ylcarbamid
N-4-(4-(2-Methoxyphenyl)piperazin-1-yl)butylimidazo[1,2-*a*]pyridin-6-ylcarbamid
N-4-(4-(2,3-Dichlorphenyl)piperazin-1-yl)butyl-1,2,4-triazolo[1,5-*a*]pyridin-2-ylcarbamid
N-4-(4-(2,3-Dichlorphenyl)piperazin-1-yl)butylpyrazolo[1,5-*b*]pyridazin-2-ylcarbamid
N-4-(4-(2-Methoxyphenyl)piperazin-1-yl)butyl-6-chlorimidazo[1,2-*b*]pyridazin-2-ylcarbamid
N-4-(4-(2-Methoxyphenyl)piperazin-1-yl)butyl-6-chlor-2-phenylimidazo[1,2-*b*]pyridazin-3-ylcarbamid

33. Verbindungen gemäß einem der vorhergehenden Ansprüche als Arzneimittel.

34. Pharmazeutische Zusammensetzung umfassend eine oder mehrere der Verbindungen nach einem der vorhergehenden Ansprüche und ein pharmazeutisch akzeptables Hilfsmittel.

35. Verwendung einer Verbindung nach einem der vorhergehenden Ansprüche zur Herstellung eines Arzneimittels zur Behandlung von ZNS-Erkrankungen.

36. Verwendung einer Verbindung nach einem der vorhergehenden Ansprüche zur Herstellung eines Arzneimittels zur Behandlung von Störungen des Urinaltrakts.

37. Verwendung einer Verbindung nach einem der vorhergehenden Ansprüche zur Herstellung eines Arzneimittels zur Behandlung von Erkrankungen aus der Gruppe Psychosen, Schizophrenie, Angststörungen, Zwangsstörungen, Drogenabhängigkeit, depressive Störungen, Arzneimittel-induzierte extrapyramidalmotorische Bewegungsstörungen, Morbus Parkinson, Segawa-Syndrom, Tourette-Syndrom, Restless-Leg Syndrom, Schlafstörungen, Übelkeit, kognitive Störungen, männliche erektile Dysfunktion, Hyperprolaktinämie, Hyperprolaktinom, Glaukoma, Hyperaktivitätssyndrom (ADHS), Autismus, Schlaganfall und urinale Inkontinenz,

38. Verwendung nach einem der vorhergehenden Ansprüche, wobei die Verbindung zur Herstellung eines Arzneimittel zur Behandlung von Schizophrenie, depressiven Störungen, L-Dopa- oder Neuroleptika-induzierten Bewegungsstörungen, Morbus Parkinson, Segawa-Syndrom, Restless-Leg-Syndrom, Hyperprolaktinämie, Hyperprolaktinom, Hyperaktivitätssyndrom (ADHS) oder urinale Inkontinenz verwendet werden.

39. Herstellung von Verbindungen nach einem der Ansprüche 1-32 durch Umsetzung eines Säurederivats A 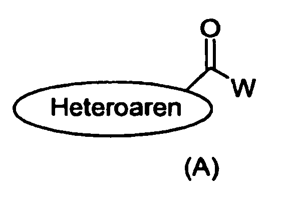 mit einer freien Base der allgemeinen Formel C 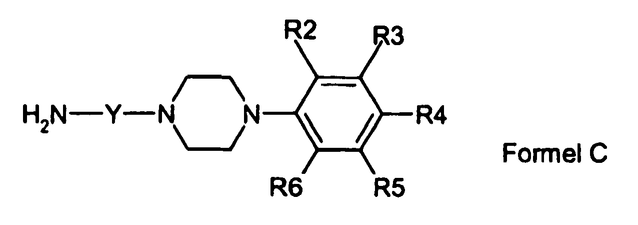 wobei gilt:
W ist ausgewählt aus OH, Cl, Br oder einer Gruppe 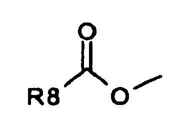 in der R8 für Alkyl steht;
Heteroaren steht jeweils für eine Gruppe, die ausgewählt ist aus 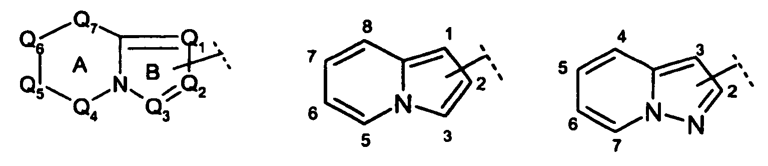 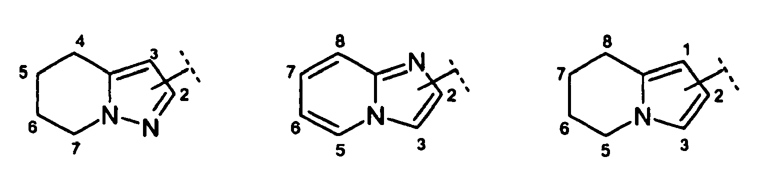 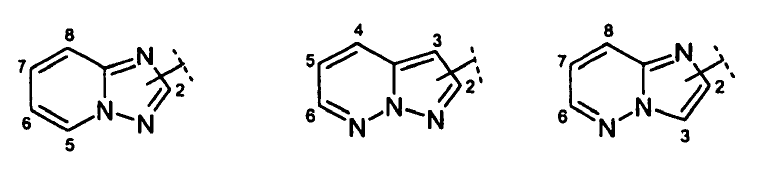 worin
A, B, Q1, Q2, Q3, Q4, Q5, Q6 und Q7 jeweils die Bedeutung haben, wie in den vorhergehenden Ansprüchen definiert und wobei die durchkreuzte Bindung bei den Heteroarenen für eine Bindung der Gruppe -C(O)-W an ein ringbildendes C-Atom eines aromatischen Rings des Heteroarens steht;
die Heteroarene jeweils einen oder mehrere weitere Substituenten R1 oder R tragen können, wie in den vorhergehenden Ansprüchen definiert;
Y, R2, R3, R4, R5 und R6 jeweils die Bedeutung haben, wie in den vorhergehenden Ansprüchen definiert,
und wobei für den Fall, dass der Substituent W eine Hydroxygruppe ist, die entsprechende Säuregruppe vor der Umsetzung mit der freien Base der allgemeinen Formel C durch Zugabe von einem oder mehreren Aktivierungsreagenzien aktiviert wird.

40. Herstellung eines Carbonsäurederivats eines Pyrazolo[1,5-a]pyridins mit der allgemeinen Formel 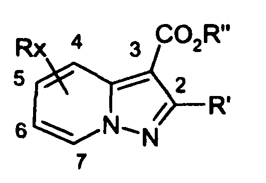 durch Umsetzung eines Pyridins der Formel 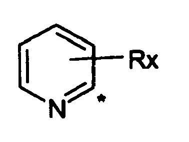 mit O-(2,4-Dinitrophenyl)hydroxylamin zu einem N-Aminopyridin der Formel 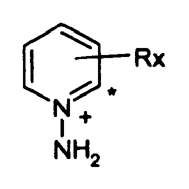 und anschließender Cycloadditionsreaktion mit einem Propiolsäureester der Formel 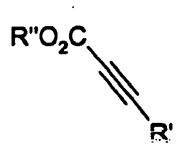 worin Rx für 0, 1, 2, 3 oder 4 gleiche oder verschiedene Substituenten ausgewählt aus Halogen, Alkyl, Alkylcarbonyl, Phenylcarbonyl, Hydroxyalkyl, Cyano, Trifluormethyl und Alkyloxycarbonyl steht, * eine unsubstituierte CH-Gruppe kennzeichnet und worin R' ausgewählt ist aus Wasserstoff, Alkyl, Phenyl und Alkyloxycarbonyl und worin R" Alkyl repräsentiert.

## Claims

1. Compounds of the general formula I, 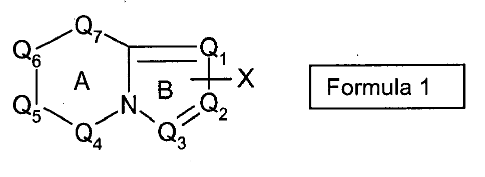 in which:
A is a saturated or aromatic 6-membered ring;
B is an aromatic 5-membered ring;
the heteroarene formed by A+B has in total a maximum of three ring-forming N-atom and precisely one X group as substituents;
Q1, Q2 and Q3 are independently of each other N, CH or C-R1;
Q4 is N-R, CH-R1' or C-R1 R1';
Q5, Q6 and Q7 are independently of each other CH-R1' or C-R1 R1';
R1 is in each case selected from the group comprising hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino;
R1' is absent, if ring A is aromatic or is hydrogen, if ring A is saturated;
R is absent, if ring A is aromatic or is selected from among hydrogen, alkyl, phenyl, alkylcarbonyl, phenylcarbonyl, phenylalkyl and phenylsulfonyl, if ring A is saturated;
X is a group of general formula X1 bonded to a C-atom of an aromatic ring A or B 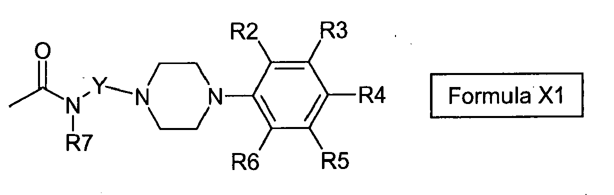 wherein:
Y is an unbranched, saturated or unsaturated hydrocarbon chain with 2-5 carbon atoms or a chain -(CH2)ₒ-Z-(CH2)ₚ, in which Z is selected from the residues cyclopentyl, cyclohexyl and cycloheptyl, wherein o and p in each case and independently of each other have the value 0, 1, 2 or 3 and wherein the sum of o and p is a maximum of 3;
R2, R3, R4, R5 and R6 are independently of each other selected from the group comprising hydrogen, hydroxy, alkyl, alkyloxy, alkylthio, alkenyl, alkinyl, phenyl, phenylalkyl, phenoxy, phenylalkyloxy, halogen, trifluoromethyl, alkylcarbonyl, phenylcarbonyl, alkyloxycarbonyl, phenylalkyloxycarbonyl, cyano, nitro, amino, carboxy, sulfo, sulfamoyl, sulfonylamino, alkylaminosulfonyl and alkylsulfonylamino, wherein two vicinal residues R2, R3, R4, R5 and R6 together with the C-atoms of the phenyl ring to which they are bonded, can form an oxygen-containing 5-, 6- or 7-membered ring;
R7 is hydrogen, alkyl or phenylalkyl;
in the form of the free base, their physiologically acceptable salts and possible enatiomers and diastereomers,
with the proviso that excluded are:
(a) compounds in which the heterocycle is a pyrazolo[1,5-a]pyridine if this carries as the sole substituent the X group, but does not carry a R1 substituent, wherein for X holds: R2 = methoxy; R3, R4, R5, R6 and R7 are in each case hydrogen and
(i) Y = ethylene, n-propylene or n-butylene or
(ii) Y = n-pentylene and X is linked with the pyrazolo[1,5-a] pyridine core in 2- or 3-position
(b) the compound N-4-(4-(2-chlorophenyl)piperazin-1-yl)butyl-7-methylpyrazolo[1,5-a]pyridin-3-ylcarbamide.

2. Compounds according to claim 1, selected from the group comprising 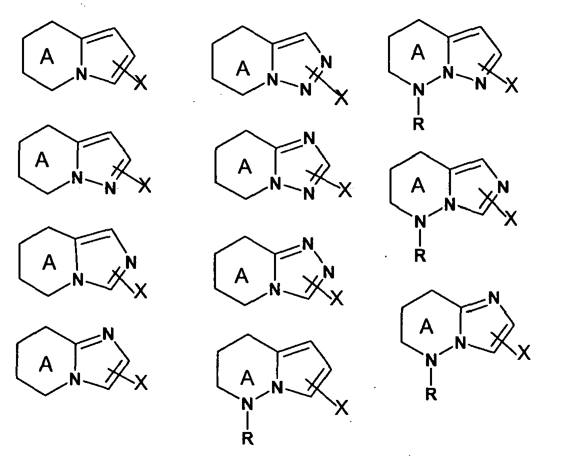 wherein:
the ring A is in each case saturated or aromatic;
the ring-forming C-atoms of rings A and B can independently of each other be substituted with R1;
R, R1 and X are as described in claim 1.

3. Compounds according to anyone of the preceding claims in which Y represents a group -(CH₂)ₙ- with n= 4 or 5.

4. Compounds according to anyone of the preceding claims, wherein R7 is hydrogen.

5. Compounds according to anyone of the preceding claims and having the general formula II 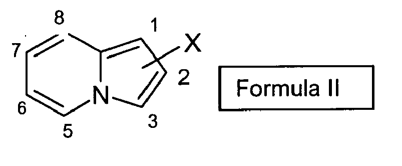 in which:
the substituent X is linked with any position 1-3 and 5-8 of the indolizine and
represents a group of general formula X1 as described in the preceding claims;
the indolizine can in positions 1-3 and 5-8 apart from X also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

6. Compounds according to claim 5, wherein X is linked to the 1-, 2-, or 3-position of the indolizine.

7. Compounds according to anyone of claims 5-6, wherein X represents a group of the general formula X2 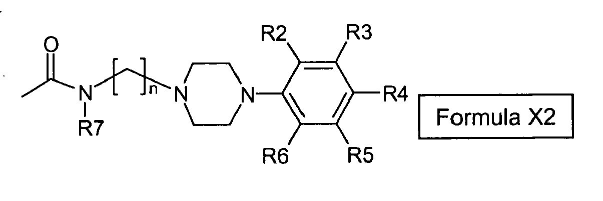 in which:
n is 4 or 5;
R2, R3, R4, R5, R6 and R7 are residues, as described in claim 1.

8. Compounds according to anyone of claims 5-7, wherein at least one of the two substituents R2 and R3 represents a halogen atom or a methoxy group or wherein R2 and R3 together with the phenyl residue, to which they are bonded form a chromane or dihydrobenzofurane, while R4 represents hydrogen.

9. Compounds according to anyone of claims 1-4, of general formula III 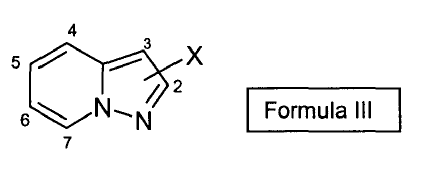 in which:
the substituent X is linked with any position 2-7 of the pyrazolo[1,5-a]pyridine and represents a group of general formula X1, as described in anyone of claims 1, 3 or 4;
the pyrazolo[1,5-a]pyridine in positions 2-7, apart from X, can also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

10. Compounds according to claim 9, wherein the X group is linked to positions 2, 5 or 6 of the pyrazolo[1,5-a]pyridine.

11. Compounds according to anyone of claims 9-10, wherein the pyrazolo[1,5-a]pyridine in position 5 carries a methoxy or trifluoromethyl residue and/or in position 6 a halogen atom.

12. Compounds according to anyone of claims 9-11, wherein X represents a group of general formula X2 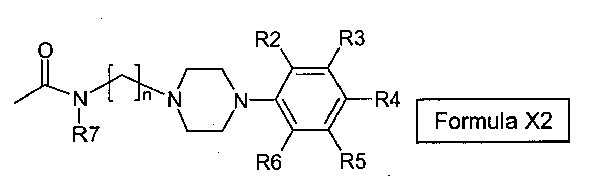 in which:
n is 4 or 5;
R2, R3, R4, R5, R6 and R7 are residues, as described in claim 1.

13. Compounds according to anyone of claims 9-12, wherein R4 represents hydrogen and the substituents R2 and R3 are selected from the group comprising halogen, alkyl, alkyloxy, phenylalkyloxy, alkylthio, trifluoromethyl, cyano or nitro or wherein the two substituents R2 and R3 together form a chromane or dihydrobenzofurane ring.

14. Compounds according to anyone of claims 1-4 of general formula IV 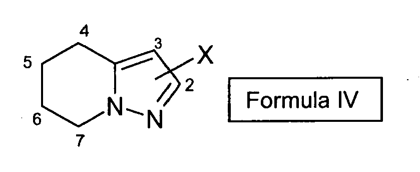 in which:
the substituent X is linked to positions 2 or 3 of the 4,5,6,7-tetrahydropyrazolo[1,5-a]pyridine and represents a group of general formula X1 as described in one of claims 1, 3 or 4;
the 4,5,6,7-tetrahydropyrazolo[1,5-a]pyridine can in positions 2-7 apart from the X group also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

15. Compounds according to either one of claims 13-14, wherein X represents a group of general formula X2 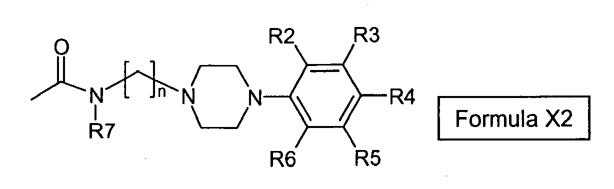 in which:
n is 4 or 5;
R2, R3, R4, R5, R6 and R7 are residues, as described in claim 1.

16. Compounds according to either one of claims 14-15, wherein R4 represents hydrogen and wherein at least one of the substituents R2 and R3 is a halogen atom or a methoxy group.

17. Compounds according to anyone of claims 1-4 of general formula V 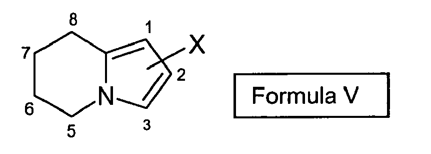 in which:
the substituent X is linked with positions 1, 2 or 3 of the 5,6,7,8-tetrahydroindolizine and represents a group of general formula X1 as described in anyone of claims 1, 3 or 4;
the 5,6,7,8-tetrahydroindolizine can in positions 1-3 and 5-8 apart from the X group also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

18. Compounds according to claim 17, wherein X represents a group of general formula X2 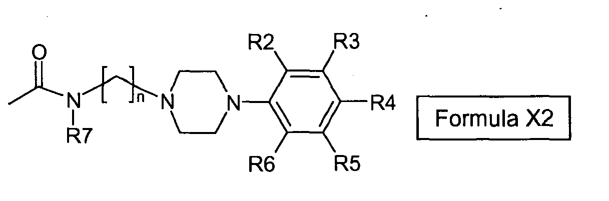 in which:
n is 4 or 5;
R2, R3, R4, R5, R6 and R7 are residues, as described in claim 1.

19. Compounds according to anyone of claims 17-18, wherein R4 represents hydrogen and at least one of the substituents R2 and R3 is a halogen atom or a methoxy group.

20. Compounds according to anyone of claims 1-4 of general formula VI 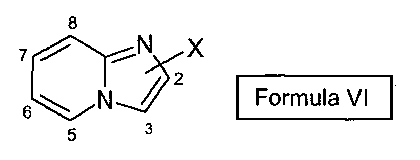 in which:
the substituent X is linked with positions 2-3 or 5-8 of the heteroarene core and represents a group of general formula X1 as described in anyone of claims 1, 3 or 4;
the heteroarene core of formula VI can in positions 2-3 and 5-8 apart from the X group also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

21. Compounds according to claim 20, wherein X represents a group of general formula X2 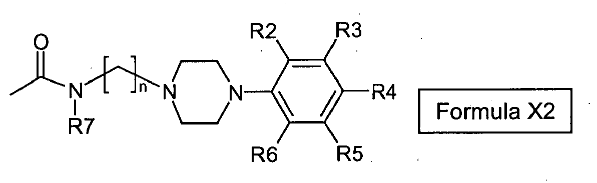 in which:
n is 4 or 5;
R2, R3, R4, R5, R6 and R7 are residues, as described in claim 1.

22. Compounds according to anyone of claims 20-21, wherein R4 represents hydrogen and at least one of the substituents R2 and R3 is a halogen atom or a methoxy group.

23. Compounds according to anyone of claims 1-4 of general formula VII 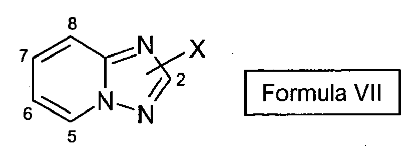 in which:
the substituent X is linked with positions 2 or 5-8 of the heteroarene core and represents a group of general formula X1 as described in anyone of claims 1, 3 or 4;
the heteroarene core of formula VII can in positions 2 and 5-8 apart from the X group also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

24. Compounds according to claim 23, wherein X represents a group of general formula X2 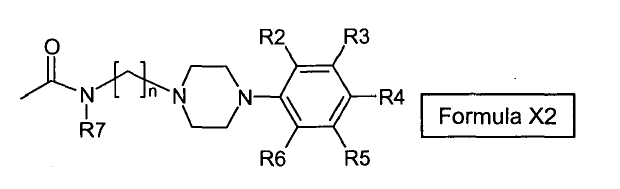 in which:
n is 4 or 5;
R2, R3, R4, R5, R6 and R7 are residues, as described in claim 1.

25. Compounds according to anyone of claims 23-24, wherein R4 represents hydrogen and at least one of the substituents R2 and R3 is a halogen atom.

26. Compounds according to anyone of claims 1-4 of general formula VIII 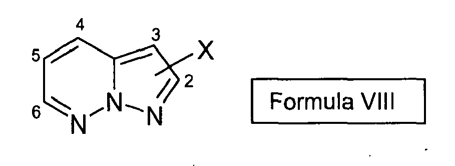 in which:
the substituent X is linked with positions 2-6 of the heteroarene core and represents a group of general formula X1 as described inanyone of claims 1, 3 or 4;
the heteroarene core of formula VIII can in positions 2-6 apart from the X group also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

27. Compounds according to claim 26, wherein X represents a group of general formula X2 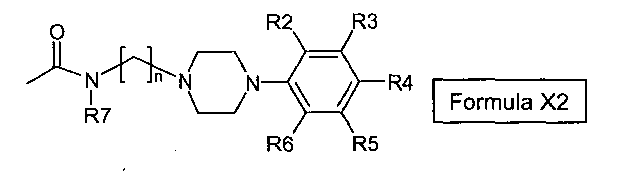 in which:
n is 4 or 5;
R2, R3, R4, R5, R6 and R7 are residues, as described in claim 1.

28. Compounds according to either one of claims 26-27, wherein R4 represents hydrogen and at least one of the substituents R2 and R3 is a halogen atom.

29. Compounds according to anyone of claims 1-4 of general formula IX 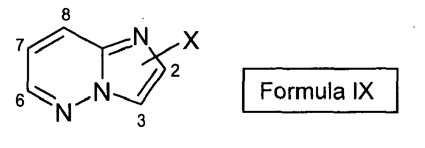 in which:
the substituent X is linked with positions 2-3 and 6-8 of the heteroarene core and represents a group of general formula X1 as described in anyone of claims 1, 3 or 4;
the heteroarene core of formula IX can in positions 2-3 and 6-8 apart from the X group also carry one or more additional substituents R1, selected from among hydroxy, alkyl, alkyloxy, alkylthio, alkenyl, alkinyl, phenyl, phenoxy, halogen, trifluoromethyl, alkylcarbonyl, phenylcarbonyl, alkyloxycarbonyl, cyano, nitro, amino, carboxy, sulfo, sulfamoyl, sulfonylamino, alkylaminosulfonyl and alkylsulfonylamino.

30. Compounds according to claim 29, wherein X represents a group of general formula X2 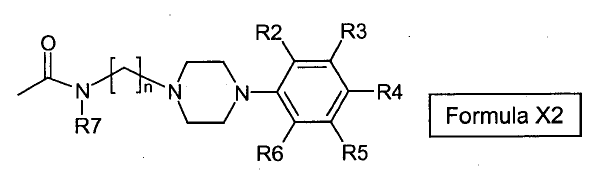 in which:
n is 4 or 5;
R2, R3, R4, R5, R6 and R7 are residues, as described in claim 1.

31. Compounds according to anyone of claims 29-30, wherein R4 represents hydrogen and at least one of the substituents R2 and R3 is a methoxy residue or a halogen atom.

32. Compound, selected from among
N-4-(4-(2-methoxyphenyl)piperazin-1-yl)butylindolizin-1-ylcarbamide
N-4-(4-(2-methoxyphenyl)piperazin-1-yl)butylindolizin-2-ylcarbamide
N-4-(4-(3-chloro-2-methoxyphenyl)piperazin-1-yl)butylindolizin-2-ylcarbamide
N-4-(4-(2,3-dichlorophenyl)piperazin-1-yl)butylindolizin-2-ylcarbamide
N-4-(4-(2,3-difluorophenyl)piperazin-1-yl)butylindolizin-2-ylcarbamide
N-4-(4-(2,3-dihydrobenzofuran-7-yl)piperazin-1-yl)butylindolizin-2-ylcarbamide
N-4-(4-(chroman-8-yl)piperazin-1-yl)butylindolizin-2-ylcarbamide
N-4-(4-(2-methoxyphenyl)piperazin-1-yl)butyl-5,6,7,8-tetrahydroindolizin-2-ylcarbamide
N-4-(4-(2,3-dichlorophenyl)piperazin-1-yl)butyl-5,6,7,8-tetrahydroindolizin-2-ylcarbamide
N-4-(4-(2-methoxyphenyl)piperazin-1-yl)butyl-1-cyano-2-methylindolizin-3-ylcarbamide
N-2-(4-(2,3-dichlorophenyl)piperazin-1-yl)ethylpyrazolo[1,5-*a*]pyridin-2-ylcarbamide
N-3-(4-(2,3-dichlorophenyl)piperazin-1-yl)propylpyrazolo[1,5-*a*]pyridin-2-ylcarbamide
N-4-(4-phenylpiperazin-1-yl)butylpyrazolo[1,5-*a*]pyridin-2-ylcarbamide
N-4-(4-(2-methylphenyl)piperazin-1-yl)butylpyrazolo[1,5-*a*]pyridin-2-ylcarbamide
N-4-(4-(2-biphenyl)piperazin-1-yl)butylpyrazolo[1,5-*a*]pyridin-2-ylcarbamide
N-4-(4-(2-ethoxyphenyl)piperazin-1-yl)butylpyrazolo[1,5-*a*]pyridin-2-ylcarbamide
N-4-(4-(2-benzy)oxyphenyl)piperazin-1-yl)butylpyrazolo[1,5-*a*]pyridin-2-ylcarbamide
N-4-(4-(2-methylmercaptophenyl)piperazin-1-yl)butylpyrazolo[1,5-*a*]pyridin-2-ylcarbamide
N-4-(4-(2-fluorophenyl)piperazin-1-yl)butylpyrazolo[1,5-*a*]pyridin-2-ylcarbamide
N-4-(4-(2-trifluoromethylphenyl)piperazin-1-yl)butylpyrazolo[1,5-*a*]pyridin-2-ylcarbamide
N-4-(4-(2-cyanophenyl)piperazin-1-yl)butylpyrazolo[1,5-*a*]pyridin-2-ylcarbamide
N-4-(4-(2-nitrophenyl)piperazin-1-yl)butylpyrazolo[1,5-*a*]pyridin-2-ylcarbamide
N-4-(4-(4-methoxyphenyl)piperazin-1-yl)butylpyrazolo[1,5-*a*]pyridin-2-ylcarbamide
N-4-(4-(3-chloro-2-methoxyphenyl)piperazin-1-yl)butylpyrazolo[1,5-*a*]pyridin-2-ylcarbamide
N-4-(4-(2,3-dichlorophenyl)piperazin-1-yl)butylpyrazolo[1,5-*a*]pyridin-2-ylcarbamide
N-4-(4-(2,3-dimethylphenyl)piperazin-1-yl)butylpyrazolo[1,5-*a*]pyridin-2-ylcarbamide
N-4-(4-(2,3-dihydrobenzofuran-7-yl)piperazin-1-yl)butylpyrazolo[1,5-*a*]pyridin-2-ylcarbamide
N-4-(4-(chroman-8-yl)piperazin-1-yl)butylpyrazolo[1,5-*a*]pyridin-2-ylcarbamide
N-4-(4-(2,4-dimethoxyphenyl)piperazin-1-yl)butylpyrazolo[1,5-*a*]pyridin-2-ylcarbamide
N-4-(4-(2-methoxyphenyl)piperazin-1-yl)butyl-3-bromopyrazolo[1,5-*a*]pyridin-2-ylcarbamide
N-4-(4-(2-methoxyphenyl)piperazin-1-yl)butyl-3-chloropyrazolo[1,5-*a*]pyridin-2-ylcarbamide
N-4-(4-(2,3-dichlorophenyl)piperazin-1-yl)butyl-5-methoxypyrazolo[1,5-*a*]pyridin-2-ylcarbamide
N-4-(4-(2-methoxyphenyl)piperazin-1-yl)butyl-5-methoxypyrazolo[1,5-*a*]pyridin-2-ylcarbamide
N-4-(4-(2-methoxyphenyl)piperazin-1-yl)butyl-5-methylpyrazolo[1,5-*a*]pyridin-2-ylcarbamide
N-4-(4-(2,3-dichlorophenyl)piperazin-1-yl)butyl-5-trifluoromethylpyrazolo[1,5-*a*]pyridin-2-ylcarbamide
N-4-(4-(2-methoxyphenyl)piperazin-1-yl)butyl-5-trifluoromethylpyrazolo[1,5-*a*]pyridin-2-ylcarbamide
N-4-(4-(2,3-dichlorophenyl)piperazin-1-yl)butyl-6-bromopyrazolo[1,5-*a*]pyridin-2-ylcarbamide
N-4-(4-(2-methoxyphenyl)piperazin-1-yl)butyl-6-bromopyrazolo[1,5-*a*]pyridin-2-ylcarbamide
N-4-(4-(2,3-dichlorophenyl)piperazin-1-yl)butyl-6-chloropyrazolo[1,5-*a*]pyridin-2-ylcarbamide
N-4-(4-(2-methoxyphenyl)piperazin-1-yl)butyl-6-chloropyrazolo[1,5-*a*]pyridin-2-ylcarbamide
N-4-(4-(2,3-dichlorophenyl)piperazin-1-yl)butyl-6-fluoropyrazolo[1,5-*a*]pyridin-2-ylcarbamide
N-4-(4-(2-methoxyphenyl)piperazin-1-yl)butyl-6-fluoropyrazolo[1,5-*a*]pyridin-2-ylcarbamide
N-4-(4-(2-methoxyphenyl)piperazin-1-yl)butyl-3-methoxycarbonylpyrazolo[1,5-*a*]pyridin-2-ylcarbamide
N-4-(4-(2,3-dichlorophenyl)piperazin-1-yl)butyl-3-methoxycarbonylpyrazolo[1,5-*a*]pyridin-2-ylcarbamide
N-5-(4-(2,3-dichlorophenyl)piperazin-1-yl)pentylpyrazolo[1,5-*a*]pyridin-2-ylcarbamide
N-5-(4-(2,3-dichlorophenyl)piperazin-1-yl)pentyl-5-methoxypyrazolo[1,5-*a*]pyridin-2-ylcarbamide
N-5-(4-(2-methoxyphenyl)piperazin-1-yl)pentyl-5-methoxypyrazolo[1,5-*a*]pyridin-2-ylcarbamide
N-5-(4-(2,3-dichlorophenyl)piperazin-1-yl)pentyl-5-trifluoromethylpyrazolo[1,5-*a*]pyridin-2-ylcarbamide
N-5-(4-(2-methoxyphenyl)piperazin-1-yl)pentyl-5-trifluoromethylpyrazolo[1,5-*a*]pyridin-2-ylcarbamide
N-5-(4-(2,3-dichlorophenyl)piperazin-1-yl)pentyl-6-bromopyrazolo[1,5-*a*]pyridin-2-ylcarbamide
N-5-(4-(2-methoxyphenyl)piperazin-1-yl)pentyl-6-bromopyrazolo[1,5-*a*]pyridin-2-ylcarbamide
N-5-(4-(2,3-dichlorophenyl)piperazin-1-yl)pentyl-6-chloropyrazolo[1,5-*a*]pyridin-2-ylcarbamide
N-5-(4-(2-methoxyphenyl)piperazin-1-yl)pentyl-6-chloropyrazolo[1,5-*a*]pyridin-2-ylcarbamide
N-5-(4-(2,3-dichlorophenyl)piperazin-1-yl)pentyl-6-fluoropyrazolo[1,5-*a*]pyridin-2-ylcarbamide
N-5-(4-(2-methoxyphenyl)piperazin-1-yl)pentyl-6-fluoropyrazolo[1,5-*a*]pyridin-2-ylcarbamide
*trans*-N-(4-((4-(2-methoxyphenyl)piperazin-1-yl)methyl)cydohex-1-yl)methyl-pyrazolo[1,5-*a*]pyridin-2-ylcarbamide
*trans*-N-(4-((4-(2,3-dichlorophenyl)piperazin-1-yl)methyl)cyclohex-1-yl)methylpyrazolo[1,5-*a*]pyridin-2-ylcarbamide
N-2-(4-(2,3-dichlorophenyl)piperazin-1-yl)ethylpyrazolo[1,5-*a*]pyridin-3-ylcarbamide
N-3-(4-(2,3-dichlorophenyl)piperazin-1-yl)propylpyrazolo[1,5-*a*]pyridin-3-ylcarbamide
N-4-(4-(2,3-dichlorophenyl)piperazin-1-yl)butylpyrazolo[1,5-*a*]pyridin-3-ylcarbamide
N-5-(4-(2,3-dichlorophenyl)piperazin-1-yl)pentylpyrazolo[1,5-*a*]pyridin-3-ylcarbamide
*trans*-N-(4-((4-(2-methoxyphenyl)piperazin-1-yl)methyl)cyclohex-1-yl)methylpyrazolo[1,5-*a*]pyridin-3-ylcarbamide
*trans*-N-(4-((4-(2,3-dichlorophenyl)piperazin-1-yl)methyl)cyclohex-1-yl)methylpyrazolo[1,5-*a*]pyridin-3-ylcarbamide
N-2-(4-(2,3-dichlorophenyl)piperazin-1-yl)ethylpyrazolo[1,5-*a*]pyridin-5-ylcarbamide
N-3-(4-(2,3-dichlorophenyl)piperazin-1-yl)propylpyrazolo[1,5-*a*]pyridin-5-ylcarbamide
N-4-(4-(2,3-dichlorophenyl)piperazin-1-yl)butylpyrazolo[1,5-*a*]pyridin-5-ylcarbamide
N-5-(4-(2,3-dichlorophenyl)piperazin-1-yl)pentylpyrazolo[1,5-*a*]pyridin-5-ylcarbamide
N-4-(4-(2-methoxyphenyl)piperazin-1-yl)butyl-3-bromopyrazolo[1,5-*a*]pyridin-5-ylcarbamide
N-4-(4-(2-methoxyphenyl)piperazin-1-yl)butyl-3-chloropyrazolo[1,5-*a*]pyridin-5-ylcarbamide
N-5-(4-(2-methoxyphenyl)piperazin-1-yl)pentylpyrazolo[1,5-*a*]pyridin-5-ylcarbamide
N-2-(4-(2,3-dichlorophenyl)piperazin-1-yl)ethylpyrazolo[1,5-*a*]pyridin-6-ylcarbamide
N-3-(4-(2,3-dichlorophenyl)piperazin-1-yl)propylpyrazolo[1,5-*a*]pyridin-6-ylcarbamide
N-4-(4-(2,3-dichlorophenyl)piperazin-1-yl)butylpyrazolo[1,5-*a*]pyridin-6-ylcarbamide
N-5-(4-(2,3-dichlorophenyl)piperazin-1-yl)pentylpyrazolo[1,5-*a*]pyridin-6-ylcarbamide
N-2-(4-(2,3-dichlorophenyl)piperazin-1-yl)ethyl-4,5,6,7-tetrahydropyrazolo[1,5-*a*]pyridin-2-ylcarbamide
N-3-(4-(2,3-dichlorophenyl)piperazin-1-yl)propyl-4,5,6,7-tetrahydropyrazolo[1,5-*a*]pyridin-2-ylcarbamide
N-4-(4-(2,3-dichlorophenyl)piperazin-1-yl)butyl-4,5,6,7-tetrahydropyrazolo[1,5-*a*]pyridin-2-ylcarbamide
N-4-(4-(2-methoxyphenyl)piperazin-1-yl)butyl-4,5,6,7-tetrahydropyrazolo[1,5-*a*]pyridin-2-ylcarbamide
N-4-(4-(2-methoxyphenyl)piperazin-1-yl)butyl-5-methyl-4,5,6,7-tetrahydropyrazolo[1,5-*a*]pyridin-2-ylcarbamide
N-5-(4-(2-methoxyphenyl)piperazin-1-yl)pentyl-4,5,6,7-tetrahydropyrazolo[1,5-*a*]pyridin-2-ylcarbamide
N-5-(4-(2,3-dichlorophenyl)piperazin-1-yl)pentyl-4,5,6,7-tetrahydropyrazolo[1,5-*a*]pyridin-2-ylcarbamide
N-2-(4-(2,3-dichlorophenyl)piperazin-1-yl)ethyl-4,5,6,7-tetrahydropyrazolo[1,5-*a*]pyridin-3-ylcarbamide
N-3-(4-(2,3-dichlorophenyl)piperazin-1-yl)propyl-4,5,6,7-tetrahydropyrazolo[1,5-*a*]pyridin-3-ylcarbamide
N-4-(4-(2,3-dichlorophenyl)piperazin-1-yl)butyl-4,5,6,7-tetrahydropyrazolo[1,5-*a*]pyridin-3-ylcarbamide
N-5-(4-(2,3-dichlorophenyl)piperazin-1-yl)pentyl-4,5,6,7-tetrahydropyrazolo[1,5-*a*]pyridin-3-ylcarbamide
N-4-(4-(2-methoxyphenyl)piperazin-1-yl)butyl-6-chloroimidazo[1,2-*a*]pyridin-2-ylcarbamide
N-4-(4-(2,3-dichlorophenyl)piperazin-1-yl)butyl-6-chloroimidazo[1,2-*a*]pyridin-2-ylcarbamide
N-4-(4-(2-methoxyphenyl)piperazin-1-yl)butyl-6-chloro-2-methylimidazo[1,2-*a*]pyridin-3-ylcarbamide
N-4-(4-(2-methoxyphenyl)piperazin-1-yl)butylimidazo[1,2-*a*]pyridin-6-ylcarbamide
N-4-(4-(2,3-dichlorophenyl)piperazin-1-yl)butyl-1,2,4-triazolo[1,5-*a*]pyridin-2-ylcarbamide
N-4-(4-(2,3-dichlorophenyl)piperazin-1-yl)butylpyrazolo[1,5-*b*]pyridazin-2-ylcarbamide
N-4-(4-(2-methoxyphenyl)piperazin-1-yl)butyl-6-chloroimidazo[1,2-*b*]pyridazin-2-ylcarbamide
N-4-(4-(2-methoxyphenyl)piperazin-1-yl)butyl-6-chloro-2-phenylimidazo[1,2-*b*]pyridazin-3-ylcarbamide

33. Compounds according to anyone of the preceding claims as a medicine.

34. Pharmaceutical composition comprising one or more of the compounds according to anyone of the preceding claims and a pharmaceutically acceptable adjuvant.

35. Use of a compound according to one of the preceding claims for the production of a pharmaceutical preparation for the treatment of central nervous system illnesses.

36. Use of a compound according to anyone of the preceding claims for the production of a pharmaceutical preparation for treatment of urinary tract disorders.

37. Use of a compound according to anyone of the preceding claims for the production of a pharmaceutical preparation for the treatment of illnesses from the group comprising psychoses, schizophrenia, anxiety disorders, compulsive disorders, drug dependency, depressive disorders, drug-induced extrapyramidal motor disturbances, Parkinson's disease, Segawa syndrome, Tourette's syndrome, restless leg syndrome, sleeping disorders, nausea, cognitive disorders, male erectile dysfunction, hyperprolactinemia, hyperprolactinomia, glaucoma, attention deficit hyperactive syndrome (ADHS), autism, stroke and urinary incontinence.

38. Use according to anyone of the preceding claims, wherein the compound is used for the production of a pharmaceutical preparation for the treatment of schizophrenia, depressive disorders, L-dopa- or neuroleptic drug-induced motor disturbances, Parkinson's disease, Segawa syndrome, restless leg syndrome, hyperprolactinemia, hyperprolactinomia, attention deficit hyperactivity syndrome (ADHS) or urinary incontinence.

39. The production of compounds according to anyone of claims 1-32 by conversion of an acid derivative A 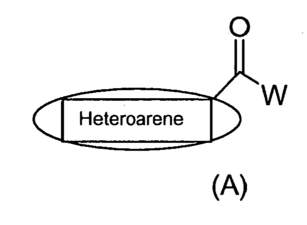 with a free base of general formula C 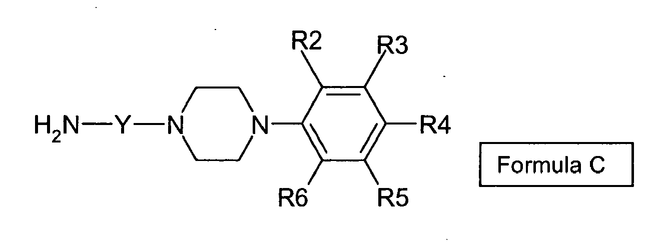 wherein:
W is selected from OH, Cl, Br or a group 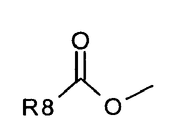 in which R8 stands for alkyl;
heteroarene in each case stands for a group which is selected from 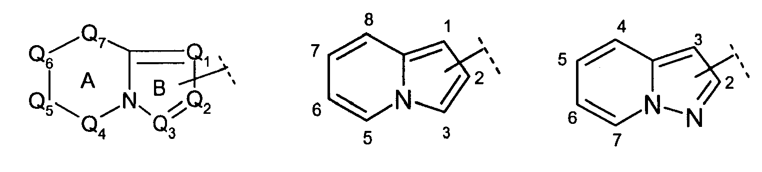 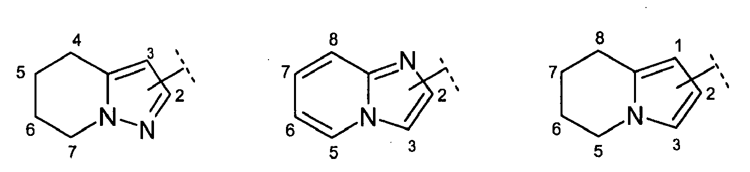 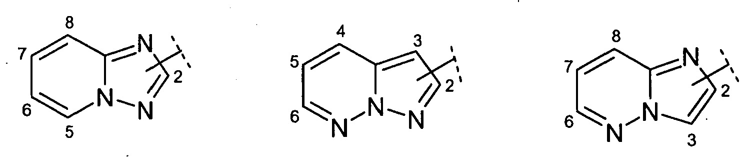 in which
A, B, Q1, Q2, Q3, Q4, Q5, Q6 and Q7 in each case have the significance as defined in the preceding claims and wherein the crossed through bond for the heteroarenes stands for a bond of the -C(O)-W group to a ring-forming C-atom of an aromatic ring of the heteroarene;
the heteroarene can in each case carry one or more further substituents R1 or R, as defined in the preceding claims;
Y, R2, R3, R4, R5 and R6 in each case have the significance as defined in the preceding claims,
and wherein in case that the substituent W is a hydroxy group, the appropriate acid group is activated by addition of one or more activation reagents prior to the conversion with the free base of general formula C.

40. The production of a carboxylic acid derivative of a pyrazolo[1,5-a]pyridine of general formula 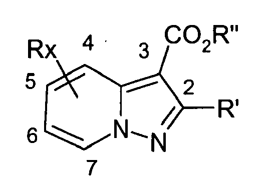 by conversion of a pyridine of formula 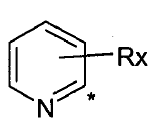 with O-(2,4-dinitrophenyl)hydroxylamine to an N-aminopyridine of formula 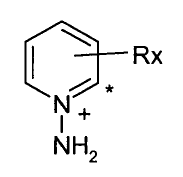 and subsequent cycloaddition reaction with a propiolic acid ester of formula 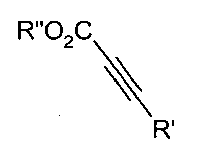 in which Rx stands for 0, 1, 2, 3 or 4 identical or different substituents selected from among halogen, alkyl, alkylcarbonyl, phenylcarbonyl, hydroxyalkyl, cyano, trifluoromethyl and alkyloxycarbonyl, * denotes an unsubstituted CH group and in which R' is selected from hydrogen, alkyl, phenyl and alkyloxycarbonyl and in which R" represents alkyl.

## Revendications

1. Composés selon la formule générale 1, 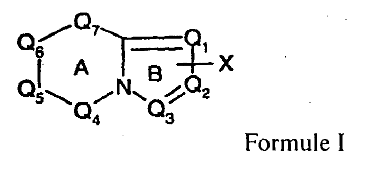 dans laquelle :
A est un cycle saturé ou aromatique à 6 éléments ;
B est un cycle aromatique à 5 éléments ;
l'hétéroarène formé par A+B possède en tout au maximum trois atomes d'azote formant un cycle et exactement un groupe X servant de substituant ;
Q1, Q2 et Q3 sont chacun, indépendamment les uns des autres, N, CH ou C-R1 ;
Q4 est N-R, CH-R1' ou C-R1R1' ;
Q5, Q6 et Q7 sont, indépendamment les uns des autres, CH-R1' ou C-R1R1' ;
R1 est choisi parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino ;
R1' est absent lorsque le cycle A est aromatique ou est l'hydrogène lorsque le cycle A est saturé ;
R est absent lorsque le cycle A est aromatique ou est choisi parmi hydrogène, alkyle, phényle, alkylcarbonyle, phénylcarbonyle, phénylalkyle et phénylsulfonyle, lorsque le cycle A est saturé ;
X est un groupe, lié à un atome de carbone d'un cycle aromatique A ou B, selon la formule X1 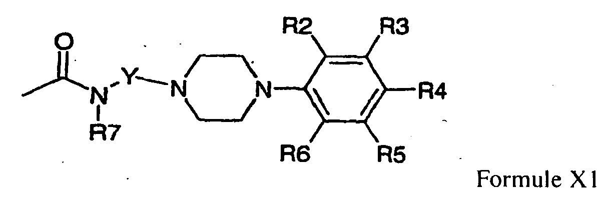 dans laquelle :
Y est une chaîne d'hydrocarbure droite, saturée ou insaturée avec 2 à 5 atomes de carbone ou une chaîne -(CH₂)ₒ-Z-(CH₂)ₚ, où Z est choisi parmi les radicaux cyclopentyle, cyclohexyle et cycloheptyle, o et p ont, indépendamment l'un de l'autre, la valeur 0, 1, 2 ou 3, et la somme de o et p est au maximum de 3 ;
R2, R3, R4, R5 et R6 sont choisis indépendamment les uns des autres parmi hydrogène, hydroxy, alkyle, alkyloxy, alkylthio, alkényle, alkynyle, phényle, phénylalkyle, phénoxy, phénylalkyloxy, halogène, trifluorométhyle, alkylcarbonyle, phénylcarbonyle, alkyloxycarbonyle, phénylalkyloxycarbonyle, cyano, nitro, amino, carboxy, sulfo, sulfamoyle, sulfonylamino, alkylaminosulfonyle et alkylsulfonylamino, deux radicaux R2, R3, R4, R5 et R6 voisins pouvant en outre former avec les atomes de carbone du cycle phényle auquel ils sont liés un cycle oxygéné à 5, 6 ou 7 éléments ;
R7 est l'hydrogène, un alkyle ou un phénylalkyle ;
sous la forme de la base libre, de ses sels physiologiquement acceptables et d'éventuels énantiomères et diastéréomères, à l'exclusion :
(a) des composés dans lesquels l'hétérocycle est une pyrazolo[1,5-*a*]pyridine portant comme seul substituant le groupe X mais pas de substituant R1, avec pour X : R2 = méthoxy ; R3, R4, R5, R6 et R7 sont l'hydrogène et
(i) Y = éthylène, n-propylène ou n-butylène ou
(ii) Y = n-pentylène et X est lié en position 2 ou 3 au noyau de pyrazolo[1,5-*a*]pyridine
(b) du composé N-4-(4-(2-chlorophényl)pipérazin-1-yl)butyl-7-méthylpyrazolo-[1,5-*a*]pyridin-3-ylcarbamide.

2. Composés selon la revendication 1, choisis parmi le groupe 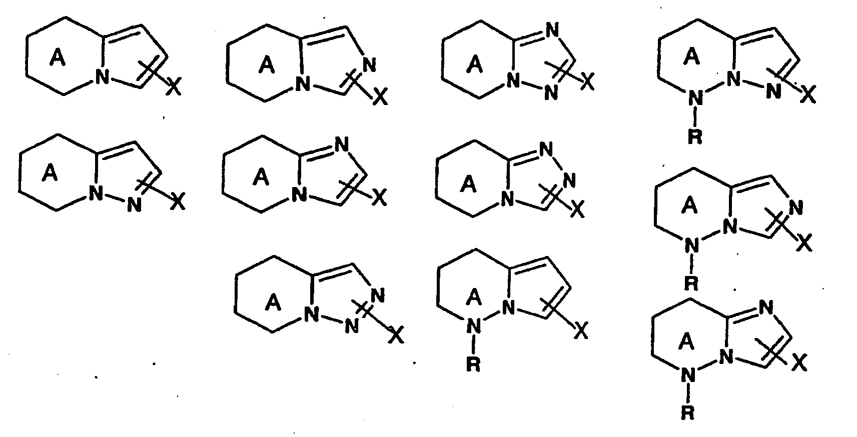 où :
le cycle A est saturé ou aromatique ;
les atomes de carbone formant le cycle dans les cycles A et B peuvent être substitués avec R1 indépendamment l'un de l'autre ;
R, R1 et X ont la signification indiquée dans la revendication 1.

3. Composés selon l'une quelconque des revendications précédentes, dans lesquels Y représente un groupe -(CH₂)ₙ- avec n = 4 ou 5. présente un groupe -(CH₂)ₙ- avec n = 4 ou 5.

4. Composés selon l'une quelconque des revendications précédentes, dans lesquels R7 est l'hydrogène.

5. Composés selon l'une quelconque des revendications précédentes ayant la formule générale II 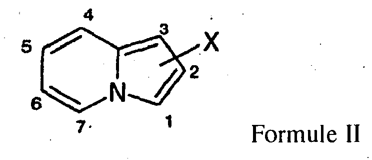 dans laquelle :
le substituant X est lié à une position 1-3 et 5-8 quelconque de l'indolizine et représente un groupe selon la formule générale X 1 tel qu'il est décrit dans les revendications précédentes ;
l'indolizine peut porter dans les positions 1-3 et 5-8, outre X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

6. Composés selon la revendication 5, dans lesquels X est lié à la position 1, 2 ou 3 de l'indolizine.

7. Composés selon l'une quelconque des revendications 5 à 6, dans lesquels X est un groupe selon la formule générale X2 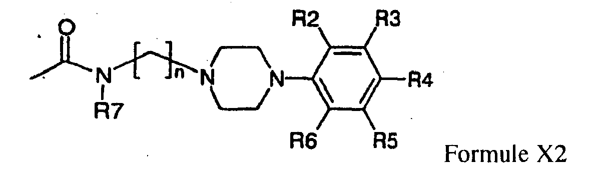 dans laquelle :
n est 4 ou 5 ;
R2, R3, R4, R5, R6 et R7 sont des radicaux tels qu'ils sont décrits dans la revendication 1.

8. Composés selon l'une quelconque des revendications 5 à 7, dans lesquels l'un au moins des deux substituants R2 et R3 représente un atome d'halogène ou un groupe méthoxy et dans lesquels R2 et R3 forment avec le radical phényle auquel ils sont liés un chromane ou du dihydrobenzofurane, tandis que R4 représente l'hydrogène.

9. Composés selon l'une quelconque des revendications 1 à 4, selon la formule générale III 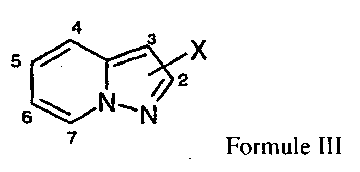 dans laquelle :
le substituant est lié à une position quelconque de 2 à 7 de la pyrazolo| 1,5-*a*|pyridine et représente un groupe selon la formule générale X1 telle qu'elle est décrite dans les revendications 1, 3 ou 4 ;
la pyrazolo| 1,5-*a*|pyridine peut porter dans les positions 2 à 7, outre X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

10. Composés selon la revendication 9, dans lesquels le groupe X est lié aux positions 2, 5 ou 6 de la pyrazolo|1,5-*a*|pyridine.

11. Composés selon l'une quelconque des revendications 9 à 10, dans lesquels la pyrazo-lo|1,5-*a*|pyridine porte dans la position 5 un radical méthoxy ou trifluorométhyle et/ou dans la position 6 un atome d'halogène.

12. Composés selon l'une quelconque des revendications 9 à 11, dans lequel X est un groupe selon la formule générale X2 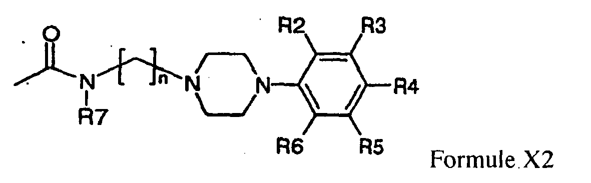 dans laquelle :
n est 4 ou 5 ;
R2, R3, R4, R5, R6 et R7 sont des radicaux tels qu'ils sont décrits dans la revendication 1.

13. Composés selon l'une quelconque des revendications 9 à 12, dans lesquels R4 représente l'hydrogène et les substituants R2 et R3 sont choisis parmi halogène, alkyle, alkyloxy, phénylalkyloxy, alkylthio, trifluorométhyle, cyano ou nitro ou dans lesquels les deux substituants R2 et R3 forment ensemble un chromane ou un cycle dihydrobenzofurane.

14. Composés selon l'une quelconque des revendications 1 à 4 ayant la formule générale IV 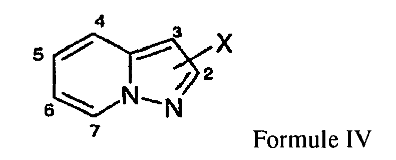 dans laquelle :
le substituant X est lié aux positions 2 ou 3 de la 4,5,6,7-tétrahydropyrazolol 1,5-*a*]pyridine et représente un groupe selon la formule générale X1 telle qu'elle est définie dans les revendications 1, 3 ou 4 ;
la 4,5,6,7-tétrahydropyrazolol 1,5-*a*[pyridine peut porter dans les positions 2 à 7, outre le groupe X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

15. Composés selon l'une quelconque des revendications 13-14, dans lesquels X est un groupe selon la formule générale X2 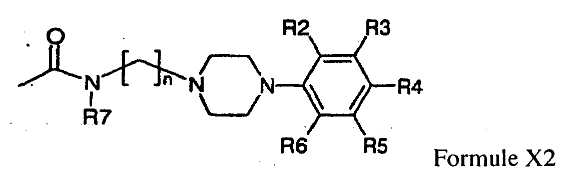 dans laquelle :
n est 4 ou 5 ;
R2, R3, R4, R5, R6 et R7 sont des radicaux tels qu'ils sont décrits dans la revendication 1.

16. Composés selon l'une quelconque des revendications 14-15, dans lesquels R4 représente l'hydrogène et l'un au moins des substituants R2 et R3 est un atome d'halogène ou un groupe méthoxy.

17. Composés selon l'une quelconque des revendications 1 à 4 ayant la formule générale V 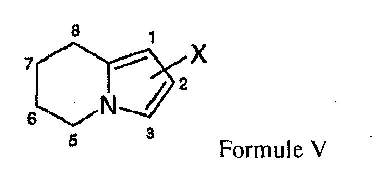 dans laquelle :
le substituant X est lié aux positions 1, 2 ou 3 de la 5,6,7,8-tétrahydroindolizine et représente un groupe selon la formule générale X1 telle qu'elle est décrite dans les revendications 1, 3 ou 4 ;
la 5,6,7,8-tétrahydroindolizine peut porter dans les positions 1-3 et 5-8, outre le groupe X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

18. Composés selon la revendication 17, dans lesquels X est un groupe selon la formule générale X2 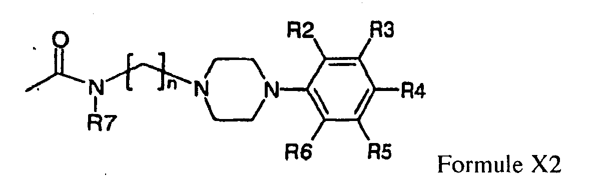 dans laquelle :
n est 4 ou 5 ;
R2, R3, R4, R5, R6 et R7 sont des radicaux tels qu'ils sont décrits dans la revendication 1.

19. Composés selon l'une quelconque des revendications 17-18, dans lesquelles R4 représente l'hydrogène et l'un au moins des substituants R2 et R3 est un atome d'halogène ou un groupe méthoxy.

20. Composés selon l'une quelconque des revendications 1 à 4 selon la formule générale VI 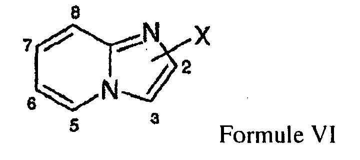 dans laquelle :
le substituant X est lié aux positions 2-3 ou 5-8 du noyau d'hétéroarène et représente un groupe selon la formule générale X1 telle qu'elle est décrite dans les revendications 1 , 3 ou 4 ;
le noyau d'hétéroarène selon la formule VI peut porter dans les positions 2-3 et 5-8, outre le groupe X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

21. Composés selon la revendication 20, dans lesquels X est un groupe selon la formule générale X2 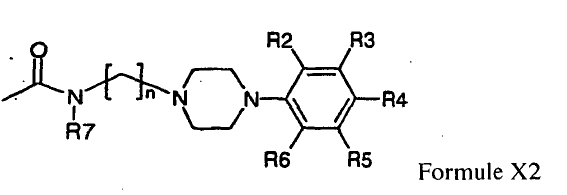 dans laquelle :
n est 4 ou 5 ;
R2, R3, R4, R5, R6 et R7 sont des radicaux tels qu'ils sont décrits dans la revendication 1.

22. Composés selon l'une quelconque des revendications 20-21, dans lesquels R4 représente l'hydrogène et l'un au moins des substituants R2 et R3 est un atome d'halogène ou un groupe méthoxy.

23. Composés selon l'une quelconque des revendications 1 à 4 ayant la formule générale VII 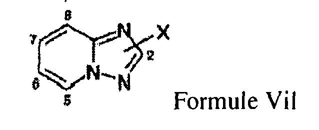 dans laquelle :
le substituant X est lié aux positions 2 ou 5-8 du noyau d'hétéroarène et représente un groupe selon la formule générale X1 telle qu'elle est décrite dans les revendications 1, 3 ou 4;
le noyau d'hétéroarène selon la formule VII peut porter dans les positions 2 et 5-8, outre le groupe X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

24. Composés selon la revendication 23, dans lesquels X est un groupe selon la formule générale X2 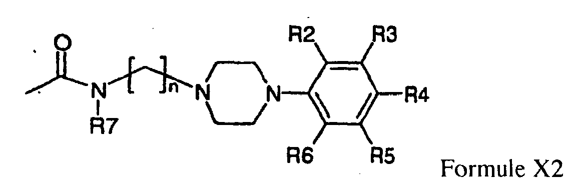 dans laquelle :
n est 4 ou 5 ;
R2, R3, R4, R5, R6 et R7 sont des radicaux tels qu'ils sont décrits dans la revendication 1.

25. Composés selon l'une quelconque des revendications 23-24, dans lesquels R4 représente l'hydrogène et l'un au moins des substituants R2 et R3 est un atome d'halogène.

26. Composés selon l'une quelconque des revendications 1 à 4 ayant la formule générale VIII 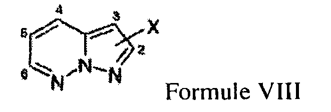 dans laquelle :
le substituant X est lié aux positions 2-6 du noyau d'hétéroarène et représente un groupe selon la formule générale XI telle qu'elle est décrite dans les revendications 1, 3 ou 4;
le noyau d'hétéroarène selon la formule VIII peut porter dans les positions 2-6, outre le groupe X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

27. Composés selon la revendication 26, dans lesquels X est un groupe selon la formule générale X2 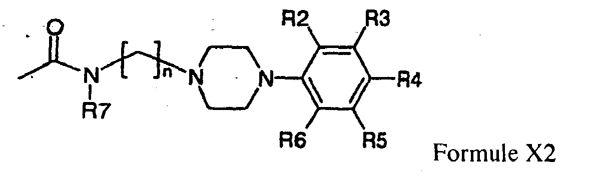 dans laquelle:
n est 4 ou 5 ;
R2, R3, R4, R5, R6 et R7 sont des radicaux tels qu'ils sont décrits dans la revendication 1.

28. Composés selon l'une quelconque des revendications 26-27, dans lesquels R4 représente l'hydrogène et l'un au moins des substituants R2 et R3 est un atome d'halogène.

29. Composés selon l'une quelconque des revendications 1 à 4 ayant la formule générale IX 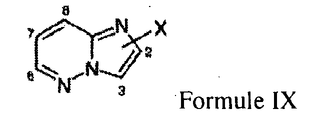 dans laquelle :
le substituant X est lié aux positions 2-3 et 6-8 du noyau d'hétéroarène et représente un groupe selon la formule générale X1 telle qu'elle est décrite dans les revendications 1, 3 ou 4;
le noyau d'hétéroarène selon la formule IX peut porter dans les positions 2-3 et 6-8, outre le groupe X, un ou plusieurs autres substituants R1 choisis parmi hydroxy, alkyle, alkyloxy, alkylthio, alkényle, alkynyle, phényle, phénoxy, halogène, trifluorométhyle, alkylcarbonyle, phénylcarbonyle, alkyloxycarbonyle, cyano, nitro, amino, carboxy, sulfo, sulfamoyle, sulfonylamino, alkylaminosulfonyle et alkylsulfonylamino.

30. Composés selon la revendication 29, dans lesquels X est un groupe selon la formule générale X2 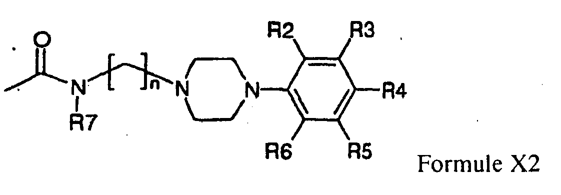 dans laquelle :
n est 4 ou 5 ;
R2, R3, R4, R5, R6 et R7 sont des radicaux tels qu'ils sont décrits dans la revendication 1.

31. Composés selon l'une quelconque des revendications 29-30, dans lesquels R4 représente l'hydrogène et l'un au moins des substituants R2 et R3 est un radical méthoxy ou un atome d'halogène.

32. Composé choisi parmi
N-4-(4-(2-méthoxyphényl)pipérazin-1-yl)butylindolizin-1-ylcarbamide
N-4-(4-(2-méthoxyphényl)pipérazin-1-yl)butylindolizin-2-ylcarbamide
N-4-(4-(3-chloro-2-méthoxyphényl)pipérazin-1-yl)butylindolizin-2-ylcarbamide
N-4-(4-(2,3-dichlorophényl)pipérazin-1-yl)butylindolizin-2-ylcarbamide
N-4-(4-(2,3-difluorophényl)pipérazin-1-yl)butylindolizin-2-ylcarbamide
N-4-(4-(2,3-dihydrobenzofuran-7-yl)pipérazin-1-yl)butylindolizin-2-ylcarbamide
N-4-(4-(chroman-8-yl)pipérazin-1-yl)butylindolizin-2-ylcarbamide
N-4-(4-(2-méthoxyphényl)pipérazin-1-yl)butyl-5,6,7,8-tétrahydroindolizin-2-ylcarbamide
N-4-(4-(2,3-dichlorophényl)pipérazin-1-yl)butyl-5,6,7,8-tétrahydroindolizin-2-ylcarbamide
N-4-(4-(2-méthoxyphényl)pipérazin-1-yl)butyl-1-cyano-2-méthylindolizin-3-ylcarbamide
N-2-(4-(2,3-dichlorophényl)pipérazin-1-yl)éthylpyrazolo[1,5-*a*]pyridin-2-ylcarbamide
N-3-(4-(2,3-dichlorophényl)pipérazin-1-yl)propylpyrazolo[1,5-*a*]pyridin-2-ylcarbamide
N-4-(4-phénylpipérazin-1-yl)butylpyrazolo[1,5-*a*]pyridin-2-ylcarbamide
N-4-(4-(2-méthylphényl)pipérazin-1-yl)butylpyrazolol[1,5-*a*]pyridin-2-ylcarbamide
N-4-(4-(2-biphényl)pipérazin-1-yl)butylpyrazolo [1,5-*a*]pyridin-2-ylcarbamide
N-4-(4-(2-éthoxyphényl)pipérazin-1-yl)butylpyrazolo[1,5-*a*]pyridin-2-ylcarbamide
N-4-(4-(2-benzyloxyphényl) pipérazin-1-yl)butylpyrazolo[1,5-*a*]pyridin-2-ylcarbamide
N-4-(4-(2-méthylmercaptophényl)pipérazin-1-yl)butylpyrazolo[1,5-*a*]pyridin-2-ylcarbamide
N-4-(4-(2-fluorophényl)pipérazin-1-yl)butylpyrazolo[1,5-*a*]pyridin-2-ylcarbamide
N-4-(4-(2-triftuorométhylphényl)pipérazin-1-yl)butylpyrazolo[1,5-*a*]pyridin-2-ylcarbamide
N-4-(4-(2-cyanophényl)pipérazin-1-yl)butylpyrazolo[1,5-*a*]pyridin-2-ylcarbamide
N-4-(4-(2-nitrophényl)pipérazin-1-yl)butylpyrazolo[1,5-*a*]pyridin-2-ylcarbamide
N-4-(4-(4-méthoxyphényl)pipérazin-1-yl)butylpyrazolo[1,5-*a*]pyridin-2-ylcarbamide
N-4-(4-(3-chloro-2-méthoxyphényl)pipérazin-1-yl)butylpyrazolo[1,5-*a*]pyridin-2-ylcarbamide
N-4-(4-(23-dichlorophényl)pipérazin-1-yl)butylpyrazolo[1,5-*a*]pyridin-2-ylcarbamide
N-4-(4-(2,3-diméthylphényl)pipérazin-1-yl)butylpyrazolo[1,5-*a*]pyridin-2-ylcarbamide
N-4-(4-(2,3-dihydrobenzofuran-7-yl)pipérazin-1-yl)butylpyrazolo[1,5-*a*]pyridin-2-ylcarbamide
N-4-(4-(chroman-8-yl)pipérazin-1-yl)butylpyrazolo[1,5-*a*]pyridin-2-ylcarbamide
N-4-(4-(2,4-diméthoxyphényl)pipérazin-1-yl)butylpyrazolo[1,5-*a*]pyridin-2-ylcarbamide
N-4-(4-(2-méthoxyphényl)pipérazin-1-yl)butyl-3-bromopyrazolo[1,5-*a*]pyridin-2-ylcarbamide
N-4-(4-(2-méthoxyphényl)pipérazin-1-yl)butyl-3-chloropyrazolol[1,5-*a*]pyridin-2-ylcarbamide
N-4-(4-(2,3-dichlorophényl)pipérazin-1-yl)butyl-5-méthoxypyrazolo[1,5-*a*]pyridin-2-ylcarbamide
N-4-(4-(2-méthoxyphényl)pipérazin-1-yl)butyl-5-méthoxypyrazolo[1,5-*a*]pyridin-2-ylcarbamide
N-4-(4-(2-méthoxyphényl)pipérazin-1-yl)butyl-5-méthylpyrazolo[1,5-*a*]pyridin-2-ylcarbamide
N-4-(4-(2,3-dichlorophényl)pipérazin-1-yl)butyl-5-trifluorométhylpyrazolo[1,5-*a*]pyridin-2-ylcarbamide
N-4-(4-(2-méthoxyphényl)pipérazin-1-yl)butyl-5-trifluorométhylpyrazolo[1,5-*a*]pyridin-2-ylcarbamide
N-4-(4-(2,3-dichlorophényl)pipérazin-1-yl)butyl-6-bromopyrazolo[1,5-*a*]pyridin-2-ylcarbamide
N-4-(4-(2-méthoxyphényl)pipérazin-1-yl)butyl-6-bromopyrazolo[1,5-*a*]pyridin-2-ylcarbamide
N-4-(4-(2,3-dichlorophényl)pipérazin-1-yl)butyl-6-chloropyrazolo[1,5-*a*]pyridin-2-ylcarbamide
N-4-(4-(2-méthoxyphényl)pipérazin-1-yl)butyl-6-chloropyrazolo[1,5-*a*]pyridin-2-ylcarbamide
N-4-(4-(2,3-dichlorophényl)pipérazin-1-yl)butyl-6-fluoropyrazolo[1,5-*a*]pyridin-2-ylcarbamide
N-4-(4-(2-méthoxyphényl)pipérazin-1-yl)butyl-6-fluoropyrazolo[1,5-*a*]pyridin-2-ylcarbamide
N-4-(4-(2-méthoxyphényl)pipérazin-1-yl)butyl-3-méthoxycarbonylpyrazolo[1,5-*a*]pyridin-2-ylcarbamide
N-4-(4-(2,3-dichlorophényl)pipérazin-1-yl)butyl-3-méthoxycarbonylpyrazolol] 1,5-*a*]pyridin-2-ylcarbamide
N-5-(4-(2,3-dichlorophényl)pipérazin-1-yl)pentylpyrazolo[1,5-*a*]pyridin-2-ylcarbamide
N-5-(4-(2,3-dichlorophényl)pipérazin-1-yl)pentyl-5-méthoxypyrazolo[1,5-*a*]pyridin-2-ylcarbamide
N-5-(4-(2-méthoxyphényl)pipérazin-1-yl)pentyl-5-méthoxypyrazolo[1,5-*a*]pyridin-2-ylcarbamide
N-5-(4-(2,3-dichlorophényl)pipérazin-1-yl)pentyl-5-trifluorométhylpyrazolo[1,5-*a*]pyridin-2-ylcarbamide
N-5-(4-(2-méthoxyphényl)pipérazin-1-yl)pentyl-5-trifluorométhylpyrazolo[1,5-*a*]pyridin-2-ylcarbamide
N-5-(4-(2,3-dichlorophényl)pipérazin-1-yl)pentyl-6-bromopyrazolo[1,5-*a*]pyridin-2-ylcarbamide
N-5-(4-(2-méthoxyphényl)pipérazin-1-yl)pentyl-6-bromopyrazolo[1,5-*a*]pyridin-2-ylcarbamide
N-5-(4-(2,3-dichlorophényl)pipérazin-1-yl)pentyl-6-chloropyrazolo[1,5-*a*]pyridin-2-ylcarbamide
N-5-(4-(2-méthoxyphényl)pipérazin-1-yl)pentyl-6-chloropyrazolo[1,5-*a*]pyridin-2-ylcarbamide
N-5-(4-(2,3-dichlorophényl)pipérazin-1-yl)pentyl-6-fluoropyrazolo[1,5-*a*]pyridin-2-ylcarbamide
N-5-(4-(2-méthoxyphényl)pipérazin-1-yl)pentyl-6-fluoropyrazolo[1,5-*a*]pyridin-2-ylcarbamide
*trans*-N-(4-((4-(2-méthoxyphényl)pipérazin-1-yl)méthyl)cyclohex-1-yl)méthylpyrazolo[1,5-*a*]pyridin-2-ylcarbamide
*trans-*N-(4-((4-(2,3-dichlorophényl)pipérazin-1-yl)méthyl)cyclohex-1-yl)méthylpyrazolo[1,5-*a*]pyridin-2-ylcarbamide
N-2-(4-(2,3-dichlorophényl)pipérazin-1-yl)éthylpyrazolo[1,5-*a*]pyridin-3-ylcarbamide
N-3-(4-(2,3-dichlorophényl)pipérazin-1-yl)propylpyrazolo[1,5-*a*]pyridin-3-ylcarbamide
N-4-(4-(2,3-dichlorophényl)pipérazin-1-yl)butylpyrazolo[1,5-*a*]pyridin-3-ylcarbamide
N-5-(4-(2,3-dichlorophényl)pipérazin-1-yl)pentylpyrazolo[15-*a*]pyridin-3-ylcarbamide
trans-N-(4-((4-(2-méthoxyphényl)pipérazin-1-yl)méthyl)cyclohex-1-yl)méthylpyrazolo[1,5-*a*]pyridin-3-ylcarbamide
trans-N-(4-((4-(2,3-dichlorophényl)pipérazin-1-yl)méthyl)cyclohex-1-yl)méthylpyrazolo[1,5-*a*]pyridin-3-ylcarbamide
N-2-(4-(2,3-dichlorophényl)pipérazin-1-yl)éthylpyrazolo[1,5-*a*]pyridin-5-ylcarbamide
N-3-(4-(2,3-dichlorophényl)pipérazin-1-yl)propylpyrazolo[1,5-*a*]pyridin-5-ylcarbamide
N-4-(4-(2,3-dichlorophényl)pipérazin-1-yl)butylpyrazolo[1,5-*a*]pyridin-5-ylcarbamide
N-5-(4-(2,3-dichlorophényl)pipérazin-1-yl)pentylpyrazolo[1,5-*a*]pyridin-5-ylcarbamide
N-4-(4-(2-méthoxyphényl)pipérazin-1-yl)butyl-3-bromopyrazolo[1,5-*a*]pyridin-5-ylcarbamide
N-4-(4-(2-méthoxyphényl)pipérazin-1-yl)butyl-3-chloropyrazolo[1,5-*a*]pyridin-5-ylcarbamide
N-5-(4-(2-méthoxyphényl)pipérazin-1-yl)pentylpyrazolo[1,5-*a*]pyridin-5-ylcarbamide
N-2-(4-(2,3-dichlorophényl)pipérazin-1-yl)éthylpyrazolol[1,5-*a*]pyridin-6-ylcarbamide
N-3-(4-(2,3-dichlorophényl)pipérazin-1-yl)propylpyrazolo[1,5-*a*]pyridin-6-ylcarbamide
N-4-(4-(2,3-dichlorophényl)pipérazin-1-yl)butylpyrazolo[1,5-*a*]pyridin-6-ylcarbamide
N-5-(4-(2,3-dichlorophényl)pipérazin-1-yl)pentylpyrazolo[1,5-*a*]pyridin-6-ylcarbamide
N-2-(4-(2,3-dichlorophényl)pipérazin-1-yl)éthyl-4,5,6,7-tétrahydropyrazolo[1,5-*a*]pyridin-2-ylcarbamide
N-3-(4-(2,3-dichlorophényl)pipérazin-1-yl)propyl-4,5,67-tétrahydropyrazolo[1,5-*a*[pyridin-2-ylcarbamide
N-4-(4-(2,3-dichlorophényl)pipérazin-1-yl)butyl-4,5,6,7-tétrahydropyrazolo[1,5-*a*]pyridin-2-ylcarbamide
N-4-(4-(2-méthoxyphényl)pipérazin-1-yl)butyl-4,5,6,7-tétrahydropyrazolo[1,5-*a*]pyridin-2-ylcarbamide
N-4-(4-(2-méthoxyphényl)pipérazin-1-yl)butyl-5-méthyl-4,5,6,7-tétrahydropyrazolo[1,5-*a*]pyridin-2-ylcarbamide
N-5-(4-(2-méthoxyphényl)pipérazin-1-yl)pentyl-4,5,6,7-tétrahydropyrazolo[1,5-*a*]pyridin-2-ylcarbamide
N-5-(4-(2,3-dichlorophényl)pipérazin-1-yl)pentyl-4,5,6,7-tétrahydropyrazolo[1,5-*a*)pyridin-2-ylcarbamide
N-2-(4-(2,3-dichlorophényl)pipérazin-1-yl)éthyl-4,5,6,7-tétrahydropyrazolo[1,5-*a*]pyridin-3-ylcarbamide
N-3-(4-(2,3-dichlorophényl)pipérazin-1-yl)propyl-4,5,6,7-tétrahydropyrazolo[1,5-*a*]pyridin-3-ylcarbamide
N-4-(4-(2,3-dichlorophényl)pipérazin-1-yl)butyl-4)5,6,7-tétrahydropyrazolo[1,5-*a*]pyridin-3-ylcarbamide
N-5-(4-(2,3-dichlorophényl)pipérazin-1-yl)pentyl-4,5,6,7-tétrahydropyrazolol 1,5-*a*]pyridin-3-ylcarbamide
N-4-(4-(2-méthoxyphényl)pipérazin-1-yl)butyl-6-chloroimidazo[1,2-*a*]pyridin-2-ylcarbamide
N-4-(4-(2,3-dichlorophényl)pipérazin-1-yl)butyl-6-chloroimidazo[1,2-*a*]pyridin-2-ylcarbamide
N-4-(4-(2-méthoxyphényl)pipérazin-1-yl)butyl-6-chloro-2-méthylimidazo[1,2-*a*]pyridin-3-ylcarbamide
N-4-(4-(2-méthoxyphényl)pipérazin-1-yl)butylimidazo[1,2-*a*]pyridin-6-ylcarbamide
N-4-(4-(2,3-dichlorophényl)pipérazin-1-yl)butyl-1,2,4-triazolo[1,5-*a*]pyridin-2-ylcarbamide
N-4-(4-(2,3-dichlorophényl)pipérazin-1-yl)butylpyrazolol]1,5-/5]pyridazin-2-ylcarbamide
N-4-(4-(2-méthoxyphényl)pipérazin-1-yl)butyl-6-chloroimidazol[1,2-*a*]pyridazin-2-ylcarbamide
N-4-(4-(2-méthoxyphényl)pipérazin-1-yl)butyl-6-chloro-2-phénylimidazol[1,2-*a*]pyridazin-3-ylcarbamide.

33. Composés selon l'une quelconque des revendications précédentes utilisés comme médicaments.

34. Composition pharmaceutique comprenant un ou plusieurs des composés selon l'une quelconque des revendications précédentes et un adjuvant pharmaceutiquement acceptable.

35. Utilisation d'un composé selon l'une quelconque des revendications précédentes pour la fabrication d'un médicament destiné au traitement de maladies du système nerveux central.

36. Utilisation d'un composé selon l'une quelconque des revendications précédentes pour la fabrication d'un médicament destiné au traitement de troubles de l'appareil urinaire.

37. Utilisation d'un composé selon l'une quelconque des revendications précédentes pour la fabrication d'un médicament destiné au traitement de maladies comprenant les psychoses, la schizophrénie, les troubles anxieux, les troubles compulsifs, la toxicomanie, les troubles dépressifs, les troubles moteurs extrapyramidaux d'origine médicamenteuse, la maladie de Parkinson, le syndrome de Segawa, le syndrome de Gilles de la Tourette, le syndrome des jambes sans repos, les troubles du sommeil, les nausées, les troubles cognitifs, les troubles de l'érection chez l'homme, l'hyperprolactinémie, les prolactinomes, le glaucome, le syndrome d'hyperactivité (THDA), l'autisme, les accidents vasculaires cérébraux et l'incontinence urinaire.

38. Utilisation selon l'une quelconque des revendications précédentes, dans laquelle le composé est utilisé pour fabriquer un médicament destiné au traitement de la schizophrénie, des troubles dépressifs, des troubles moteurs induits par la L-dopa ou les neuroleptiques, de la maladie de Parkinson, du syndrome de Segawa, du syndrome des jambes sans repos, de l'hyperprolactinémie, des prolactinomes, du syndrome d'hyperactivité (THDA) ou de l'incontinence urinaire.

39. Fabrication de composés selon l'une quelconque des revendications 1 à 32 par transformation d'un dérivé d'acide A 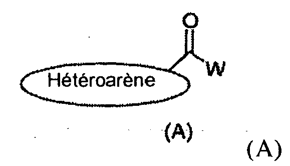 avec une base libre selon la formule générale C 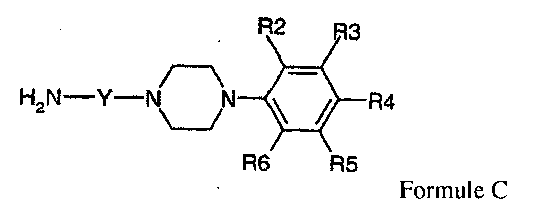 dans laquelle :
W est choisi parmi OH, Cl, Br ou un groupe 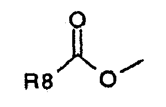 dans lequel R8 est un alkyle ;
« hétéroarène » désigne un groupe choisi parmi 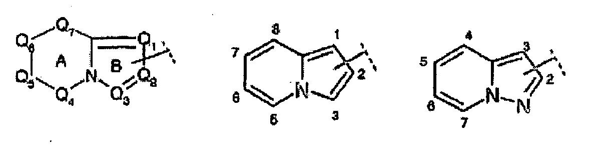 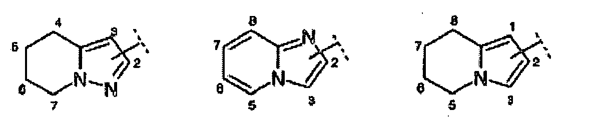 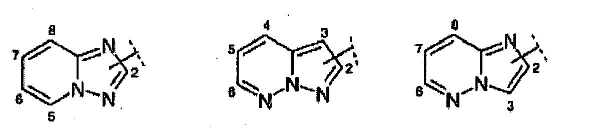 où :
A, B, Q1, Q2, Q3, Q4, Q5, Q6 et Q7 ont chacun la signification définie dans les revendications précédentes et la liaison barrée dans les hétéroarènes représente une liaison du groupe -C(O)-W sur un atome de carbone formant le cycle d'un cycle aromatique de l'hétéroarène ;
les hétéroarènes peuvent porter chacun un ou plusieurs autres substituants R1 ou R tels qu'ils sont définis dans les revendications précédentes ;
Y, R2, R3, R4, R5 et R6 ont chacun la signification définie dans les revendications précédentes ;
et dans le cas où le substituant W est un groupe hydroxy, le groupe acide correspondant est activé avant l'activation avec la base libre selon la formule générale C par addition d'un ou plusieurs réactifs d'activation.

40. Préparation d'un dérivé d'acide carboxylique d'une pyrazolo[1,5-*a*]pyridine selon la 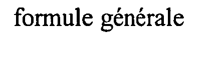 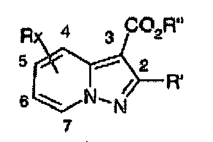 par conversion d'une pyridine selon la formule 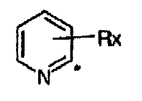 avec de l'O-(2,4-dinitrophényl)hydroxylamine en une N-aminopyridine selon la formule 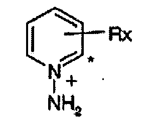 puis réaction de cycloaddition avec un ester d'acide propiolique selon 1a formule 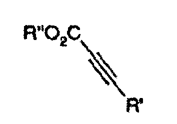 où Rx désigne 0, 1, 2, 3 ou 4 substituants identiques ou différents choisis parmi halogène, alkyle, alkylcarbonyle, phénylcarbonyle, hydroxyalkyle, cyano, trifluorométhyle et alkyloxycarbonyle, * désigne un groupe CH non substitué et R' est choisi parmi hydrogène, alkyle, phényle et alkyloxycarbonyle et R" représente un alkyle.
